(19) Europäisches Patentamt
European Patent Office
Office européen des brevets

(11) EP 0 661 271 B1

(12) **EUROPEAN PATENT SPECIFICATION**

(45) Date of publication and mention of the grant of the patent:
**26.04.2000 Bulletin 2000/17**

(21) Application number: **93919635.8**

(22) Date of filing: **09.09.1993**

(51) Int. Cl.[7]: **C07D 221/24**, C07D 401/00, C07D 405/00, C07D 409/00, A61K 31/435, A61K 31/44

(86) International application number:
**PCT/JP93/01281**

(87) International publication number:
**WO 94/06773 (31.03.1994 Gazette 1994/08)**

(54) **AZEPINE DERIVATIVES, PRODUCTION THEREOF, AND USE THEREOF AS SIGMA-RECEPTOR LIGANDS**

AZEPIN-DERIVATE, DEREN HERSTELLUNG UND VERWENDUNG ALS SIGMA-REZEPTOR-LIGANDEN

DERIVES D'AZEPINE, LEUR PRODUCTION ET UTILISATION COMME LIGANDS DU RECEPTEUR SIGMA

(84) Designated Contracting States:
**CH DE ES FR GB IT LI SE**

(30) Priority: **18.09.1992 JP 25015892**
**06.10.1992 JP 26770292**

(43) Date of publication of application:
**05.07.1995 Bulletin 1995/27**

(73) Proprietor:
**Asahi Kasei Kogyo Kabushiki Kaisha**
**Osaka-shi, Osaka 530-8205 (JP)**

(72) Inventors:
- **TAKAHASHI, Nobuyuki**
  **883, Mifuku**
  **Tagata-gun Shizuoka 410-23 (JP)**
- **MOCHIZUKI, Daisuke**
  **277-3, Chujo**
  **Tagata-gun Shizuoka 410-21 (JP)**

(74) Representative:
**Boeters, Hans Dietrich, Dr. et al**
**Patentanwälte Boeters & Bauer,**
**Bereiteranger 15**
**81541 München (DE)**

(56) References cited:

| | |
|---|---|
| **DE-A- 2 341 925** | **DE-A- 2 914 052** |
| **GB-A- 1 053 085** | **US-A- 3 328 390** |
| **US-A- 3 408 446** | **US-A- 4 945 097** |

- **Arzneim-Forsch., 22(5), 861-869 (1972).**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The present invention relates to an azepine derivative having activity for specific binding to σ-receptor, production thereof, pharmaceutical use thereof, and intermediate thereof.

Prior arts

A σ-receptor has been defined as one of the opioid receptor including with μ-, δ-, κ- and ε-receptor. At present, the σ-receptor is classified not as opiate but as an independent receptor because an opioid antagonist naloxon has no affinity to σ-receptor.

Pharmacological action through σ-receptor has not been investigated. Psychotomimetic drug phencyclidine has affinities for σ-receptor other than NMDA receptor, and antipsychotic drug haloperidol has been known to bind strongly σ-receptor other than dopamine receptor. Therefore, σ-receptor may participate psychic function, however no specific drug for σ-receptor has been reported.

British Patent 852971 (1959), Acta pharmacol, et Toxicol., 17, 277-287 (1960) and Acta Chemica Scandinavica, 17, 2069-2078 (1963) disclosed N-substituted phenylalkyl camphidine derivative of the formula

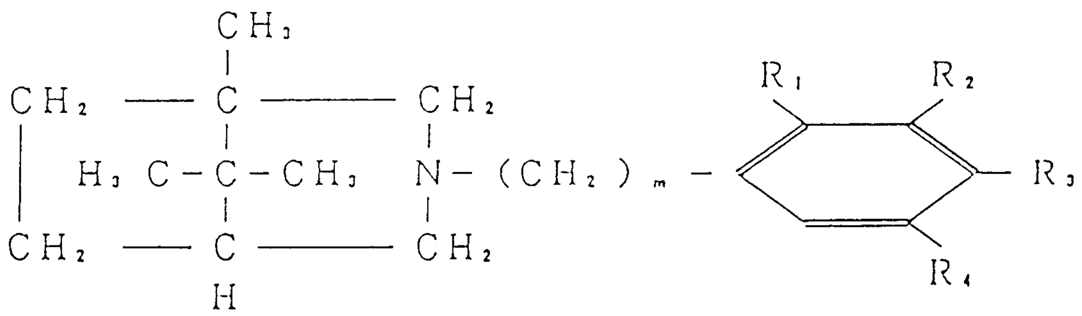

wherein $R_1$, $R_2$, $R_3$ and $R_4$ is H, Cl $-NO_2$, $-NH_2$, $-CH_3$, $-OCH_3$ or -OH and m is an integer of 1-3, and indicated the usefulness for depressor activity upon the central nervous system and tranquilizer.

Yakugaku Zasshi, 84(10), 918-929 (1964) disclosed N-substituted phenylalkyl camphidine derivative of the formula

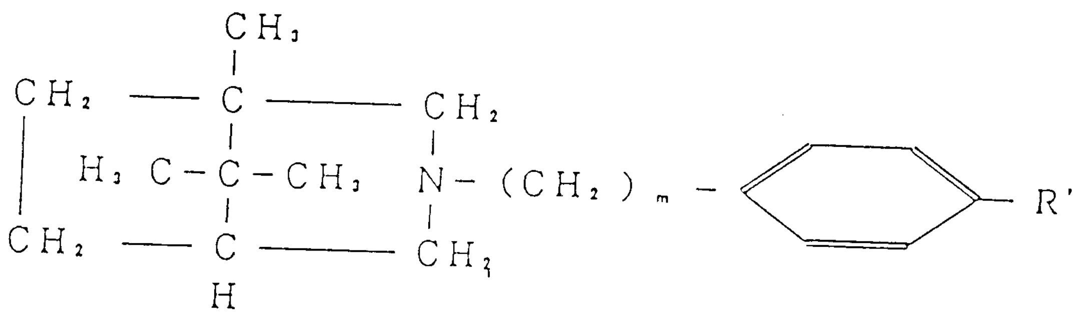

wherein R' is H, Cl or $-NH_2$ and m is 1 or 2 and almost no effect of analgesic action was reported.

In the above prior arts, no usefulness of these prior known compound on apomorphine induced climbing model, a frequently used animal model for schizophrenia, has disclosed.

Problems to be solved by the invention

Creating the specific drug for σ-receptor and finding the novel pharmacological effect are important for developing new type of drugs.

Means for solving the problems

In the course of screening pharmacological activities of novel synthesized compound, we have found that

azepine derivative of the following general formula has high affinity on σ-receptor without indicating affinities on the other receptors, and shows effectiveness for suppressive action of apomorphine induced climbing applied for evaluation of antischizophrenic drug. The present invention has been completed upon the above findings.

Similar compounds have been described in the documents DE-A-2 914 042, DE-A-2 341 925 and GB-A-1 053 085. These compounds differ, however, from the compounds of the present invention in that they concern triazines, pyrimidines or oxadi-, thiadi- or triazoles, respectively.

Documents Arzneim. Forsch. 22,861-9 (1972) and US-A-4 945 097 disclose compounds which differ from the inventive compounds in that they concern phenyl-aminoalcohols.

The compounds, disclosed in document US-A-3 328 390 differ from the compounds of the present invention in that they have a carbonyl-group in the chain linking the two cyclic groups.

The compounds of the present invention are different from the compounds of US-A-3 408 446 in that R may not be phenyl.

An object of the present invention is to provide azepine derivative of the formula

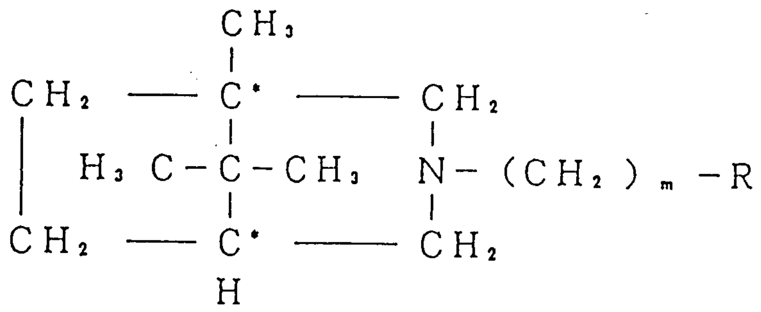

(1)

wherin R is the formula

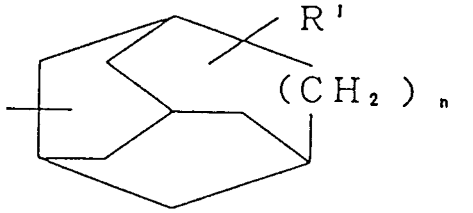

(2)

in which $R^1$ is hydrogen, $C_{1-4}$ alkyl, $C_{1-4}$ alkoxy, hydroxy, halogen or phenyl optionally substituted by 1-3 groups of $C_{1-4}$ alkyl, $C_{1-4}$ alkoxy, hydroxy or halogen , and n is 0 or 1, cycloalkyl of $C_{5-8}$ which may be substituted by $C_{1-4}$ alkyl, norbornyl, bicyclo [3.3.1 ] nonyl, naphthyl, 1,3-benzodioxolyl, pyridyl or thienyl, and m is an integer of 0-4, and C* is asymmetric carbon atom, or a nontoxic salt thereof.

Another object of the present invention is to provide a process for producing an azepine derivative of the above formula (1) or nontoxic salt thereof comprising reducing a carbonyl of the formula

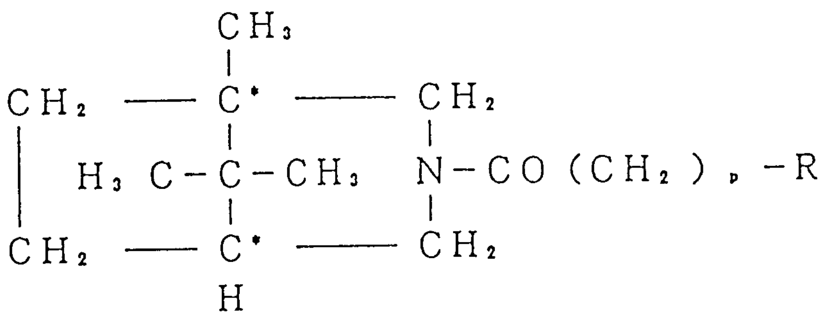

(3)

wherein C* is an asymmetric carbon atom, p is an integer of 0-3, and R has the same meanings as hereinbefore, or reacting an amine of the formula

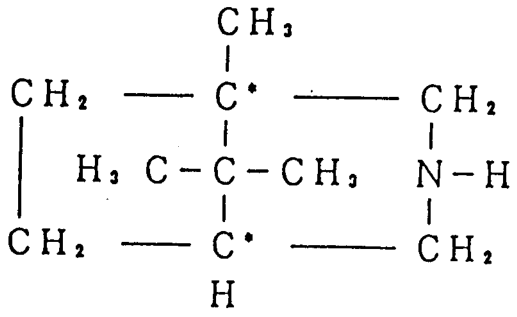

(4)

wherein C* is an asymmetric carbon atom, with a reactive derivative of the formula

$$X - (CH_2)m\text{-}R \qquad (5)$$

wherein X is a reactive organic sulfonyloxy or halogen, m is an integer of 0-4, and R has the same meanings as hereinabove, in inert organic solvent in the presence of base.

A further object of the present invention is to provide a medicament for treatment of diseases participating σ-receptor comprising an azepine derivative of the formula (1) or nontoxic salt thereof as hereinbefore as an active ingredient.

A further object of the present invention is to provide a medicament for treatment of schizophrenia containing an azepine derivative of the formula (1) hereinbefore or a nontoxic salt thereof as an active ingredient.

A still further object of the present invention is to provide an azepine derivative of the formula (3) or a salt thereof as hereinbefore, which is useful for an intermediate of an azepine derivative of the formula (1) hereinbefore and a salt thereof.

Examples of a group R defined in the formula (1) hereinbefore are adamantyl or noradamantyl which is substituted by hydrogen, $C_{1-4}$ alkyl, $C_{1-4}$ alkoxy, hydroxy, halogen or phenyl optionally substituted by 1-3 groups of $C_{1-4}$ alkyl, $C_{1-4}$ alkoxy, hydroxy or halogen. The above substituents may be substituted in any position of carbon atom in adamantane ring or noradamantane ring. Lower alkyl means alkyl of $C_{1-4}$ which may have branched chain. Lower alkoxy means alkoxy of $C_{1-4}$ which may have branched chain. Halogen means chlorine, bromine, fluorine etc. Binding position of those substituents with $-(CH_2)m-$ in adamantane ring or noradmantane ring can be at any position of carbon in the said ring.

Examples of the other group R are cycloalkyl of $C_{5-8}$, norbornyl, bicyclo [3.3.1 ] nonyl, all of which may optionally be substituted by $C_{1-4}$ alkyl. Binding position of the substituent with $-(CH_2)m-$ in norbonyl and bicyclo [3.3.1 ] nonyl may be any position of carbon atom in the said ring.

Examples of cycloalkyl of $C_{5-8}$ are cyclopentyl, cyclohexyl, cycloheptyl and cyclcocyl. Lower alkyl means alkyl of $C_{1-4}$ which may have branched chain.

Examples of further different R are naphthyl, 1,3-benzodioxolyl, pyridyl or thienyl. Naphthyl means α-naphthyl or β-naphthyl. 1,3-benzodioxolyl means 1,3-benzodioxol-2-yl, 1,3-benzodioxol-4-yl or 1,3-benzodioxol-5-yl. Pyridyl means 2-pyridyl 3-pyridyl or 4-pyridyl. Thienyl means 2-thienyl or 3-thienyl.

Azepine derivative (1) and nontoxic salt thereof of the present invention can be produced by the following process.

A) Production of the compound (1) wherein m is 1-4, i.e. the compound (1a) of the formula

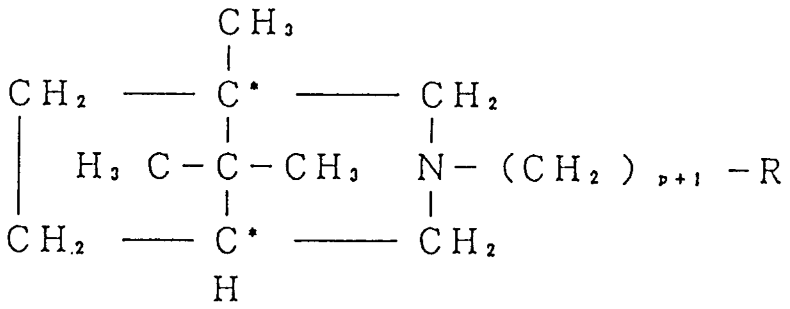

(1 a)

wherein C* is asymmetric carbon, p is an integer of 0-3, and R has the same meaning hereinbefore, by reduction of carbonyl in the compound (3).

The compound (3) hereinbefore can be produced by acylating the amine of the formula (4) hereinbefore with a carboxylic acid of the formula

$$R - (CH_2)p\text{-}COOH \qquad (6)$$

wherein p is an integer of 0-3 and R has the same meanings hereinbefore, or reactive derivative thereof.

The above starting material of amine of the formula (4), i.e. 1,8,8-trimethyl-3-azabicyclo [3.2.1 ] octane has an asymmetric carbon atom as shown in the formula (4), accordingly, an optical active compound of R-configuration or S-configuration is used. Compound of R-configuration is a known compound and is reported, for example, in Acta Chem. Scand., 17, 2069 (1963) and Tetrahedron Letters, 21, 4593 (1980). Compound of S-configuration is unknown compound in the prior references, and can be obtained by reducing (1S) -1,8,8-trimethyl-3-azabicyclo [3.2.1 ] octane-2-one with reducing agent such as lithium aluminium hydride in an inert organic solvent.

Preferable examples of the carboxylic acid of the formula (6) are 1-adamantyl acetic acid. 2-adamantyl acetic acid, 3-methyl-1-adamantyl acetic acid, 1-adamantane carboxylic acid, 2-adamantyl-3-propionic acid, 2-adamantyl-4-butyric acid, 3-noradamantane carboxylic acid, 2-norbornyl acetic acid, 9-bicyclo [3.3.1 ] nonyl acetic acid, cyclohexanecarboxylic acid, cyclopentanecarboxylic acid, cyclopentyl acetic acid, cyclohexyl acetic acid, cycloheptyl acetic acid, cyclooctyl acetic acid, 3-cyclohexylpropionic acid, 4-cyclohexyl butyric acid, 4-methylcyclohexyl acetic acid, 5-hydroxy-2-adamantyl acetic acid, 5-methoxy-2-adamantyl-acetic acid. 5-phenyl-2-adamantyl acetic acid, 4-hydroxy-2-adamantyl acetic acid and 4-phenyl-2-adamantyl acetic acid. A large number of those are known compound and can be available commercially or obtainable by synthesis.

Examples of the carboxylic acid of the formula (6) are α-naphthyl acetic acid, β-naphthyl acetic acid, 1,3-benzodioxol-2-acetic acid, 1,3-benzodioxol-4-acetic acid, 1,3-benzodioxol-5-acetic acid, 2-pyridyl acetic acid, 3-pyridyl acetic acid, 4-pyridyl acetic acid, 2-thienyl acetic acid and 3-thienyl acetic acid. These are known compound and can be obtained commercially or by synthesis.

The above acylation reaction can be performed by known amidation reaction. Free form of the carboxylic acid (6) can be used in the presence of condensing agent. Examples of the condensing agent are carbodiimides such as N,N' -dicyclohexylcarbodiimide (DCC), 1-ethyl-3-(3' -dimethylaminopropyl) carbodiimide (WSC) and N-cyclohexyl-N'-(4-diethylaminocyclohexyl) carbodiimide, a reagent such as diphenylphosphoryl azide, benzotriazoryl-N-hydroxy tris (dimethylamino) phosphonium hexafluoro phosphate and carbonyldiimidazole, a reagent (Vilsmeier reagent) which is synthesized by a reaction of amide compound such as N-methylformamide or N,N' -dimethylformamide with halide such as thionyl chloride, phosphorus chloride or phosgene. The other known condensing agent can be used.

Examples of reactive derivative of the carboxylic acid (6) are halide thereof, acid anhydride. acid azide, activated ester and activated amide. Preferable examples are acid chloride and acid bromide, mixed anhydride with acetic acid, pivalic acid, isovaleric acid, trichloroacetic acid and carboxylate monoalkyl ester, activated ester such as p-nitrophenyl ester, 2,4-dinitrophenyl ester, trichlorophenyl ester, pentachlorophenyl ester, 1-hydroxy-1H-pyridone, N-hydroxysuccinimide ester and N-hydroxyphthalimide ester, and activated amide of pyrazole, imidazole, dimethylpyrazole or benzotriazole.

A reaction using acid halide or acid anhydride of reactive derivative in the above acylating reaction is preferably performed in the presence of deacidification reagent. Examples of deacidification reagent are tertiary amine such as triethylamine, ethyldiisopropylamine, N,N - dimethylaniline, N-methylmorpholine and pyridine, or known inorganic base.

An amount of ratio in amine (4) and carboxylic acid (6) or reactive derivative thereof is theoretically equimolar,

however carboxylic acid (6) or reactive derivative may be used in excess.

Acylating reaction hereinabove is proceeded in an organic solvent which does not effect detrimental effect. Examples of organic solvent are chloroform, methylene chloride, tetrahydrofuran. 1,4-dioxane, N,N-dimethylformamide, N,N-dimethylacetamide and acetone, or mixed solvent thereof. Reaction temperature is not specifically limited, and reaction is usually proceeded at room temperture. Reaction time depends upon reaction temperature and carboxylic acid (6) or reactive derivative thereof and can not be specified, but is atmost up to 48 hours.

Isolating of the compound (3) produced by acylating reaction hereinabove from reaction mixture can be performed by that, in case of reaction solvent being water immiscible organic solvent, after the reaction mixture is washed with aqueous alkaline solution, aqueous acidic solution or saturated sodium chloride solution, organic solvent layer is collected and concentrated, or in case of reaction solvent being water miscible organic solvent, after removal of the solvent, the residue is dissolved in water immiscible organic solvent, then treated the same as of the above to obtain the compound (3). The compound (3) can be purified by known conventional means such as column chromatography and recrystallization.

In the present invention, carbonyl group of the compound (3) is reduced to obtain the compound (1a) of the present invention. The reaction can be performed by reducing the compound (3) in inert organic solvent with reducing agent such as lithium aluminium hydride, sodium aluminium hydride and boron hydride. Examples of inert organic solvent are tetrahydrofuran, diethyl ether, 1,4-dioxane and pyridine. Reaction temperature is usually under heating preferably with reflux condition. Reaction time depends on reaction temperature, type of reducing agent, and the reaction can be checked by means of thin layer chromatography or high performance liquid chromatography, accordingly the reaction can be terminated by observing disappearing the compound (3). The reaction time is, not specified, approximately 1-10 hours.

The compound (1a) produced by reduction reaction hereinabove can be isolated from the reaction mixture by, in case of reaction solvent being water immiscible organic solvent, after washing the reaction mixture with aqueous acidic solution, aqueous alkaline solution or aqueous saturated sodium chloride solution, concentrating the organic solvent layer, and, in case of reaction solvent being water miscible solvent, after removing the solvent by distillation, and if required dissolving the residue in water immiscible organic solvent, and treating the mixture as same as of the above, to obtain the compound (1a). The compound (1a) can be purified further, for example, by conventional known purification method such as column chromatography and recrystallization.

B) Production of compound (1) by N-alkylation of amine (4)

A compound (1) can be produced by reacting an amine of the formula

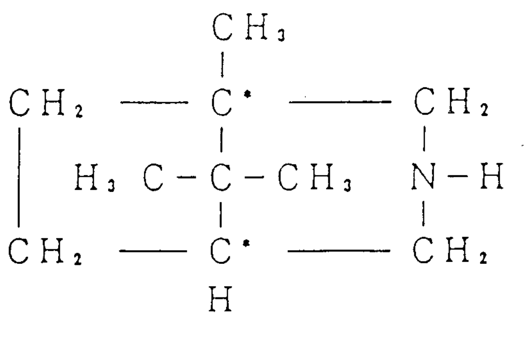

(4)

wherein C* asymmetric carbon atom, with a reactive derivative of the formula

$$X - (CH_2)m\text{-}R \quad (5)$$

wherein X is reactive organic sulfonyloxy or halogen, m is an integer of 0-4, and R has the same meanings hereinbefore, in an inert organic solvent in the presence of base.

Sulfonyloxy derivative in the reactive derivative (5) hereinabove can be produced by sulfonylation of alcohol of the formula

$$HO - (CH_2)m\text{-}R \quad (7)$$

wherein m is an integer of 0-4, with methanesulfonyl chloride or p-toluenesulfonyl chloride in a dry inert solvent such as methylene chloride. The thus produced compound (1) of the present invention can be converted, if required, to pharmaceutically acceptable nontoxic salt thereof. Examples of the alcohol (7) hereinabove are 1-adamantyl ethanol, 1-adamantyl methanol, 2-adamantyl ethanol, 2-norbornyl ethanol, 3-noradamantyl methanol, 9-bicyclo [3.3.1 ] nonyl ethanol, cyclopentyl alcohol, cyclohexyl alcohol, cyclopentyl methanol, cyclohexyl methanol, cycloheptyl methanol, cyclooctyl methanol, cyclopentyl ethanol, cyclohexyl ethanol, cyclopentyl ethanol, 3-cyclohexyl-1-propanol, 4-cyclohexyl-1-butanol, 5-methoxy-2-adamantyl ethanol, 5-chloro-2-adamantyl ethanol, 5-phenyl-2-adamantyl ethanol and 4-phenyl-2-adamantyl ethanol. These compounds are known compound and can be obtainable commercially or by synthesis.

Examples of the other alcohol (7) are α-naphthyl ethanol, β-naphthyl ethanol, 1,3-benzodioxole-2-ethanol, 1,3-benzodixole-4-ethanol, 1,3-benzodixole-5-ethanol, 2-pyridyl ethanol, 3-pyridyl ethanol, 4-pyridyl ethanol, 2-thienyl ethanol and 3-thienyl ethanol. These compounds are known compound and can be obtainable commercially or by synthesis.

Examples halogenide of the reactive derivative (5) are cyclohexyl bromide, cyclohexylmethyl bromide, 1-adamantyl bromide and 2-adamantyl bromide. These compounds are known compound which is listed in the catalogue of the reagents manufacturer, and can be obtainable.

Further examples of halogenide of the reactive derivative (5) hereinabove are α-naphthylethyl bromide, β-naphthylethyl bromide. 1,3-benzodioxole-2-ethyl bromide, 1,3-benzodioxole-4-ethyl bromide. 1,3-benzodioxole-5-ethyl bromide, 2-pyridylethyl bromide, 3-pyridylethyl bromide, 4-pyridylethyl bromide, 2-thienylethyl bromide and 3-thienylethyl bromide.

Examples of base used in a reaction of amine (4) and reactive derivative (5) are, for example, tertiary organic base such as triethylamine, ethyldiisopropylamine, N,N-dimethylaniline. N-methylmorpholine, pyridine and N,N-dimethylaminopyridine, and inorganic base such as carbonate, hydrogen carbonate, hydroxide or hydride of alkaline metal or alkaline earth metal.

Examples of inert organic solvent used in the reaction hereinbefore are acetonitrile, benzene, acetone and tetrahydrofuran, and acetonitrile is most preferable. The above reaction proceeds, usually, at reflux temperature of the reaction solvent used. Reaction time depends on a reaction temperature or base, and reaction progress can be checked by thin layer chromatography or high performance liquid chromatography, and the reaction can be terminated by checking disappearance of amine (4). Reaction time is, not specified, approximately 2-72 hours.

Isolation of the compound (1) from reaction mixture hereinbefore can be performed by filtering insoluble material, consentrating the solvent, and purifying the residue by conventional silica-gel column chromatography with the developer of mixture of chloroform and acetone or methanol.

In the present invention, when a compound of the present invention (1) is disclosed, the compound can easily be synthesised by combining prior known reactions other than the process hereinabove explained.

The thus produced compound of the present invention (1) can be converted, if required, to a pharmaceutically acceptable nontoxic salt thereof.

Examples of those salts are acid addition salt, and are inorganic salt of hydrochloride, sulfate and phosphate, and organic salt of acetate, propionate, tartrate, citrate, glycolate, gluconate, succinate, malate, glutamate, aspartate, metanesulfonate, mandelate, p-toluenesulfonate and maleinate.

The compound of the present invention (1) or nontoxic salt thereof showed no death in an intraveneous administration of 30 mg/kg to rat and the compound can be said quite safety to use as a medicament.

The compound of the present invention or nontoxic salt thereof is used in the form of formulation and is administered per oral or parenterally such as injection including drop infusion. An amount of administration may be varied by dosage form, age, body weight and condition of recipients, and is 0.1 mg-100 mg/adult/day.

A preparations of the above formulation are tablets, pills, powders, granules, capsuls and injectable form. Production of the formula can be made by adding various kinds of carriers, for example fillers such as starch, lactose, sugars, mannit, carboxylmethyl cellulose, corn starch or inorganic salt, binders such as starch, dextrin, arabic gum, gelatin, hydroxylpropyl starch, methylcellulose, sodium carboxymethyl cellulose, hydroxypropyl cellulose, crystalline cellulose, ethyl cellulose, polyvinylpyrrolidone and macrogol, disintegrators such as starch, hydroxypropyl starch, carboxylpropyl starch, sodium carboxymethyl cellulose and hydroxypropyl cellulose, surface active agents such as sodium lauryl sulfate, soybean lecithin, sucrose fatty acid ester and polysorbate 80, lubricants such as talc, wax, hydrogenated vegetable oil, sucrose fatty acid ester, magnesium stearate and calcium stearate, fluidity stimulant, correctives, coloring agents and perfume.

The compound of the present invention (1) or nontoxic salt thereof can be made suspension, emulsion, syrup or elixirs. Parenteral formulation can be contained as diluting agent such as injectable distilled water, physiological saline, glucose solution, injectable vegetable oil, propylene glycol and polyethyleneglycol. If necessary, bacetricides, antiseptics, stabilizers, tonicity agents and soothing agents.

Effect of the invention

Binding activities of a compound of the present invention (1) or notoxic salt thereof to σ-receptor are assayed by the following methods. Results are shown Tables 1 and 2.

(A) Binding activities of the compound of the present invention on σ-receptor are assayed by a method described in E. Weber et al., Proc. Natl. Acad. Sci. U.S.A. vol. 83, 8784-8788 (1986).

Immediately after decapitation of Sprague-Dowley rat, male, 7 weeks age, supplied by Charles River Inc., brains were collected and whole brains except cerebella were homogenized with 50 mM Tris-HCl buffer solution (pH 8.0. TH buffer solution) and centrifuged at 700 xg for 10 minutes. The supernatant was centrifuged at 48,000 xg for 15 minutes, and precipitates were suspended in TH buffer solution which was incubated at 37°C for 20 minutes. The suspension was centrifuged at 48,000 xg for 15 minutes and the thus obtained precipitates were suspended in TH buffer solution to prepare a membrane standard preparation.

The standard preparation of membrane (approx. 600 μg protein) and [$^3$H] 1,3-di(2-tolyl) guanidine (DTG, New England Nuclear Corp. )(final concentration : 3nM) were reacted at 25°C for 60 minutes, and the reaction was stopped by suction filtering through Whatman GF/C filter. Radioactivity adsorbed on the filter was measured by Scintillation Counter, and the value obtained was set as total binding amount. Non specific binding amount (NB) was set up by measuring the assay mixture with adding 10 μM haloperidol. Binding assay of the sample was performed by adding the sample in place of adding haloperidol in the assay system to obtain the assaying result (DTB).

(B) Ki-value (affinity of the drug to receptor)

Binding inhibitory rate of the sample at constant concentration was calculated by the following equation.

$$\text{Binding inhibition rate (\%)} = [1-(DTB-NB) \div (TB-NB)] \times 100$$

Binding inhibition rates at various concentrations from high to low concentrations were measured in each sample, and plotted a logarithmic value of concentration on a transverse and binding inhibition rate on an ordinate, then drawing a curve by the nonlinear method of least squares to obtain $IC_{50}$ value.

Ki value is calculated by the following equation.

$$Ki = (IC_{50}) \div [1 + (L)/Kd]$$

wherein (L) is a concentration of radioactive ligand (3nM) in the experiment. Kd is concentration of the affinity of radioactive ligand for receptor (10.6 nM) and $IC_{50}$ is a concentration of drug which inhibits at 50 % for binding receptor and radioactive ligand.

Table 1

| Binding activity for σ-receptor | |
|---|---|
| Compound numbered in Examples (hydrochloride) | Sigma [$^3$H] DTG Ki (nM) |
| 1 | 0.26 |
| 2 | 0.39 |
| 3 | 0.53 |
| 4 | 0.28 |
| 6 | 0.16 |
| 7 | 0.57 |
| 8 | 0.50 |
| 9 | 3.72 |
| 10 | 0.92 |
| 11 | 3.63 |
| 12 | 2.53 |
| 13 | 6.37 |
| 14 | 0.32 |
| 15 | 0.32 |

Table 1 (continued)

| Binding activity for σ-receptor | |
|---|---|
| Compound numbered in Examples (hydrochloride) | Sigma [$^3$H] DTG Ki (nM) |
| 16 | 0.41 |
| 18 | 2.23 |
| 19 | 0.87 |
| 20 | 4.16 |
| 21 | 0.88 |
| 23 | 0.44 |
| 24 | 0.67 |
| 25 | 0.58 |
| 27 | 1.60 |

Table 2

| Binding activity for σ-receptor | |
|---|---|
| Compound numbered in Examples (hydrochloride) | Sigma [$^3$H] DTG Ki (nM) |
| 45 | 2.22 |
| 46 | 8.57 |
| 47 | 16.10 |
| 48 | 15.05 |
| 49 | 9.00 |
| 50 | 28.32 |
| 51 | 31.82 |
| 52 | 79.54 |
| 53 | 21.50 |
| 54 | 13.35 |
| 55 | 7.04 |

As shown hereinabove, the compound of the present invention (1) and nontoxic salt thereof has strong affinity to σ-receptor and has no affinity to the other receptors.

Pharmacological action of the compound of the present invention (1) is explained hereinbelow.

Suppressive activity of apomorphine induced climbing

Administering dopamine agonist, apomorphine in mouse resulted various abnormal movings, and especially, a climbing the cage has been known to be correlated with human schizophrenia. Therefore, a drug showing suppressive activity for apomorphine induced climbing in mice can be presumed to be effective as antischizophrenia activity on humans.

Compounds of the present invention were administered orally or intraperitoneally in ICR mice, male (Charles River Corp.), and after 30 minutes apomorphine 2 mg/kg was administered subcutaneously. Climbing behaviors of 3 minutes after 20-23 minutes of apomorphine administration were observed. Climbing behavior was observed by entering the mice in stainless steel made wire netting cage and measuring a time remaining above the upper part of half part of the cage at observing. A climbing time of apomorphine single administered group is set as 100 % and shortened time by administering the drug is shown in suppression rate.

Table 3

| Suppressive action on apomorphine induced climbing | |
|---|---|
| Sample | Suppression rate (mg/kg, p.o) |
| Compound numbered as Example No. | |
| 1 (hydrochloride) | 37 % (30) |
| 15 (hydrochloride) | 78 % (10) |
| 16 (hydrochloride) | 79 % (30) |
| 21 (hydrochloride) | 54 % (30) |
| 23 (hydrochloride) | 69 % (10) |
| 24 (hydrochloride) | 78 % (10) |
| 25 (hydrochloride) | 43 % (30) |
| 28 (hydrochloride) | 74 % (30) |

Table 4

| Suppressive action on apomorphine induced climbing | |
|---|---|
| Sample | Suppression (mg/kg, i.p.) |
| Compound numbered as Example No. | |
| 46 (hydrochloride) | 45 % (10) |
| 50 (hydrochloride) | 50 % (1) |
| Control | |
| Chlorpromazine | 40 % (1) |

As shown in Tables 3 and 4, the compound of the present invention (1) suppressed apomorphine induced climbing. These results indicated that the compound of the present invention (1) has strong anti schizophrenia activity.

As clearly shown in the experimental results hereinbefore, the compound of the present invention (1) or nontoxic salt thereof has strong affinity to σ-receptor without showing affinity to the other receptors. Further the compound is effective for suppressing apomorphine induced climbing, which is used for evaluation of schizophrenia, therefore, it is useful for treatment of the diseases relating to σ-receptor, for example treatment of schizophrenia.

<u>Examples</u>

The present invention is explained in details by the referential examples and examples hereinbelow, however the present invention is not limited within these examples. Mass spectrum (MS) data and Nuclear Magnetic Resonance spectrum data ($^1$H-NMR) of the compounds obtained by these examples are shown in Tables 5-7.

Referential example 1

Production of (1S)-1,8,8-trimethyl-3-azabicyclo[3.2.1]octan-2-one:

(1S)-camphor 8.31g and hydroxylamine-O-sulphonic acid 9.25g in acetic acid 200 ml were refluxed under heating for 7 hours. The reaction mixture was concentrated in vacuo and the residue dissolved in chloroform was washed with saturated aqueous sodium bicarbonate solution. Organic layer was dried by anhydrous sodium sulfate, which was thereafter removed by filtration, then the filtrate was concentrated in vacuo. The thus obtained residue was purified by means of silica gel column chromatography (Wako gel™, C200, chloroform) to obtain the product.
Yield: 5.57 g (Yield 56 %)

$[\alpha]_D^{25}$ =+25.0° (c=1, methanol)

Referential example 2

Production of (1S)-1,8,8-trimethyl-3-azabicyclo[3.2.1]octane:

(1S)-1,8,8-trimethyl-3-azabicyclo[3.2.1]octan-2-one 1.93g was added in tetrahydrofuran (THF) 20 ml solution of lithium aluminium hydride 0.88g. and the reaction mixture was refluxed under heating for 22 hours. The reaction mixture was cooled and added 6N HCl and 5N NaOH therein to alkalize the mixture. Insoluble material was filtered and the filtrate was extracted with diethyl ether (ether), dried by anhydrous sodium sulfate, which was thereafter removed by filtration, then the filtrate was concentrated in vacuo to obtain the product.
Yield: 1.70 g (Yield 97 %)

$[\alpha]_D^{25}$ =-17.2° (c=1, methanol)

Example 1

Production of (1S)-3-[2-(1-adamantyl)ethyl]-1,8,8-trimethyl-3-azabicyclo [3.2.1] octane:

Triethylamine 0.43 ml was added in a methylene chloride 20 ml solution of (1S)-1,8,8-trimethyl-3-azabicyclo [3.2.1] octane hydrochloride 490 mg, and stirred at 0°C for 1 hour. 1-adamantyl acetic acid 552 mg and 1-ethyl-3-(3' -dimethylaminopropyl) carbodiimide hydrochloride (WSC) 595 mg were added therein, then the reaction mixture was gradually changed to room temperature and stirred for 23 hours. The reaction mixture was washed with 10 % sodium hydroxide and dried by anhydrous sodium sulfate. After filtering the drying agent, the filtrate was concentrated in vacuo to obtain crude (1S)-3-(1-adamantylacetyl)-1,8,8-trimethyl-3-azabicyclo [3.2.1] octane. The concentrate was added to THF 25 ml solution of lithium aluminium hydride 196 mg and refluxed for 1 hour. The reaction mixture was cooled, then added ether, water and 10 % aqueous sodium hydroxide thereto, and filtered the insoluble material. The filtrate was extracted with ether and dried by anhydrous sodium sulfate. After filtering the drying agent, a residue obtained by concentrating the filtrate in vacuo was purified by means of flash column chromatography (Merck, silica gel 60 : 230-600 mesh, chloroform) to obtain the product.
Yield: 737 mg (Yield 91 %)

Two excess molar amount of methanol solution of hydrogen chloride was added to the thus obtained product, concentrated and crystallized by adding diethyl ether to obtain the hydrochloride.

$[\alpha]_D^{25}$ =-8.6° (c=0.5, methanol)

Example 2

Production of (1S)-3-[2-(1-norbornyl)ethyl]-1,8,8-trimethyl-3-azabicyclo [3,2,1] octane:

Triethylamine 0.43 ml was added in a methylene chloride 20 ml solution of (1S)-1,8,8-trimethyl-3-azabicyclo [3.2.1] octane hydrochloride 485 mg, and stirred at 0°C for 40 min. 2-norbornyl acetic acid 0.41 ml and WSC 588 mg were added therein, then the reaction mixture was gradually changed to room temperature and stirred for 23 hours. The reaction mixture was concentrated in vacuo and the concentrate was dissolved in ether, then washed with 5N sodium hydroxide and dried by anhydrous sodium sulfate. After filtering the drying agent, the filtrate was concentrated in vacuo to obtain crude (1S)-3-(2-norbornylacetyl)-1,8,8-trimethyl-3-azabicyclo [3.2.1] octane. The concentrate was added to THF 20 ml solution of lithium aluminium hydride 194 mg and refluxed for 1 hour. The reaction mixture was cooled, then added ether, water and 10 % aqueous sodium hydroxide thereto, and filtered the insoluble material. The filtrate was extracted with ether and dried by anhydrous sodium sulfate. After filtering the drying agent, a residue obtained by concentrating the filtrate in vacuo was purified by means of flash column chromatography (Merck, silica gel 60 : 230-600

mesh, chloroform-chloroform : acetone = 20 : 1) to obtain the product.
Yield: 618 mg (Yield 88 %)

The product was treated by the same way as of in Example 1 to obtain the hydrochloride.

$[\alpha]_D^{25}$ =-11.3° (c=0.5, methanol)

Example 3

Production of (1S)-3-(2-cyclopentylethyl)-1,8,8-trimethyl-3-azabicyclo[3.2.1] octane:

Triethylamine 0.43 ml was added in a methylene chloride 20 ml solution of (1S)-1,8,8-trimethyl-3-azabicyclo [3.2.1] octane hydrochloride 0.49 g, and stirred at 0°C for 45 min. Cyclopentyl acetic acid 0.36 ml and WSC 0.59g were added therein, then the reaction mixture was gradually changed to room temperature and stirred for 24 hours. The reaction mixture was washed with 10 % sodium hydroxide and dried by anhydrous sodium sulfate. After filtering the drying agent, the filtrate was concentrated in vacuo to obtain crude (1S)-3-cyclopentylacetyl-1,8,8-trimethyl-3-azabicyclo [3.2.1] octane. The concentrate was added to THF 30 ml solution of lithium aluminium hydride 0.19 g and refluxed for 1.5 hours. The reaction mixture was cooled, then added ether, water and 10 % aqueous sodium hydroxide thereto, and filtered the insoluble material. The filtrate was extracted with ether and dried by anhydrous sodium sulfate. After filtering the drying agent, a residue obtained by concentrating the filtrate in vacuo was purified by means of flash column chromatography (Merck, silica gel 60 : 230-600 mesh, chloroform-chloroform : acetone = 20 : 1) to obtain the product.
Yield: 0.58 g (Yield 91 %)

The product was treated by the same way as of in Example 1 to obtain the hydrochloride.

$[\alpha]_D^{25}$ =-11.8° (c=0.5, methanol)

Example 4

Production of (1S)-3-(2-cyclohexylethyl)-1,8,8-trimethyl-3-azabicyclo[3.2.1] octane:

Triethylamine 0.84 ml was added in a methylene chloride 20 ml solution of (1S)-1,8,8-trimethyl-3-azabicyclo [3.2.1] octane hydrochloride 0.95 g, and stirred at 0°C for 30 min. Cyclohexyl acetic acid 0.72 g and WSC 1.35 g were added therein, then the reaction mixture was gradually changed to room temperature and stirred for 25 hours. The reaction mixture was washed with saturated sodium chloride solution and dried by anhydrous sodium sulfate. After filtering the drying agent, the filtrate was concentrated in vacuo to obtain crude (1S)-3-cyclohexylacetyl-1,8,8-trimethyl-3-azabicyclo [3.2.1] octane. The concentrate was added to THF 30 ml solution of lithium aluminium hydride 0.38g and refluxed for 1 hour. The reaction mixture was cooled, then added 6N hydrochloric acid and 1N sodium hydroxide thereto, and filtered the insoluble material. The filtrate was concentrated in vacuo. The thus obtained residue was purified by means of silica gel column chromatography (Wako gel, C200, chloroform : acetone : methanol = 10 : 1 : 0.1) to obtain the product.
Yield: 1.01 g (Yield 77 %)

The product was treated by the same way as of in Example 1 to obtain the hydrochloride.

$[\alpha]_D^{25}$ =-11.5° (c=0.5, methanol)

Example 5

Production of (1R)-3-cyclohexylacetyl-1,8,8-trimethyl-3-azabicyclo[3.2.1] octane:

Triethylamine 0.84 ml was added in a methylene chloride 20 ml solution of (1R)-1,8,8-trimethyl-3-azabicyclo [3.2.1] octane hydrochloride 0.95 g, and stirred at 0°C for 30 min. Cyclohexyl acetic acid 0.72 g and WSC 1.35 g were added therein, then the reaction mixture was gradually changed to room temperature and stirred for 24 hours. The reaction mixture was washed with saturated sodium chloride and dried by anhydrous sodium sulfate. After filtering the drying agent, a residue obtained by concentrating the filtrate in vacuo was purified by means of silica gel column chromatography (Wako gel, C200, chloroform : methanol = 50 : 1) to obtain the product.
Yield: 0.52 g (Yield 37 %)

Example 6

Production of (1R)-3-(2-cyclohexylethyl)-1,8,8-trimethyl-3-azabicyclo[3.2.1] octane:

(1R)-3-cyclohexylacetyl-1,8,8-trimethyl-3-azabicyclo [3.2.1] octane 0.52 g was added to THF 20 ml solution

of lithium aluminium hydride 0.15 g and refluxed for 1 hour. The reaction mixture was cooled, then added water thereto, and filtered the insoluble material. The filtrate was concentrated in vacuo. The thus obtained residue was purified by means of silica gel column chromatography (Wako gel, C200, chloroform : acetone : methanol = 5 : 1 : 0.1) to obtain the product.
Yield: 0.30 g (Yield 61 %)

The product was treated by the same way as of in Example 1 to obtain the hydrochloride.
$[\alpha]_D$ $^{25}$ =-11.6° (c=0.5, methanol)

Referential example 3

Production of methyl cycloheptylidene acetate:

Cycloheptanone 1.18 ml and methyl diethylphosphonoacetate 2.21 ml were dissolved in benzene 10 ml, and sodium hydride (60%, oil dispersion) 0.08 g was added thereto, then stirred at room temperature for 15 hours, thereafter refluxed under heating for 6 hours. Water was added to the reaction mixture and extracted with chloroform, then dried by anhydrous sodium sulfate. The drying agent was removed by filtration and the filtrate was concentrated in vacuo. The thus obtained residue was purifiedby means of silica gel column chromatography (Wako gel, C200. chloroform) to obtain the product.
Yield: 1.20 g (Yield 71 %)

Referential example 4

Production of cycloheptylethyl alcohol:

Methyl cycloheptylidene acetate 1.20 g was added in THF 35 ml solution of lithium aluminium hydride 0.81g, and the reaction mixture was refluxed under heating for 4 hours. The reaction mixture was cooled, and added water and 10 % sodium hydroxide therein, then insoluble material was filtered. The filtrate was extracted with ether and dried by anhydrous sodium sulfate. After removing the drying agent by filtration, the filtrate was concentrated in vacuo. The concentrate was dissolved in methanol 50 ml, added 10 % Pd-C 0.12 g, and being subjected to catalytic hydrogenation under hydrogen atmosphere at normal temperature and normal pressure for 4 hours. Thereafter Pd-C was filtered and the filtrate was concentrated in vacuo to obtain the product.
Yield: 0.45 g (Yield 45 %)

Example 7

Production of (1S)-3-(2-cycloheptylethyl)-1,8,8-trimethyl-3-azabicyclo[3.2.1] octane:

Cycloheptylethyl alcohol 0.45 g was dissolved in methylene chloride 20 ml and cooled to 0 °C, then methanesulfonyl chloride 0.30 ml and triethylamine 0.62 ml were added thereto. Temperature of the reaction mixture was gradually changed to room temperature and stirred for 22 hours. The reaction mixture was concentrated in vacuo to obtain the crude mesylate. (1S)-1,8,8-trimethyl-3-azabicyclo [3.2.1] octane hydrochloride 0.51 g and potassium carbonate 1.10 g were added thereto, then prepared acetonitrile 20 ml solution. The solution was refluxed under heating for 24 hours. In soluble material was removed by filtration and the filtrate was concentrated in vacuo. The thus obtained residue was purified by means of flash column chromatography (Merck, silica gel 60: 230-600 mesh, chloroform) to obtain the product.
Yield: 0.61 g (Yield 82%)

The product was treated by the same way as of in Example 1 to obtain the hydrochloride.
$[\alpha]_D$ $^{25}$ =-11.7° (c=0.5, methanol)

Referential example 5

Production of methyl cyclooctylidene acetate:

Cyclooctanone 2.00g and methyl diethylphosphonoacetate 3.50 ml were dissolved in THF 20 ml, and the solution was cooled to 0 °C. Tertiary butanol 10 ml solution of potassium tertiary butoxide 2.67 g was added dropwise for 20 minutes. Temperature of the reaction mixture was gradually changed to room temperature and stirred for 12 hours. Water was added to the reaction mixture and THF was removed by concentration in vacuo. The thus obtained residue was extracted with ethylacetate and dried by anhydrous sodium sulfate. The drying agent was removed by filtration and the filtrate was concentrated in vacuo. The thus obtained residue was purified by means of silica gel column chromatography (Wako gel, C200, chloroform) to obtain the product.
Yield: 1.33 g (Yield 46 %)

Referential example 6

Productioin of cyclooctyl acetic acid methyl ester:

Methyl cyclooctylidene acetate 0.50 g was dissolved in methanol 5 ml, added 10 % Pd-C 0.10 g thereto, and being subjected to catalytic hydrogenation under hydrogen atmosphere at normal temperature and normal pressure for 3 hours. Thereafter Pd-C was filtered and the filtrate was concentrated in vacuo to obtain the product.
Yield: 0.45 g (Yield 88 %)

Referential example 7

Productioin of cyclooctyl acetic acid:

Cyclooctyl acetic acid methyl ester 0.45 g was dissolved in methanol 10 ml, added 1N sodium hydroxide 4.0 ml thereto and stirred at room temperature for 7 hours. Then reaction mixture was concentrated in vacuo. Saturated aqueous sodium bicarbonate was added to the residue and the mixture was washed with ether. Aqueous layer was cooled, acidified by adding 6N hydrochloric acid and extracted with ethylacetate. The extract was dried by anhydrous sodiumn sulfate. After removing the drying agent by filtration, the filtrate was concentrated in vacuo to obtain the product.
Yield: 0.28 g (Yield 67 %)

Example 8

Production of (1S)-3-(2-cyclooctylethyl)-1,8,8-trimethyl-3-azabicyclo[3,2,1] octane:

Triethylamine 0.24 ml was added in a methylene chloride 10 ml solution of (1S)-1,8,8-trimethyl-3-azabicyclo [3.2.1] octane hydrochloride 0.28 g, and stirred at 0°C for 30 min. Cyclooctyl acetic acid 0.25g and WSC 0.40g were added therein, then temperature of the reaction mixture was gradually changed to room temperature and stirred for 21 hours. The reaction mixture was washed with saturated aqueous sodium chloride solution and dried by anhydrous sodium sulfate. After filtering the drying agent, the filtrate was concentrated in vacuo to obtain crude (1S)-3-cyclooctylacetyl-1,8,8-trimethyl-3-azabicyclo [3.2.1] octane. The concentrate was added to THF 15 ml solution of lithium aluminium hydride 0.12 g and refluxed for 1 hour. The reaction mixture was cooled, then added water and filtered the insoluble material. The filtrate was concentrated in vacuo. The thus obtained residue was purified by means of silica gel column chromatography (Wako gel, C200, chloroform : acetone = 10 : 1) to obtain the product.
Yield: 0.26 g (Yield 61 %)

The product was treated by the same way as of in Example 1 to obtain the hydrochloride.

$[\alpha]_D^{25}$ =-12.9° (c=0.5, methanol)

Example 9

Production of (1S)-3-(4-cyclohexyl-1-butyl)-1,8,8-trimethyl-3-azabicyclo[3.2.1] octane:

4-cyclohexyl-1-butanol 0.52 ml was dissolved in methylene chloride 20 ml and cooled to 0 °C, then methanesulfonyl chloride 0.28 ml and triethylamine 0.50 ml were added thereto. Temperature of the reaction mixture was gradually changed to room temperature and stirred for 6.5 hours. The reaction mixture was concentrated in vacuo to obtain the crude mesylate. (1S)-1,8,8-trimethyl-3-azabicyclo [3.2.1] octane hydrochloride 0.47 g and potassium carbonate 1.04 g were added thereto, then prepared acetonitrile 20 ml solution. The solution was refluxed under heating for 15 hours. Insoluble material was removed by filtration and the filtrate was concentrated in vacuo. The thus obtained residue was purified by means of flash column chromatography (Merck, silica gel 60 : 230-600 mesh, chloroform) to obtain the product.
Yield: 0.55 g (Yield 75 %)

The product was treated by the same way as of in Example 1 to obtain the hydrochloride.

$[\alpha]_D^{25}$ =-13.1° (c=0.5, methanol)

Example 10

Production of (1S)-3-(3-cyclohexylpropyl)-1,8,8-trimethyl-3-azabicyclo[3.2.1] octane:

Triethylamine 0.43 ml was added in a methylene chloride 20 ml solution of (1S)-1,8,8-trimethyl-3-azabicyclo [3.2.1] octane hydrochloride 0.49 g, and stirred at 0 °C for 40 min. 3-cyclohexyl propionic acid 0.44 ml and WSC 0.59 g were added therein, then temperature of the reaction mixture was gradually changed to room temperature and stirred for 45 hours. The reaction mixture was concentrated in vacuo and the residue was dissolved in ether. The ether solution was washed with 10 % sodium hydroxide solution and dried by anhydrous sodium sulfate. After filtering the drying agent, the filtrate was concentrated in vacuo to obtain crude (1S)-3-(3-cyclohexylpropionyl)-1,8,8-trimethyl-3-azabicyclo [3.2.1]octane. The concentrate was added to THF 20 ml solution of lithium aluminium hydride 0.19 g and refluxed for 1.5 hour. The reaction mixture was cooled, then added ether, water and 10 % sodium hydroxide solution, and filtered the insoluble material. The filtrate was extracted with ether and dried by anhydrous sodium sulfate. After removing the drying agent, the filtrate was concentrated in vacuo. The thus obtained residue was purified by means of flash column chromatography (Merck, silicagel 60 : 230-600 mesh, chloroform-chloroform : acetone = 20 : 1) to obtain the product.
Yield: 0.56 g (Yield 79%)

The product was treated by the same way as of in Example 1 to obtain the hydrochloride.

$[\alpha]_D^{25}$ =-11.3° (c=0.5, methanol)

Example 11

Production of (1S)-3-cyclohexylmethyl-1,8,8-trimethyl-3-azabicyclo[3.2.1] octane:

(1S)-1,8,8-trimethyl-3-azabicyclo [3.2.1] octane hydrochloride 0.50 g and cyclohexylmethyl bromide 0.44 ml were dissolved in acetonitrile 25 ml, added potassium carbonate 1.10 g thereto and refluxed under heating for 26 hours. Insoluble material was removed from the reaction mixture by filtration and the filtrate was concentrated in vacuo. The thus obtained residue was purified by means of flash column chromatography (Merck, silica gel 60 : 230-600 mesh, chloroform-chloroform : aceton = 20 : 1) to obtain the product.
Yield: 0.66 g (Yield 100 %)

The product was treated by the same way as of in Example 1 to obtain the hydrochloride.

$[\alpha]_D^{25}$ =-11.0° (c=0.5, methanol)

Example 12

Production of (1R)-3-cyclohexylmethyl-1,8,8-trimethyl-3-azabicyclo[3.2.1] octane:

(1R)-1,8,8-trimethyl-3-azabicyclo [3.2.1] octane 3.00 g and cyclohexylmethyl bromide 5.48 ml were dissolved in acetonitrile 50 ml, added potassium carbonate 6.78 g thereto and refluxed under heating for 39 hours. Insoluble material was removed from the reaction mixture by filtration and the filtrate was concentrated in vacuo. The thus obtained residue was purified by means of silica gel column chromatography (Wako gel, C200, chloroform) to obtain the product.
Yield: 4.62 g (Yield 95 %)

The product was treated by the same way as of in Example 1 to obtain the hydrochloride.

$[\alpha]_D^{25}$ =+13.0° (c=0.5, methanol)

Example 13

Production of (1S)-3-cyclohexyl-1,8,8-trimethyl-3-azabicyclo[3.2.1] octane:

(1S)-1,8,8-trimethyl-3-azabicyclo [3.2.1] octane hydrochloride 0.12 g and cyclohexyl bromide 0.092 ml were dissolved in acetonitrile 5 ml, added potassium carbonate 0.26g thereto and refluxed under heating for 51 hours. Insoluble material was removed from the reaction mixture by filtration and the filtrate was concentrated in vacuo. The thus obtained residue was purified by means of flash column chromatography (Merck, silica gel 60 : 230-600 mesh, chloroform-chloroform : acetone = 20 : 1) to obtain the product.
Yield: 32.0 mg (Yield 22 %)

The product was treated by the same way as of in Example 1 to obtain the hydrochloride.

Example 14

Production of (1R)-3-[2-(1-adamantyl)ethyl]-1,8,8-trimethyl-3-azabicyclo[3.2.1] octane:

(1R)-1,8,8-trimethyl-3-azabicyclo [3.2.1] octane hydlrochloride 0.95 g (5.0 mM) and 1-adamantyl acetic acid 1.17g (6.0 mM) were treated by the same method as of the compound in Example 1 and the resulted compound was purifed by means of silica gel column chromatography (Wako gel, C200, chloroform : acetone = 20 : 1) to obtain the product.
Yield: 1.21 g (Yield 77 %)

The product was treated by the same way as of in Example 1 to obtain the hydrochloride.
$[\alpha]_D{}^{25}$ =+9.2 ° (c=0.5, methanol)

Referential example 8

Production of methyl 2-adamantylidene acetate:

Methanol 500 ml solution of 2-adamantanone 115.00 g (0.77 mole) and methyl-diethylphosphonoacetate 210.78 ml (1.15 mole) was cooled to 0°C. and 28 % methanol solution 371.7 g of sodium methylate was added dropwise. Temperature of the reaction mixture was gradually changed to room temperature and stirred for 4 hours. The reaction mixture was concentrated in vacuo and water was added to the thus obtained residue, then extracted with ethyl acetate, and dried by anhydrous sodium sulfate. The drying agent was removed by filtration and the filtrate was concentrated in vacuo. The thus obtained residue was purified by means of silica gel column chromatography (Wako gel, C200, n-hexane : ethyl acetate = 20 : 1) to obtain the product.
Yield: 157.70 g (Yield 100 %)

Referential example 9

Productioin of 2-adamantyl acetic acid methyl ester:

10% Pd-C 2.90g and ammonium formate 178 g (2.8 mole) were added to methanol 1150 ml solution of methyl 2-adamantylidene acetate 145g (0.74 mole) and stirred at room temperature for 3 hours. Pd-C was filtered and the filtrate was concentrated in vacuo. Water was added to the thus obtained residue, extracted with ethyl acetate and dried by anhydrous sodium sulfate. The drying agent was removed by filtration and the filtrate was concentrated in vacuo to obtain the product.
Yield: 146.41 g (Yield 100 %)

Referential example 10

Productioin of 2-adamantyl acetic acid:

2.5N sodium hydroxide 433 ml was added to methanol 1125 ml solution of 2-adamantyl acetic acid methyl ester 150g (0.72 mole) and stirred at room temperature for 6 hours. The reaction mixture was concentrated in vacuo, and aqueous sodium bicarbonate was added to the residue and the mixture was washed with ether. Aqueous layer was adjusted to pH 1 by adding 12N hydrochloric acid and extracted with ethylacetate. The extract was dried by anhydrous sodiumn sulfate. After removing the drying agent by filtration, the filtrate was concentrated in vacuo to obtain the product.
Yield: 129.39 g (Yield 93 %)

Example 15

Production of (1S)-3-[2-(2-adamantyl)ethyl]-1,8,8-trimethyl-3-azabicyclo[3.2.1] octane:

Triethylamine 0.69 ml was added in a methylene chloride 20 ml solution of (1S)-1,8,8-trimethyl-3-azabicyclo [3.2.1] octane hydrochloride 0.78 g (4.1 mM), and stirred at 0°C for 30 min. 2-adamantyl acetic acid 0.80g (4.1 mM) and WSC 1.11 g (5.79 mM) were added therein, then temperature of the reaction mixture was gradually changed to room temperature and stirred for 23 hours. The reaction mixture was washed with saturated aqueous sodium chloride solution and dried by anhydrous sodium sulfate. After filtering the drying agent, the filtrate was concentrated in vacuo to obtain crude (1S)-3-(2-adamantylacetyl)-1,8,8-trimethyl-3-azabicyclo [3.2.1] octane. The concentrate was added to

THF 50 ml solution of lithium aluminium hydride 0.47 g (12 mM) and refluxed for 2 hours. The reaction mixture was cooled, added water and filtered the insoluble material. The filtrate was concentrated in vacuo. The thus obtained residue was purified by means of silica gel column chromatography (Wako gel, C200, chloroform : acetone = 10 : 1-5 : 1) to obtain the product.
Yield: 1.26 g (Yield 97 %)

The product was treated by the same way as of in Example 1 to obtain the hydrochloride.
$[\alpha]_D^{25}$ =-10.2° (c=0.5, methanol)

Example 16

Production of (1R)-3-[2-(2-adamantyl)ethyl]-1,8,8-trimethyl-3-azabicyclo [3.2.1] octane:

(1R)-1,8,8-trimethyl-3-azabicyclo [3.2.1] octane hydlrochloride 0.38 g (2.0 mM) and 2-adamantyl acetic acid 0.43 g (2.2 mM) were treated by the same method as of the compound in Example 15 and the resulted compound was purified by means of silica gel column chromatography (Wako gel, C200, chloroform : acetone = 10 : 1) to obtain the product.
Yield: 0.57 g (Yield 91 %)

The product was treated by the same way as of in Example 1 to obtain the hydrochloride.
$[\alpha]_D^{25}$ =+10.5° (c=0.5, methanol)

Referential example 11

Production of 2-adamantyl ethanol:

2-admantyl acetic acid methyl ester 0.57 g (2.47 mM) was added to diethyl ether 20 ml solution of lithium aluminium hydride 0.16 g (4.2 mM). and the reaction mixture was refluxed under heating for 2 hours. The reaction mixture was cooled and added water. Insoluble material was filtered and the filtrate was extracted with ether, then dried by anhydrous sodium sulfate, which was thereafter removed by filtration. The filtrate was concentrated in vacuo to obtain the product.
Yield: 0.23 g (Yield 47 %)

Example 17

Production of (1S)-3-[2-(2-adamantyl)ethyl]-1,8,8-trimethyl-3 azabicyclo[3.2.1] octane:

Methylene chloride 10 ml solution of 2-adamantyl ethanol 0.64 g (3.6 mM) was cooled to 0°C, then methanesulfonyl chloride 0.42 ml(5.4 mM) and triethylamine 1.00 ml (7.17 mM) were added thereto. Temperature of the reaction mixture was gradually changed to room temperature and stirred for 15 hours. The reaction mixture was concentrated in vacuo to obtain the crude mesylate. (1S)-1,8,8-trimethyl-3-azabicyclo [3.2.1] octane hydrochloride 0.68 g (3.6 mM) and potassium carbonate 1.49 g (10.8 mM) were added thereto, then prepared acetonitrile 30 ml solution. The solution was refluxed under heating for 18 hours. Insoluble material was removed by filtration and the filtrate was concentrated in vacuo. The thus obtained residue was purified by means of silica gel column chromatography (Wako gel, C200, chloroform : acetone = 20 : 1-5 : 1) to obtain the product.
Yield: 0.59 g (Yield 53 %)

The product was treated by the same way as of in Example 1 to obtain the hydrochloride.

Example 18

Production of (1S)-3-[2-(4-methylcyclohexyl)ethyl]-1,8,8-trimethyl-3-azabicyclo [3.2.1] octane:

Triethylamine 0.84 ml was added in a methylene chloride 20 ml solution of (1S)-1,8,8-trimethyl-3-azabicyclo [3.2.1] octane hydrochloride 0.95 g, and stirred at 0°C for 30 min. 4-methylcyclohexyl acetic acid 0.94 g and WSC 1.35 g were added therein, then temperature of the reaction mixture was gradually changed to room temperature and stirred for 24 hours. The reaction mixture was washed with saturated sodium chloride solution and dried by anhydrous sodium sulfate. After filtering the drying agent, the filtrate was concentrated in vacuo to obtain crude (1S)-3-(4-methylcyclohexyl acetyl)-1,8,8-trimethyl-3-azabicyclo [3.2.1] octane. The concentrate was added to THF 30 ml solution of lithium aluminium hydride 0.39 g and refluxed for 1 hour. The reaction mixture was cooled, then added water, and filtered the insoluble material. The filtrate was concentrated in vacuo. The thus obtained residue was purified by means of silica gel column

chromatography (Wako gel, C200, chloroform : acetone = 10 : 1), to obtain the product.
Yield: 1.37 g (Yield 100 %)

The product was treated by the same way as of in Example 1 to obtain the hydrochloride.
$[\alpha]_D^{25}$ =-11.0° (c=0.5, methanol)

Example 19

Production of (1S)-3-[2-(3-methyl-1-adamantyl)ethyl]-1,8,8-trimethyl-3-azabicyclo [3.2.1] octane:

Triethylamine 0.53 ml was added in a methylene chloride 15 ml solution of (1S)-1,8,8-trimethyl-3-azabicyclo [3.2.1] octane hydrochloride 0.51 g, and stirred at 0°C for 30 min. 3-methyl-1-adamantyl acetic acid 0.68 g and WSC 0.73 g were added therein, then temperature of the reaction mixture was gradually changed to room temperature and stirred for 23.5 hours. The reaction mixture was washed with saturated aqueous sodium chloride and dried by anhydrous sodium sulfate. After filtering the drying agent, the filtrate was concentrated in vacuo to obtain crude (1S)-3-(3-methyl-1-adamantylacetyl)-1,8,8-trimethyl-3-azabicyclo [3.2.1] octane. The concentrate was added to THF 15 ml solution of lithium aluminium hydride 0.21 g and refluxed under heating for 3 hours. The reaction mixture was cooled, then added water thereto, and removed the insoluble material by filtration. The filtrate was concentrated in vacuo. The thus obtained residue was purified by means of flash column chromatography (Merck, silica gel 60 : 230-600 mesh, chloroform) to obtain the product.
Yield: 0.63 g (Yield 71 %)

The product was treated by the same method as of in Example 1 to obtain the hydrochloride.
$[\alpha]_D^{25}$ =-9.4 ° (c=0.5, methanol)

Example 20

Production of (1S)-3-(3-noradamantylmethyl)-1,8,8-trimethyl-3-azabicyclo[3.2.1] octane:

Triethylamine 0.096 ml was added in a methylene chloride 5 ml solution of (1S)-1,8,8-trimethyl-3-azabicyclo [3.2.1] octane hydrochloride 100 mg, and stirred at 0°C for 30 min. WSC 0.132 g and 3-noradamantan carboxylic acid 96.5 mg were added therein, then temperature of the reaction mixture was gradually changed to room temperature and stirred for 15.5 hours. The reaction mixture was washed with saturated sodium chloride solution and dried by anhydrous sodium sulfate. After filtering the drying agent, the filtrate was concentrated in vacuo to obtain crude (1S)-3-(3-noradamantyl carbonyl)-1,8,8-trimethyl-3-azabicyclo [3.2.1] octane. The concentrate was added to THF 10 ml solution of lithium aluminium hydride 24.0 mg and refluxed under heating for 1.5 hours. The reaction mixture was cooled, then added water, and removed the insoluble material by filtration. The filtrate was concentrated in vacuo. The thus obtained residue was purified by means of silica gel column chromatography (Wako gel, C200, chloroform) to obtain the product.
Yield: 118.2 mg (Yield 78 %)

The product was treated by the same way as of in Example 1 to obtain the hydrochloride.
$[\alpha]_D^{25}$ =-9.4 ° (c=0.5, methanol)

Referential example 12

Production of methyl 9-bicyclo [3.3.1] nonylidene acetate:

Sodiuim hydride (60%, oil dispersion) 0.30g was added to 1,2-dimethoxyethane (DME) 10 ml solution of bicyclo [3.3.1] nonan-9-one 0.50 g and methyldiethylphosphono acetate 1.00 ml and stirred at room temperature for 3 hours. Water was added to the reaction mixture to decompose unreacted sodium hydride, and DME was concentrated in vacuo. The residue was extracted with chloroform and dried by anhydrous sodium sulfate. After removing the drying agent, the filtrate was concentrated. The residue was purified by means of silica gel column chromatography (Wako gel, C200, n-hexane : ethyl acetate = 20 : 1) to obtain the product.
Yield: 0.70 g (Yield 100 %)

Referential example 13

Production of 9-bicyclo [3.3.1] nonylacetic acid methyl ester:

10% Pd-C 0.07 g was added in a methanol solution 10 ml of methyl 9-bicyclo[3.3.1] nonylidene acetate 0.70 g, and the reaction mixture was subjected to catalytic hydrogenation under hydrogen atmosphere at normal temperature and normal pressure for 2 hours. Pd-C was removed from the reaction mixture by filtration and the filtrate was concentrated in vacuo to obtain the product.
Yield: 0.67 g (Yield 94 %)

Referential example 14

Production of 9-bicyclo [3.3.1] nonylacetic acid:

1N aqueous sodium hydroxide 5.2 ml was added to methanol 15 ml solution of 9-bicyclo [3.3.1] nonylacetic acid methyl ester 0.67 g and stirred at room temperature for 5 hours. The reaction mixture was concentrated in vacuo, and saturated aqueous sodium bicarbonate was added to the residue and the mixture was washed with ether. Aqueous layer was adjusted to pH 1 by adding 12N hydrochloric acid and extracted with ethyl acetate. The extract was dried by anhydrous sodium sulfate. After removing the drying agent by filtration, the filtrate was concentrated in vacuo to obtain the product.
Yield: 0.44 g (Yield 71 %)

Example 21

Production of (1S)-3-[2-(bicyclo [3.3.1] nonan-9-yl)-1-ethyl]-1,8,8-trimethyl-3-azabicyclo [3.2.1] octane:

Triethylamine 0.12 ml was added in a methylene chloride 10 ml solution of (1S)-1,8,8-trimethyl-3-azabicyclo[3.2.1]octane hydrochloride 100 mg and stirred at 0°C for 30 min. 9-bicyclo [3.3.1] nonylacetic acid 97.0 mg and WSC 0.15 g were added therein, then temperature of the reaction mixture was gradually changed to room temperature and stirred for 19 hours. The reaction mixture was washed with saturated aqueous sodium chloride solution and dried by anhydrous sodium sulfate. After filtering the drying agent, the filtrate was concentrated in vacuo to obtain crude (1S)-3-(bicyclo [3.3.1] nonan-9-yl) acetyl-1,8,8-trimethyl-3-azabicyclo [3.2.1] octane. The concentrate was added to THF 10 ml solution of lithium aluminium hydride 41 mg and refluxed under heating for 1 hour. The reaction mixture was cooled, then water was added thereto and the insoluble material was removed by filtration. The filtrate was concentrated in vacuo. The thus obtained residue was purified by means of silica gel column chromatography (Wako gel, C200, chloroform : acetone = 10 : 1-5 : 1) to obtain the product.
Yield: 110 mg (Yield 69 %)

The product was treated by the same way as of in Example 1 to obtain the hydrochloride.

$[\alpha]_D^{25}$ =-17.9° (c=0.5, methanol)

Example 22

Production of (1S)-3-(1-adamantylmethyl)-1,8,8-trimethyl-3-azabicyclo[3.2.1] octane:

Triethylamine 0.096 ml was added in a methylene chloride 5 ml solution of (1S)-1,8,8-trimethyl-3-azabicyclo [3.2.1] octane hydrochloride 100 mg, and stirred at 0°C for 30 min. WSC 0.13 g and 1-adamantan carboxylic acid 0.11 g were added therein, then temperature of the reaction mixture was gradually changed to room temperature and stirred for 15 hours. The reaction mixture was washed with saturated sodium chloride solution and dried by anhydrous sodium sulfate. After filtering the drying agent, the filtrate was concentrated in vacuo to obtain crude (1S)-3-(1-adamantyl carbonyl)-1,8,8-trimethyl-3-azabicyclo [3.2.1] octane. The concentrate was added to THF 10 ml solution of lithium aluminium hydride 24.0 mg and refluxed under heating for 1.5 hour. The reaction mixture was cooled, then added water, and removed the insoluble material by filtration. The filtrate was concentrated in vacuo. The thus obtained residue was purified by means of silica gel column chromatography (Wako gel, C200, chloroform) to obtain the product.
Yield: 71.2 mg (Yield 45 %)

The product was treated by the same way as of in Example 1 to obtain the hydrochloride.

$[\alpha]_D^{25}$ =-10.5° (c=0.5, methanol)

Referential example 15

Production of methyl 5-hydroxy-2-adamantylidene acetate:

DME 5 ml solution of methyl diethylphosphono acetate 0.200 ml was cooled to -10 °C. Sodium hydride (60 %, oil dispersion) 54.3 mg was added thereto and stirred at the same temperature for 1 hour. DME 5 ml solution of 5-hydroxy-2-adamantanone 150 mg was added thereto and stirred at the same temperature for 1.5 hours. Water was

added to the reaction mixture and DME was concentrated in vacuo. The residue was extracted with chloroform and dried by anhydrous sodium sulfate. After removing the drying agent, the filtrate was concentrated. The residue was purified by means of silica gel column chromatography (Wako gel, C200, chloroform) to obtain the product.
Yield: 180 mg (Yield 90 %)

Referential example 16

Production of 5-hydroxy-2-adamantylacetic acid methyl ester:

10% Pd-C 18.0 mg was added to a methanol solution 5 ml of methyl 5-hydroxy-2-adamantylidene acetate 179.9 mg, and the reaction mixture was subjected to catalytic hydrogenation under hydrogen atmosphere at normal temperature and normal pressure for 2.5 hours. Pd-C was removed from the reaction mixture by filtration and the filtrate was concentrated in vacuo to obtain the colorless oily product.
Yield: 166.3 mg (Yield 92 %)

Referential example 17

Production of 5-hydroxy-2-adamantylacetic acid:

2.5N aqueous sodium hydroxide 0.49 ml was added to methanol 10 ml solution of 5-hydroxy-2-adamantylacetic acid methyl ester 181.5 mg and stirred at room temperature for 14 hours. The reaction mixture was concentrated in vacuo, and aqueous sodium bicarbonate was added to the residue and the mixture was washed with ether. Aqueous layer was adjusted to pH 1 by adding 12N hydrochloric acid and extracted with ethyl acetate. The extract was dried by anhydrous sodium sulfate. After removing the drying agent by filtration, the filtrate was concentrated in vacuo to obtain the product.
Yield: 150 mg (Yield 88 %)

Example 23

Production of (1S)-3-[2-(5-hydroxy-2-adamantyl)ethyl]-1,8,8-trimethyl-3-azabicyclo [3.2.1] octane:

Triethylamine 0.38 ml was added to a methylene chloride 15 ml solution of (1S)-1,8,8-trimethyl-3-azabicyclo [3.2.1] octane hydrochloride 0.44 g, and stirred at 0°C for 30 min. WSC 0.53 g and 5-hydroxy-2-adamantyl acetic acid 0.44 g were added therein, then the temperature of the reaction mixture was gradually changed to room temperature and stirred for 14.5 hours. The reaction mixture was washed with saturated sodium chloride solution and dried by anhydrous sodium sulfate. After filtering the drying agent, the filtrate was concentrated in vacuo to obtain crude (1S)-3-(5-hydroxy-2-adamantyl acetyl)-1,8,8-trimethyl-3-azabicyclo [3.2.1] octane. The concentrate was added to THF 30 ml solution of lithium aluminium hydride 95.5 mg and refluxed under heating for 1.5 hours. The reaction mixture was cooled, then added water, and removed the insoluble material by filtration. The filtrate was concentrated in vacuo. The thus obtained residue was purified by means of silica gel column chromatography (Wako gel, C200, chloroform : methanol = 150 : 1) to obtain the product.
Yield: 556 mg (Yield 79 %)

The product was treated by the same way as of in Example 1 to obtain the hydrochloride.

$[\alpha]_D^{25}$ =-10.9° (c=0.5, methanol)

Referential example 18

Production of 5-methoxy-2-adamantanone:

THF 5 ml solution of 5-hydroxy-2-adamantanone 100mg and methyl iodide 0.113 ml was cooled to 0°C. Sodium hydride (60 % oil dispersion) 48.2 mg was added to thereto and temperature of the reaction mixture was gradually changed to room temperature and stirred for 25 hours. Water was added to the reaction mixture and THF was concentrated in vacuo. The thus obtained residue was extracted with chloroform and dried by anhydrous sodium sulfate. After removing the drying agent, the filtrate was concentrated in vacuo. The residue was purified by means of silica gel column chromatography (Wako gel, C200 n-hexane : ethyl acetate = 10 : 1) to obtain the product.
Yield: 57.7 mg (Yield 54 %)

Referential example 19

Production of methyl 5-methoxy adamantylidene acetate:

DME 2 ml solution of 5-methoxy-2-adamantanone 57.7 mg and methyl diethylphosphono acetate 70.6 µl was cooled to 0°C. Sodium hydride (60 %, oil dispersion) 25.6 mg was added thereto and temperature of the reaction mixture was gradually changed to room temperature and stirred for 3.5 hours. Water was added to the reaction mixture to decompose unreacted sodium hydride and DME was concentrated in vacuo. The residue was extracted with chloroform and dried by anhydrous sodium sulfate. After removing the drying agent, the filtrate was concentrated. The residue was purified by means of silica gel column chromatography (Wako gel, C200, n-hexane : ethyl acetate = 10 : 1) to obtain the product.
Yield: 53.2 mg (Yield 70 %)

Referential example 20

Production of 5-methoxy-2-adamantyl acetic acid methyl ester:

10% Pd-C 5.3 mg was added to a methanol solution 2 ml of methyl 5-hydroxy-2-adamantylidene acetate 53.2 mg, and the reaction mixture was subjected to catalytic hydrogenation under hydrogen atmosphere at normal temperature and normal pressure for 1.5 hours. Pd-C was removed from the reaction mixture by filtration and the filtrate was concentrated in vacuo to obtain the product.
Yield: 50.0 mg (Yield 93 %)

Referential example 21

Production of 5-methoxy-2-adamantyl acetic acid:

2.5N aqueous sodium hydroxide 0.13 ml was added to methanol 2 ml solution of 5-methoxy-2-adamantyl acetic acid methyl ester 50.0 mg and stirred at room temperature for 7 hours. The reaction mixture was concentrated in vacuo, and saturated aqueous sodium bicarbonate was added to the residue and the mixture was washed with ether. Aqueous layer was adjusted to pH 1 by adding 12N hydrochloric acid and extracted with ethyl acetate. The extract was dried by anhydrous sodium sulfate. After removing the drying agent by filtration, the filtrate was concentrated in vacuo to obtain the product.
Yield: 36.2 mg (Yield 77 %)

Example 24

Production of (1S)-3-[2-(5-methoxy-2-adamantyl)ethyl]-1,8,8-trimethyl-3-azabicyclo [3.2.1] octane:

Triethylamine 29.3 µl was added to a methylene chloride 1 ml solution of (1S)-1,8,8-trimethyl-3-azabicyclo [3.2.1] octane hydrochloride 33.7 mg and stirred at 0°C for 30 min. 5-methoxy-2-adamantyl acetic acid 36.2 mg and WSC 40.3 mg were added therein, then temperature of the reaction mixture was gradually changed to room temperature and stirred for 20.5 hours. The reaction mixture was washed with saturated aqueous sodium chloride solution and dried by anhydrous sodium sulfate. After filtering the drying agent, the filtrate was concentrated in vacuo to obtain crude (1S)-3-(5-methoxy-2-adamantyl acety)-1,8,8-trimethyl-3-azabicyclo [3.2.1] octane. The concentrate was added to THF 3 ml solution of lithium aluminium hydride 7.4 mg and refluxed under heating for 1.5 hours. The reaction mixture was cooled, then water was added thereto and then insoluble material was removed by filtration. The filtrate was concentrated in vacuo. The thus obtained residue was purified by means of silica gel column chromatography (Wako gel, C200, chloroform : acetone = 20 : 1-10 : 1) to obtain the product.
Yield: 32.1 mg (Yield 58 %)
The product was treated by the same way as of in Example 1 to obtain the hydrochloride.
$[\alpha]_D^{25}$ =-22.5° (c=0.1, methanol)

Example 25

Production of (1S)-3-[2-(5-chloro-2-adamantyl)ethyl]-1,8,8-trimethyl-3-azabicyclo [3.2.1] octane:

Thionyl chloride 5 ml was added to (1S)-3-[2-(5-hydroxy-2-adamantyl) ethyl]-1,8,8-trimethyl-3-azabicyclo

[3.2.1] octane 80.3 mg and refluxed under heating for 4.5 hours. The reaction mixture was concentrated in vacuo, and the residue obtained was alkalified by adding 1N sodium hydroxide, then extracted with chloroform. The extract was dried by anhydrous sodium sulfate. After the drying agent was removed by filtration, the filtrate was concentrated in vacuo. The thus obtained residue was purified by means of silica gel column chromatography (Wako gel, C200, chloroform : acetone = 20 : 1) to obtain the product,
Yield: 44.8 mg (Yield 53 %)

The product was treated by the same way as of in Example 1 to obtain the hydrochloride.
$[\alpha]_D^{25}$ =-12.2° (c=0.5, methanol)

Referential example 22

Production of methyl 5-phenyl-2-adamantylidene acetate:

DME 2 ml solution of 5-phenyl-2-adamantanone 86.1 mg and methyl diethylphosphono acetate 84.0 μl was cooled to 0°C, and sodiuim hydride (60 % oil dispersion) 22.9 mg was added thereto, then stirred at 0°C for 1.5 hours. Water was added to the reaction mixture to decompose unreacted sodium hydride, and DME was concentrated in vacuo. The residue was extracted with chloroform and dried by anhydrous sodium sulfate. After removing the drying agent, the filtrate was concentrated. The residue was purified by means of silica gel column chromatography (Wako gel, C200, n-hexane : ethyl acetate = 30 : 1) to obtain the product.
Yield: 55.3 mg (Yield 52 %)

Referential example 23

Production of 5-phenyl-2-adamantyl acetic acid methyl ester:

10% Pd-C 5.3 mg was added to a methanol solution 2 ml of methyl 5-phenyl-2-adamantylidene acetate 55.3 mg, and the reaction mixture was subjected to catalytic hydrogenation under hydrogen atmosphere at normal temperature and normal pressure for 2.5 hours. Pd-C was removed from the reaction mixture by filtration and the filtrate was concentrated in vacuo to obtain the product.
Yield: 55.7 mg (Yield 100 %)

Referential example 24

Production of 5-phenyl-2-adamantyl acetic acid:

2.5N aqueous sodium hydroxide 0.12 ml was added to methanol 3 ml solution of 5-phenyl-2-adamantyl acetic acid methyl ester 55.7 mg and stirred at room temperature for 14.5 hours. The reaction mixture was concentrated in vacuo, and saturated aqueous sodium bicarbonate was added to the residue and the mixture was washed with ether. Aqueous layer was adjusted to pH 1 by adding 12N hydrochloric acid and extracted with ethyl acetate. The extract was dried by anhydrous sodium sulfate. After removing the drying agent by filtration, the filtrate was concentrated in vacuo to obtain the product.
Yield: 22.6 mg (Yield 43 %)

Example 26

Production of (1S)-3-[2-(5-phenyl-2-adamantyl)ethyl]-1,8,8-trimethyl-3-azabicyclo [3.2.1] octane:

Triethylamine 14.0 μl was added to a methylene chloride 1 ml solution of (1S)-1,8,8-trimethyl-3-azabicyclo [3.2.1] octane hydrochloride 15.9 mg and stirred at 0°C for 30 min. 5-phenyl-2-adamantyl acetic acid 22.6mg and WSC 19.3 mg were added therein, then temperature of the reaction mixture was gradually changed to room temperature and stirred for 64 hours. The reaction mixture was washed with saturated aqueous sodium chloride solution and dried by anhydrous sodium sulfate. After filtering the drying agent, the filtrate was concentrated in vacuo to obtain crude (1S)-3-(5-phenyl-2-adamantylacetyl)-1,8,8-trimethyl-3-azabicyclo [3.2.1] octane. The concentrate was added to THF 3 ml solution of lithium aluminium hydride 3.8 mg and refluxed under heating for 1.5 hours. The reaction mixture was cooled, then water was added thereto and the insoluble material was removed by filtration. The filtrate was concentrated in vacuo. The thus obtained residue was purified by means of silica gel column chromatography (Wako gel, C200, chloroform : acetone = 20 : 1) to obtain the product.
Yield: 7.0 mg (Yield 21 %)

The product was treated by the same way as of in Example 1 to obtain the hydrochloride.

Referential example 25

Production of methyl 4-hydroxy-2-adamantylidene acetate:

DME 20 ml solution of 4-hydroxy-2-adamantanone 536.1 mg and methyl diethylphosphono acetate 0.72 ml was cooled to -10°C, and sodium hydride (60%, oil dispersion) 259 mg was added thereto, then stirred at -10°C for 1.5 hours. Water was added to the reaction mixture to decompose unreacted sodium hydride, and DME was concentrated in vacuo. The residue was extracted with chloroform and dried by anhydrous sodium sulfate. After removing the drying agent, the filtrate was concentrated. The residue was purified by means of silica gel column chromatography (Wako gel, C200 chloroform) to obtain the product.
Yield: 0.51 g (Yield 71 %)

Referential example 26

Production of 4-hydroxy-2-adamantyl acetic acid methyl ester:

10% Pd-C 51 mg was added to a methanol solution 20 ml of methyl 4-hydroxy-2-adamantylidene acetate 0.51 g, and the reaction mixture was subjected to catalytic hydrogenation under hydrogen atmosphere at normal temperature and normal pressure for 1 hour. Pd-C was removed from the reaction mixture by filtration and the filtrate was concentrated in vacuo to obtain the product.
Yield: 0.47 g (Yield 92 %)

Referential example 27

Production of 4-hydroxy-2-adamantyl acetic acid:

2.5N aqueous sodium hydroxide 1.26 ml was added to methanol 15 ml solution of 4-hydroxy-2-adamantyl acetic acid methyl ester 0.47 g and stirred at room temperature for 5.5 hours. The reaction mixture was concentrated in vacuo, and saturated aqueous sodium bicarbonate was added to the residue and the mixture was washed with ether. Aqueous layer was adjusted to pH 1 by adding 12N hydrochloric acid and extracted with ethyl acetate. The extract was dried by anhydrous sodium sulfate. After removing the drying agent by filtration, the filtrate was concentrated in vacuo to obtain the product.
Yield: 0.26 g (Yield 59 %)

Example 27

Production of (1S)-3-[2-(4-hydroxy-2-adamantyl)ethyl]-1,8,8-trimethyl-3-azabicyclo [3.2.1] octane:

Triethylamine 0.23 ml was added to a methylene chloride 5 ml solution of (1S)-1,8,8-trimethyl-3-azabicyclo [3.2.1] octane hydrochloride 0.26 g and stirred at 0°C for 30 min. 4-hydroxy-2-adamantyl acetic acid 0.26 g and WSC 0.31 g were added therein, then temperature of the reaction mixture was gradually changed to room temperature and stirred for 25 hours. The reaction mixture was washed with saturated aqueous sodium chloride solution and dried by anhydrous sodium sulfate. After filtering the drying agent, the filtrate was concentrated in vacuo to obtain crude (1S)-3-(4-hydroxy-2-adamantylacety)-1,8,8-trimethyl-3-azabicyclo [3.2.1] octane. The concentrate was added to THF 25 ml solution of lithium aluminium hydride 70.5 mg and refluxed under heating for 1.5 hours. The reaction mixture was cooled, then water was added thereto and the insoluble material was removed by filtration. The filtrate was concentrated in vacuo. The thus obtained residue was purified by means of silica gel column chromatography (Wako gel, C200, chloroform : methanol = 150 : 1) to obtain the product.
Yield: 160.5 mg (Yield 39 %)

The product was treated by the same way as of in Example 1 to obtain the hydrochloride.

Referential example 28

Production of methyl 4-phenyl-2-adamantylidene acetate:

Sodium hydride (60 %, oil dispersion) 506 mg was added to DME 50 ml solution of 4-phenyl-2-adamantanone 1.43g and methyl diethylphosphono acetate 1.74 ml and stirred at room temperature for 23.5 hours. Ether was added to the reaction mixture and washed with water, then dried by anhydrous sodium sulfate. After removing the drying agent, the filtrate was concentrated. The residue was purified by means of flash silica gel column chromatography (Merck, silica gel 60 : 230-400 mesh, n-hexane : ethyl acetate = 8 : 1) to obtain the product.
Yield: 866 mg (Yield 49 %)

Referential example 29

Production of 4-phenyl-2-adamantyl acetic acid methyl ester:

10% Pd-C 100 mg was added in a methanol solution 15 ml of methyl 4-phenyl-2-adamantylidene acetate 838 mg, and the reaction mixture was subjected to catalytic hydrogenation under hydrogen atmosphere at normal temperature and normal pressure for 1 hour. Pd-C was removed from the reaction mixture by filtration and the filtrate was concentrated in vacuo to obtain the product.
Yield: 838 mg (Yield 96 %)

Referential example 30

Production of 4-phenyl-2-adamantyl acetic acid:

1N aqueous sodium hydroxide 6 ml was added to methanol 20 ml solution of 4-phenyl-2-adamantyl acetic acid methyl ester 838 mg and stirred at room temperature for 13.5 hours. The reaction mixture was concentrated in vacuo and saturated aqueous sodium bicarbonate was added to the residue and the mixture was washed with ether. Aqueous layer was adjusted to pH 1 by adding 12N hydrochloric acid and extracted with methylene chloride. The extract was dried by anhydrous sodium sulfate. After removing the drying agent by filtration, the filtrate was concentrated in vacuo to obtain the product.
Yield: 774 mg (Yield 97 %)

Example 28

Production of (1S)-3-[2-(4-phenyl-2-adamantyl)ethyl]-1,8,8-trimethyl-3-azabicyclo [3.2.1] octane:

Triethylamine 1.00 ml was added in a methylene chloride 30 ml solution of (1S)-1,8,8-trimethyl-3-azabicyclo [3.2.1] octane hydrochloride 815 mg, and stirred at 0°C for 30 min. 4-phenyl-2-adamantyl acetic acid 774 mg and WSC 1.65 g were added therein, then temperature of the reaction mixture was gradually changed to room temperature and stirred for 16.5 hours. The reaction mixture was washed with 1N hydrochloric acid and dried by anhydrous sodium sulfate. After filtering the drying agent, the filtrate was concentrated in vacuo to obtain crude (1S)-3-(4-phenyl-2-adamantylacetyl)-1,8,8-trimethyl-3-azabicyclo [3.2.1] octane. The concentrate was added to THF 30 ml solution of lithium aluminium hydride 229 mg and refluxed under heating for 1 hour. The reaction mixture was cooled, then added water and 2.5N sodium hydroxide thereto, and removed the insoluble material by filtration. The filtrate was concentrated in vacuo. The thus obtained residue was purified by means of flash column chromatography (Merck, silica gel 60 : 230-400 mesh, chloroform) to obtain the product.
Yield: 878 mg (Yield 78 %)
The product was treated by the same method as of in Example 1 to obtain the hydrochloride.
$[\alpha]_D^{25}$ =-8.9 ° (c=0.5, methanol)

Example 29

Production of (1R)-3-[2-(4-phenyl-2-adamantyl)ethyl]-1,8,8-trimethyl-3-azabicyclo [3.2.1] octane:

Example 28 was repeated except that (1R)-1,8,8-trimethyl-3-azabicyclo [3.2.1] octane hydrochloride 0.48 g and 4-phenyl-2-adamantyl acetic acid 0.59 g were used and purification was performed by silica gel column chromatography (Wako gel, C200, chloroform) and the other processes were taken almost same way as of in Example 28 to obtain the product.
Yield: 0.69 g (Yield 82 %)
The product was treated by the same method as of in Example 1 to obtain the hydrochloride.
$[\alpha]_D^{25}$ =+9.5 ° (c=0.5, methanol)

Example 30

Production of (1R)-3-[2-(5-methoxy-2-adamantyl)ethyl]-1,8,8-trimethyl-3-azabicyclo [3.2.1] octane:

Example 24 was repeated except that (1R)-1,8,8-trimethyl-3-azabicyclo [3.2.1] octane hydrochloride 28.4mg and 5-methoxy-2-adamantyl acetic acid 36.9mg were used and purification was performed by silica gel column chromatography (Wako gel, C200, chloroform : acetone = 10 : 1), and the other processes were taken almost same way as of in Example 24 to obtain the product.
Yield: 34.1 mg (Yield 66 %)
The product was treated by the same method as of in Example 1 to obtain the hydrochloride.
$[\alpha]_D^{25}$ =+18.7° (c=0.5 methanol)

Example 31

Production of (1R)-3-[2-(5-hydroxy-2-adamantyl)ethyl]-1,8,8-trimethyl-3-azabicyclo [3.2.1] octane:

Example 23 was repeated except that (1R)-1,8,8-trimethyl-3-azabicyclo [3.2.1] octane hydrochloride 0.15 g and 5-hydroxy-2-adamantyl acetic acid 0.15 g were used and purification was performed by silica gel column chromatography (Wako gel, C200, chloroform : methanol = 100 : 1), and the other processes were taken almost same way as of in Example 23 to obtain the product.
Yield: 0.16 g (Yield 66%)
The product was treated by the same method as of in Example 1 to obtain the hydrochloride.
$[\alpha]_D^{25}$ =+11.2° (c=0.5, methanol)

Example 32

Production of (1R)-3-[2-(5-chloro-2-adamantyl)ethyl]-1,8,8-trimethyl-3-azabicyclo [3.2.1] octane:

Thionyl chloride 5 ml was added to (1R)-3-[2-(5-hydroxy-2-adamantyl) ethyl]-1,8,8-trimethyl-3-azabicyclo[3.2.1]octane 82.8 mg and refluxed under heating for 5 hours. The reaction mixture was concentrated in vacuo, and the residue obtained was alkalified by adding 1N sodium hydroxide, then extracted with chloroform. The extract was dried by anhydrous sodium sulfate. After the drying agent was removed by filtration, the filtrate was concentrated in vacuo. The thus obtained residue was purified by means of silica gel column chromatography (Wako gel, C200, chloroform : acetone = 20 : 1) to obtain the product.
Yield: 48.1 mg (Yield 55 %)
The product was treated by the same method as of in Example 1 to obtain the hydrochloride.
$[\alpha]_D^{25}$ =+13.6° (c=0.5, methanol)

Example 33

Production of (1R)-3-[2-(5-phenyl-2-adamantyl)ethyl]-1,8,8-trimethyl-3-azabicyclo [3.2.1] octane:

Example 26 was repeated except that (1R)-1,8,8-trimethyl-3-azabicyclo [3.2.1]octane hydrochloride 37.2 mg and 5-phenyl-2-adamantyl acetic acid 53.0 mg were used and purification was performed by silica gel column chromatography (Wako gel, C200, chloroform : acetone = 20 : 1), and the other processes were taken almost same way as of In Example 26 to obtain the product.
Yield: 11.0 mg (Yield 13 %)
The product was treated by the same method as of in Example 1 to obtain the hydrochloride.

Example 34

Production of (1R)-3-[2-(4-hydroxy-2-adamantyl)ethyl]-1,8,8-trimethyl-3-azabicyclo [3.2.1] octane:

Example 27 was repeated except that (1R)-1,8,8-trimethyl-3-azabicyclo [3.2.1]octane hydrochloride 0.22 g and 4-hydroxy-2-adamantyl acetic acid 0.37 g were used and purification was performed by silica gel column chromatography (Wako gel, C200, chloroform : methanol = 150 : 1) and the other processes were taken almost same way as of in Example 27 to obtain the product.
Yield: 74 mg (Yield 19 %)

The product was treated by the same method as of in Example 1 to obtain the hydrochloride.

Example 35

Production of (1R)-3-[2-(3-methyl-1-adamantyl)ethyl]-1,8,8-trimethyl-3-azabicyclo [3.2.1] octane:

Example 19 was repeated except that (1R)-1,8,8-trimethyl-3-azabicyclo [3.2.1] octane hydrochloride 0.50 g and 3-methly-1-adamantyl acetic acid 0.65 g were used and purification was performed by silica gel column chromatography (Wako gel, C200, chloroform), and the other processes were taken almost same way as of in Example 19 to obtain the product.
Yield: 0.74 g (Yield 85 %)
The product was treated by the same method as of in Example 1 to obtain the hydrochloride.
$[\alpha]_D^{25}$ =+10.6° (c=0.5, methanol)

Example 36

Production of (1S)-3-[3-(2-adamantyl)-1-propyl]-1,8,8-trimethyl-3-azabicyclo [3.2.1] octane:

Triethylamine 44.1 µl was added to a methylene chloride 3 ml solution of (1S)-1,8,8-trimethyl-3-azabicyclo [3.2.1] octane hydrochloride 50.0 mg and stirred at 0°C for 30 min. 3-(2-adamantyl) propionic acid 60.4 mg and WSC 65.7 mg were added therein, then temperature of the reaction mixture was gradually changed to room temperature and stirred for 13 hours. The reaction mixture was washed with saturated aqueous sodium chloride solution and dried by anhydrous sodium sulfate. After filtering the drying agent, the filtrate was concentrated in vacuo to obtain crude (1S)-3-[3-(2-adamantyl)propionyl)-1,8,8-trimethyl-3-azabicyclo [3.2.1] octane. The concentrate was added to THF 5 ml solution of lithium aluminium hydride 12.0 mg and refluxed under heating for 3 hours. The reaction mixture was cooled, then water was added thereto and the insoluble material was removed by filtration. The filtrate was concentrated in vacuo. The thus obtained residue was purified by means of silica gel column chromatography (Wako gel, C200, chloroform) to obtain the product.
Yield: 61.6 mg (Yield 71%)
The product was treated by the same way as of in Example 1 to obtain the hydrochloride.
$[\alpha]_D^{25}$ =-9.8 ° (c=0.5, methanol)

Example 37

Production of (1R)-3-[3-(2-adamantyl)-1-propyl]-1,8,8-trimethyl-3-azabicyclo [3.2.1] octane:

Example 36 was repeated except that (1R)-1,8,8-trimethyl-3-azabicyclo [3.2.1] octane hydrochloride 50.0 mg and 3-(2-adamantyl) propionic acid 60.4 mg were used and purification was performed by silica gel column chromatography (Wako gel. C200, chloroform), and the other processes were taken almost same way as of in Example 36 to obtain the product.
Yield: 59.0 mg (Yield 68 %)
The product was treated by the same method as of in Example 1 to obtain the hydrochloride.
$[\alpha]_D^{25}$ =+9.6 ° (c=0.5 methanol)

Example 38

Production of (1S)-3-[2-(3-noradamantyl)ethyl]-1,8,8-trimethyl-3-azabicyclo [3.2.1] octane:

Triethylamine 44.1 µl was added to a methylene chloride 3 ml solution of (1S)-1,8,8-trimethyl-3-azabicyclo [3.2.1] octane hydrochloride 50.0 mg, and stirred at 0°C for 30 min. WSC 65.7 mg and 3-noradamantyl acetic acid 52.2 mg were added therein, then temperature of the reaction mixture was gradually changed to room temperature and stirred for 18 hours. The reaction mixture was washed with saturated sodium chloride solution and dried by anhydrous sodium sulfate. After filtering the drying agent, the filtrate was concentrated in vacuo to obtain crude (1S)-3-(3-noradamantyl acetyl)-1,8,8-trimethyl-3-azabicyclo [3.2.1] octane. The concentrate was added to THF 5 ml solution of lithium aluminium hydride 12.0 mg and refluxed under heating for 3 hours. The reaction mixture was cooled, then added water, and removed the insoluble material by filtration. The filtrate was concentrated in vacuo. The thus obtained residue was purified by means of silica gel column chromatography (Wako gel, C200, chloroform) to obtain the product.
Yield: 65.9 mg (Yield 83 %)

The product was treated by the same way as of in Example 1 to obtain the hydrochloride.
$[\alpha]_D^{25}$ =-9.8 ° (c=0.5, methanol)

Example 39

Production of (1S)-3-(2-adamantylmethyl)-1,8,8-trimethyl-3-azabicyclo [3.2.1] octane:

Triethylamine 0.096 ml was added to a methylene chloride 5 ml solution of (1S)-1,8,8-trimethyl-3-azabicyclo [3.2.1] octane hydrochloride 100 mg and stirred at 0°C for 30 min. 2-adamantane carboxylic acid 0.11 g and WSC 0.13 g were added therein, then temperature of the reaction mixture was gradually changed to room temperature and stirred for 17 hours. The reaction mixture was washed with saturated aqueous sodium chloride solution and dried by anhydrous sodium sulfate. After filtering the drying agent, the filtrate was concentrated in vacuo to obtain crude (1S)-3-(2-adamantylcarbonyl)-1,8,8-trimethyl-3-azabicyclo [3.2.1] octane. The concentrate was added to THF 10 ml solution of lithium aluminium hydride 24.0 mg and refluxed under heating for 2 hours. The reaction mixture was cooled, then water was added thereto and the insoluble material was removed by filtration. The filtrate was concentrated in vacuo. The thus obtained residue was purified by means of silica gel column chromatography (Wako gel, C200, chloroform) to obtain the product.
Yield: 53.0 mg (Yield 33 %)

The product was treated by the same way as of in Example 1 to obtain the hydrochloride.
$[\alpha]_D^{25}$ =-9.7 ° (c=0.5, methanol)

Example 40

Production of (1R)-3-(2-adamantylmethyl)-1,8,8-trimethyl-3-azabicyclo [3.2.1] octane:

Example 39 was repeated except that (1R)-1,8,8-trimethyl-3-azabicyclo [3.2.1] octane hydrochloride 100 mg and 2-adamantane carboxylic acid 0.11 g were used and purification was performed by silica gel column chromatography (Wako gel, C200, chloroform), and the other processes were taken almost same way as of in Example 39 to obtain the product.
Yield: 55.2 mg (Yield 34 %)

The product was treated by the same method as of in Example 1 to obtain the hydrochloride.
$[\alpha]_D^{25}$ =+10.3° (c=0.5, methanol)

Example 41

Manufacture of preparation for injection:

(1S)-3-[2-(2-adamantyl)ethyl]-1,8,8-trimethyl-3-azabicyclo [3.2.1]octane hydrochloride 1.0 g was dissolved in distilled water for injection 1 liter, adjusted to pH 6-7 with 1N aqueous sodium hydroxide, sterilized, and filled each 5 ml in ampule to prepare a preparation for injection.

Example 42

Manufacture of tablets:

| | |
|---|---|
| (1S)-3-[2-(2-adamantyl)ethyl]-1,8,8-trimethyl-3-azabicyclo [3.2.1] octane hydrochloride | 50.0 mg |
| corn starch | 40.0 mg |
| crystalline cellulose | 80.0 mg |
| magnesium stearate | 0.5 mg |
| talc | 29.5 mg |

A tablet of 200 mg consisting of the above composition was prepared by means of dry tabletting.

Example 43

Production of (1R)-3-(1-naphthylacetyl)-1,8,8-trimethyl-3-azabicyclo [3.2.1] octane:

Triethylamine 0.84 ml was added to a methylene chloride 20 ml solution of (1R)-1,8,8-trimethyl-3-azabicyclo [3.2.1] octane hydrochloride 0.95 g and stirred at 0°C for 30 min. 1-naphthylacetic acid 0.93 g(5.0 mM) and 1-ethyl-3-(3' -dimethylamino propyl) carbodiimide hydrochloride (WSC) 1.35 g (7.0 mM) were added thereto, then temperature of the reaction mixture was gradually changed to room temperature and stirred for 24 hours. The reaction mixture was washed with saturated aqueous sodium chloride solution and dried by anhydrous sodium sulfate. After removing the drying agent by filtration from organic solvent, the filtrate was concentrated in vacuo. The thus obtained residue was purified by means of silica gel (Wako gel C200, Wako Pure Chemical Co.) column chromatography, developer : chloroform) to obtain the product as white powder.
Yield: 1.09 g (Yield 67.7 %)

Example 44

Production of (1R)-3-(2-naphthylacetyl)-1,8,8-trimethyl-3-azabicyclo [3.2.1] octane:

In Example 43, 2-naphthylacetic acid was used in place of 1-naphthylaceticacid to obtain the product as white powder.
Yield: 1.28g (Yield 79.5%)

Example 45

Production of (1R)-3-[2-(1-naphthyl)ethyl]-1,8,8-trimethyl-3-azabicyclo [3.2.1] octane:

(1R)-3-(1-naphthylacetyl)-1,8,8-trimethyl-3-azabicyclo [3.2.1] octane 1.09 g (3.4 mM) was added to tetrahydrofuran (THF) 20 ml solution of lithium aluminium hydride 0.25 g (6.8 mM) and refluxed under heating for 1 hour. The reaction mixture was cooled and added 6N hydrochloric acid and 1N aqueous sodium hydroxide thereto. The precipitate was removed by filtration and the filtrate was concentrated in vacuo. The thus obtained residue was purified by means of silica gel (Wako gel C-200, Wako Pure Chemical Co.) chromatography (developer : chloroform : acetone = 10 : 1) to obtain the product as colorless oily material.
Yield: 1.03 g (Yield 99.0 %)

Two molar excess of hydrogen chloride methanol solution was added to the above compound and concentrated in vacuo. The residue was crystallized by diethyl ether to prepare hydrochloride.

$[\alpha]_D^{25}$ =+6.0 ° (c=0.5, methanol)

Example 46

Production of (1R)-3-[2-(2-naphthyl)ethyl]-1,8,8-trimethyl-3-azabicyclo [3.2.1] octane:

(1R)-3-(2-naphthylacetyl)-1,8,8-trimethyl-3-azabicyclo [3.2.1] octane 1.28 g (3.99 mM) was added to THF 25 ml solution of lithium aluminium hydride 0.30 g (7.89 mM) and refluxed under heating for 1 hour. The reaction mixture was coold and added 6N hydrochloric acid and 1N aqueous sodium hydroxide thereto. The precipitate was removed by filtration and the filtrate was concentrated in vacuo. The thus obtained residue was purified by means of silica gel (Wako gel C200, Wako Pure Chemical Co.) chromatography (developer : chloroform : acetone = 10 : 1) to obtain the product as colorless oily material.
Yield: 1.22 g (Yield 100 %)

The compound was treated by the same method as of in Example 45 to obtain hydrochloride.

$[\alpha]_D^{25}$ =+3.2 ° (c=0.5, methanol)

Example 47

Production of (1R)-3-[2-(2-pyridyl)ethyl]-1,8,8-trimethyl-3-azabicyclo [3.2.1] octane:

Triethylamine 1.75 ml (12.6 mM) was added to a methylene chloride 20 ml solution of (1R)-1,8,8-trimethyl-3-azabicyclo [3.2.1] octane hydrochloride 0.95 g (5.0 mM) and 2-pyridylacetic acid hydrochloride 0.96 g (5.5 mM) and stirred at 0 °C for 30 min. WSC 1.35g (7.0 mM) was added therein, then temperature of the reaction mixture was gradually changed to room temperature and stirred for 24 hours. The reaction mixture was washed with saturated aqueous sodium chloride solution and dried by anhydrous sodium sulfate. After filtering the drying agent, the filtrate was concentrated in vacuo to obtain crude (1R)-3-(2-pyridyl-acetyl)-1,8,8-trimethyl-3-azabicyclo [3.2.1] octane. The concentrate was added to THF 20 ml solution of lithium aluminium hydride 0.29 g (7.6 mM) and refluxed under heating for 1 hour. The reaction mixture was cooled, then water was added thereto and the insoluble material was removed by filtration. The filtrate was concentrated in vacuo. The thus obtained residue was purified by means of silica gel column chromatography (Wako gel, C200, chloroform : methanol = 10 : 1-5 : 1) to obtain the product as yellowish oily material.
Yield: 0.20 g (Yield 15.5 % by 2 steps)

The product was treated by the same way as of in Example 45 to obtain the hydrochloride.
$[\alpha]_D^{25}$ =+5.9 ° (c=0.5, methanol)

<u>Example 48</u>

Production of (1R)-3-[2-(4-pyridyl) ] ethyl-1,8,8-trimethyl-3-azabicyclo [3.2.1] octane:

Example 47 was repeated except that (1R)-1,8,8-trimethyl-3-azabicyclo [3.2.1] octane hydrochloride 0.95 g (5.0 mM) and 4-pyridyl acetic acid hydrochloride 0.96 g (5.5 mM) were used and a crude sabstance obtained from the reaction mixture was purified by silica gel column chromatography (Wako gel, C200, chloroform : methanol = 50 : 1), and the other processes were taken almost same way as of in Example 47 to obtain the product as yellowish oily material.
Yield: 0.37 g (Yield 28.7 % by 2 steps)

The product was treated by the same method as of in Example 45 to obtain the hydrochloride.
$[\alpha]_D^{25}$ =+7.9 ° (c=0.5, methanol)

<u>Referential example 31</u>

Production of (1S)-1,8,8-trimethyl-3-azabicyclo[3.2.1]octan-2-one:

(1S)-camphor 8.31 g (54.6 mM) and hydroxylamine-O-sulphonic acid 9.25 g (81.9 mM) in acetic acid 200 ml were refluxed under heating for 7 hours. The reaction mixture was concentrated in vacuo and the residue dissolved in chloroform was washed with saturated aqueous sodium bicarbonate solution. Organic layer was dried by anhydrous sodium sulfate, which was thereafter removed by filtration, then the filtrate was concentrated in vacuo. The thus obtained residue was purified by means of silica gel column chromatography (Wako gel, C200, chloroform) to obtain the product as white needle crystal.
Yield: 5.57 g (Yield 55.6 %)
$[\alpha]_D^{25}$ =+25.0° (c=1, methanol)

<u>Referential example 32</u>

Production of (1S)-1,8,8-trimethyl-3-azabicyclo[3.2.1]octane:

(1S)-1,8,8-trimethyl-3-azabicyclo [3.2.1] octan-2-one 1.93 g (11.6 mM) was added in THF 20 ml solution of lithium aluminium hydride 0.88 g (23 mM), and the reaction mixture was refluxed under heating for 22 hours. The reaction mixture was cooled and added 6N HCl and 5N NaOH therein to alkalize the mixture. Insoluble material was removed by filtration and the filtrate was extracted with ether, dried by anhydrous sodium sulfate. The drying agent was removed by filtration and the filtrate was concentrated in vacuo to obtain the product.
Yield: 1.70 g (Yield 96.6 %)
$[\alpha]_D^{25}$ =-17.2° (c=1, methanol)

<u>Example 49</u>

Production of (1S)-3-[2-(1-naphtyl)ethyl]-1,8,8-trimethyl-3-azabicyclo-[3.2.1] octane:

Methylene chloride 20 ml solution of 2-(1-naphthyl)ethyl alcohol 0.87 g (5.0 mM) was cooled to 0°C, then methanesulfonyl chloride 0.47 ml (6.1 mM) and triethylamine 0.84 ml (6.0 mM) were added thereto. Temperature of the reaction mixture was gradually changed to room temperature and stirred for 21 hours. The reaction mixture was concentrated in vacuo to obtain the crude mesylate. (1S)-1,8,8-trimethyl-3-azabicyclo [3.2.1] octane hydrochloride 0.95 g (5.0 mM) and potassium carbonate 2.08 g (15.0 mM) were added thereto, then prepared acetonitrile 20 ml solution. The solution was refluxed under heating for 18 hours. Insoluble material was removed by filtration and the filtrate was concentrated in vacuo. The thus obtained residue was purified by means of silica gel column chromatography (Wako gel, C200, chloroform) to obtain the product.
Yield: 0.92 g (Yield 59.4 %)

The product was treated by the same way as of in Example 45 to obtain the hydrochloride.
$[\alpha]_D^{25}$ =-5.5 ° (c=0.5, methanol)

Example 50

Production of (1S)-3-[2-(2-naphthyl)ethyl]-1,8,8-trimethyl-3-azabicyclo [3.2.1] octane:

Triethylamine 0.84 ml (6.0 mM) was added to a methylene chloride 20 ml solution of (1S)-1,8,8-trimethyl-3-azabicyclo [3.2.1] octane hydrochloride 0.95 g(5.0 mM) and stirred at 0 °C for 30 min. β-naphthylacetic acid 0.93g (5.0 mM) and WSC 1.35 g (7.04 mM) were added therein, then temperature of the reaction mixture was gradually changed to room temperature and stirred for 23 hours. The reaction mixture was washed with saturated aqueous sodium chloride solution and dried by anhydrous sodium sulfate. After the drying agent was removed by filtration, the filtrate was concentrated in vacuo to obtain crude (1S)-3-(2-naphthylacetyl)-1,8,8-trimethyl-3-azabicyclo [3.2.1] octane. The concentrate was added to THF 30 ml solution of lithium aluminium hydride 0.39 g (10.3 mM) and refluxed under heating for 1 hour. The reaction mixture was cooled, then water was added thereto and the insoluble material was removed by filtration. The filtrate was concentrated in vacuo. The thus obtained residue was purified by means of silica gel column chromatography (Wako gel, C200, chloroform) to obtain the product.
Yield: 1.11 g (Yield 72.1 %)

The product was treated by the same way as of in Example 45 to obtain the hydrochloride.
$[\alpha]_D^{25}$ =-6.2 ° (c=0.5, methanol)

Example 51

Production of (1S)-3-[2-(2-pyridyl)ethyl]-1,8,8-trimethyl-3-azabicyclo [3.2.1] octane:

Triethylamine 3.50 ml (25.1 mM) was added to a methylene chloride 40 ml solution of (1S)-1,8,8-trimethyl-3-azabicyclo [3.2.1] octane hydrochloride 1.90 g(10.0 mM) and 2-pyridylacetic acid hydrochloride 1.92 g (11.6 mM) and stirred at 0°C for 30 min. WSC 2.70 g (14.1 mM) was added therein, then temperature of the reaction mixture was gradually changed to room temperature and stirred for 48 hours. The reaction mixture was washed with saturated aqueous sodium chloride solution and dried by anhydrous sodium sulfate. After the drying agent was removed by filtration the filtrate was concentrated in vacuo to obtain crude (1S)-3-(2-pyridylacetyl)-1,8,8-trimethyl-3-azabicyclo [3.2.1] octane. The concentrate was added to THF 60 ml solution of lithium aluminium hydride 0.77 g (20.3 mM) and refluxed under heating for 1 hour. The reaction mixture was cooled, then water was added thereto and the insoluble material was removed by filtration. The filtrate was concentrated in vacuo. The thus obtained residue was purified by means of silica gel column chromatography (Wako gel, C200 chloroform : acetone = 10 : 1) to obtain the product as orange colored oily material.
Yield: 0.99 g (Yield 38.2 %)

The product was treated by the same way as of in Example 45 to obtain the hydrochloride.
$[\alpha]_D^{25}$ =-18.2° (c=0.5, methanol)

Example 52.

Production of (1S)-3-[2-(3-pyridyl)ethyl]-1,8,8-trimethyl-3-azabicyclo [3.2.1] octane:

Triethylamine 3.20 ml (23.0 mM) was added to a methylene chloride 40 ml solution of (1S)-1,8,8-trimethyl-3-azabicyclo [3.2.1] octane hydrochloride 1.74 g(9.18 mM) and 3-pyridylacetic acid hydrochloride 1.76 g (10.1 mM) and stirred at 0°C for 30 min. WSC 2.47 g (12.9 mM) was added therein, then temperature of the reaction mixture was gradually changed to room temperature and stirred for 46 hours. The reaction mixture was washed with saturated aqueous sodium chloride solution and dried by anhydrous sodium sulfate. After the drying agent was removed by filtration, the filtrate was concentrated in vacuo to obtain crude (1S)-3-(3-pyridylacetyl)-1,8,8-trimethyl-3-azabicyclo [3.2.1] octane. The concentrate was added to THF 60 ml solution of lithium aluminium hydride 0.77 g (20.3 mM) and refluxed under heating for 3 hours. The reaction mixture was cooled, then water was added thereto and the insoluble material was

removed by filtration. The filtrate was concentrated in vacuo. The thus obtained residue was purified by means of silica gel column chromatography (Wako gel, C200, chloroform : acetone = 10 : 1-5 : 1) to obtain the product as yellowish oily material.
Yield: 0.46g (Yield 19.4%)

The product was treated by the same way as of in Example 45 to obtain the hydrochloride.
$[\alpha]_D^{25}$ =-3.7 ° (c=0.5, methanol)

Example 53

Production of (1S)-3-[2-(4-pyridyl)ethyl]-1,8,8-trimethyl-3-azabicyclo [3.2.1] octane:

Triethylamine 1.75 ml (12.6 mM) was added to a methylene chloride 20 ml solution of (1S)-1,8,8-trimethyl-3-azabicyclo [3.2.1] octane hydrochloride 0.95 g(5.0 mM) and 4-pyridylacetic acid hydrochloride 0.96g (5.5 mM) and stirred at 0°C for 30 min. WSC 1.35g (7.04mM) was added thereto, then temperature of the reaction mixture was gradually changed to room temperature and stirred for 24 hours. The reaction mixture was washed with saturated aqueous sodium chloride solution and dried by anhydrous sodium sulfate. After the drying agent was removed by filtration, the filtrate was concentrated in vacuo to obtain crude (1S)-3-(4-pyridylacetyl)-1,8,8-trimethyl-3-azabicyclo [3.2.1] octane. The concentrate was added to THF 30 ml solution of lithium aluminium hydride 0.39 g (10.3 mM) and refluxed under heating for 3 hours. The reaction mixture was cooled, then water was added thereto and the insoluble material was removed by filtration. The filtrate was concentrated in vacuo. The thus obtained residue was purified by means of silica gel column chromatography (Wako gel, C200, chloroform : acetone = 10 : 1) to obtain the product as orange colored oily material.
Yield: 0.35 g (Yield 27.1 %)

The product was treated by the same way as of in Example 45 to obtain the hydrochloride.
$[\alpha]_D^{25}$ =-11.0° (c=0.5, methanol)

Example 54

Production of (1S)-3-[2-(1,3-benzodioxol-5-yl)ethyl]-1,8,8-trimethyl-3-azabicyclo [3.2.1] octane:

Triethylamine 0.54 ml (3.9 mM) was added to a methylene chloride 15 ml solution of (1S)-1,8,8-trimethyl-3-azabicyclo [3.2.1] octane hydrochloride 0.52 g(2.8 mM) and stirred at 0 °C for 30 min. 1,3-benzodioxole-5-acetic acid 0.60g (3.3 mM) and WSC 0.74 g (3.9 mM) were added therein then temperature of the reaction mixture was gradually changed to room temperature and stirred for 21 hours. The reaction mixture was washed with saturated aqueous sodium chloride solution and dried by anhydrous sodium sulfate. After the drying agent was removed by filtration, the filtrate was concentrated in vacuo to obtain crude (1S)-3-(1,3-benzodioxole-5-yl-acetyl)-1,8,8-trimethyl-3-azabicyclo [3.2.1] octane. The concentrate was added to THF 10 ml solution of lithium aluminium hydride 0.21 g (5.5 mM) and refluxed under heating for 2.5 hours. The reaction mixture was cooled, then water was added thereto and the insoluble material was removed by filtration. The filtrate was concentrated in vacuo. The thus obtained residue was purified by means of flash silica gel column chromatography (Merck, silica gel 60 : 230-600 mesh, chloroform) to obtain the product as colorless oily material.
Yield: 0.66 g (Yield 78.2 %)

The product was treated by the same way as of in Example 45 to obtain the hydrochloride.
$[\alpha]_D^{25}$ =-6.5 ° (c=0.5, methanol)

Example 55

Production of (1S)-3-[2-(2-thienyl)ethyl]-1,8,8-trimethyl-3-azabicyclo [3.2.1] octane:

Triethylamine 0.34 ml (2.4 mM) was added to a methylene chloride 10 ml solution of (1S)-1,8,8-trimethyl-3-azabicyclo [3.2.1] octane hydrochloride 0.38 g(2.0 mM) and stirred at 0 °C for 30 min. 2-thienyl acetic acid 0.35 g (2.5 mM) and WSC 0.54 g (2.8 mM) were added therein, then temperature of the reaction mixture was gradually changed to room temperature and stirred for 21 hours. The reaction mixture was washed with saturated aqueous sodium chloride solution and dried by anhydrous sodium sulfate. After the drying agent was removed by filtration, the filtrate was concentrated in vacuo to obtain crude (1S)-3-(2-thienylacetyl)-1,8,8-trimethyl-3-azabicyclo [3.2.1] octane. The concentrate was added to THF 15 ml solution of lithium aluminium hydride 0.16 g (4.2 mM) and refluxed under heating for 1 hour. The reaction mixture was cooled, then water was added thereto and the insoluble material was removed by filtration. The filtrate was concentrated in vacuo. The thus obtained residue was purified by means of silica gel column chromatography (Wako gel, C200, chloroform) to obtain the product as pale yellowish oily material.
Yield: 0.37 g (Yield 65.8 %)

The product was treated by the same way as of in Example 45 to obtain the hydrochloride.
$[\alpha]_D^{25}$ =-12.8° (c=0.5, methanol)

Example 56

Manufacture of preparation for injection:

(1S)-3-[2-(2-naphthyl)ethyl]-1,8,8-trimethyl-3-azabicyclo [3.2.1] octane hydrochloride 1.0 g was dissolved in distilled water for injection 1 liter, adjusted to pH 6-7 with 1N aqueous sodium hydroxide, sterilized, and filled each 5 ml in ampule to prepare a preparation for injection.

Example 57

Manufacture of tablets:

| | |
|---|---|
| (1S)-3-[2-(2-naphthyl)ethyl]-1,8,8-trimethyl-3-azabicyclo [3.2.1] octane hydrochloride | 50.0 mg |
| corn starch | 40.0 mg |
| crystalline cellulose | 80.0 mg |
| magnesium stearate | 0.5 mg |
| talc | 29.5 mg |

A tablet of 200mg consisting of the above composition was prepared by means of dry tabletting.

Table 5

| Ref.No. | $^1H-NMR$ ($CDCl_3$)δ (ppm) | MS |
|---|---|---|
| 1 | 0.95 (3H, s), 1.04 (3H, s), 1.10 (3H, s), 1.5 ~ 1.7 (1H, m), 1.7 ~ 1.8 (1H, m), 1.8 ~ 1.9 (1H, m), 2.0 ~ 2.2 (2H, m), 3.00 (1H, d, J= | (FAB) 168 ($MH^+$) |

| | | |
|---|---|---|
| | 10.9 Hz), 3.48(1H, dd, J=3.0, 10.9 Hz), 5.50 (1H, bs) | |
| 2 | 0.75 (3H, s), 0.86 (3H, s), 1.03 (3H, s), 1.4 ~ 1.7 (4H, m), 1.8 ~ 2.0 (1H, m), 2.13 (1H, d, J=12.9 Hz), 2.42 (1H, dd, J=3.0, 12.9 Hz), 2.86 (1H, d, J= 13.2 Hz), 3.16 (1H, d, J=12.9 Hz) | (EI) 153 ($M^+$) |
| 3 | 1.5 ~ 1.8 (8H, m), 2.38 (2H, dd, J=5.6, 6.3), 2.88 (2H, dd, J=5.9, 6.3), 3.68 (3H, s), 5.67 (1H, s) | |
| 4 | 1.1 ~ 1.9 (16H, m), 3.67 (2H, t, J=6.6) | |
| 5 | 1.4 ~ 1.6 (10H, m), 2.32 (2H, dd, J=5.9, 6.3), 2.76 (2H, t, J=6.3), 3.68 (3H, s), 5.72 (1H, s) | |
| 6 | 1.2 ~ 1.8 (14H, m), 1.9 ~ 2.1 (1H, m), 2.22 (2H, d, J=7.6), 3.66 (3H, s) | |
| 7 | 1.2 ~ 1.8 (14H, m), 2.0 ~ 2.2 (1H, m), 2.26 (2H, d, J=7.3) | |
| 8 | 1.8 ~ 2.0 (12H, m), 2.44 (1H, bs), 3.68 (3H, s), 4.06 (1H, bs), 5.59 (1H, s) | |
| 9 | 1.5 ~ 1.9 (13H, m), 2.23 (1H, dd, J=7.3, 7.9 Hz), 2.46 (2H, d, J=7.6 Hz), 3.66 (3H, s) | |

| | | |
|---|---|---|
| 10 | 1.56 (2H, d, J=11.9 Hz), 1.6 ~ 1.9 (12H, m), 2.24 (1H, t, J=7.3 Hz), 2.50 (2H, d, J=7.3 Hz) | (FAB) 193 ($M^- H^-$) |
| 11 | 1.1 ~ 1.2 (1H, m), 1.5 ~ 2.0 (16H, m), 3.67 (2H, dd, J=6.1, 12.0 Hz) | |
| 12 | 1.4 ~ 2.1 (12H, m), 2.36 (1H, bs), 3.68 (3H, , s), 3.99 (1H, bs), 5.64 (1H, bs) | |
| 13 | 1.4 ~ 1.8 (14H, m), 2.02 (1H, dd, J=7.3, 7.9 Hz), 2.46 (2H, d, J=7.9 Hz), 3.66 (3H, s) | |
| 14 | 1.4 ~ 1.9 (14H, m), 2.03 (1H, t, J=7.6 Hz), 2.50 (2H, d, J=7.6 Hz) | |
| 15 | 1.54 (1H, s), 1.7 ~ 1.9 (10H, m), 2.25 (1H, b), 2.61 (1H, b), 3.69 (3H, s), 4.33 (1H, b), 5.61 (1H, s) | (FAB) 223 ($MH^+$) |
| 16 | 1.4 ~ 2.2 (15H, m), 2.41 (1H, d, J=7.6 Hz), 2.45 (1H, d, J=7.6 Hz), 3.67 (1.5H, s), 3.67 (1.5H, s) | (FAB) 223 ($M^- H^-$) |
| 17 | 1.4 ~ 2.2 (15H, m), 2.45 (1H, d, J=7.6 Hz), 2.45 (1H, d, J=7.6 Hz) | (FAB) 209 ($M^- H^-$) |

| | | |
|---|---|---|
| 18 | 1.9 ~ 2.1 (10H, m), 2.35 (1H, b), 2.64 (2H, b), 3.26 (3H, s) | |
| 19 | 1.7 ~ 1.9 (10H, m), 2.26 (1H, b), 2.63 (1H, b), 3.23 (3H, s), 3.69 (3H, s), 4.24 (1H, b), 5.61 (1H, s) | (FAB) 237 ($MH^+$) |
| 20 | 1.3 ~ 2.2 (14H, m), 2.41 (1H, d, J=7.3 Hz), 2.45 (1H, d, J=7.6 Hz), 3.23 (1.5H, s), 3.23 (1.5H, s), 3.67 (1.5H, s), 3.67 (1.5H, s) | (FAB) 237 ($M^- H^-$) |
| 21 | 1.3 ~ 2.2 (14H, m), 2.38 (1H, d, J=7.9 Hz), 2.41 (1H, d, J=7.6 Hz), 3.17 (3H, s) | |
| 22 | 1.8 ~ 2.1 (10H, m), 2.20 (1H, b), 2.61 (1H, b), 3.70 (3H, s), 4.23 (1H, b), 5.66 (1H, s), 7.1 ~ 7.4 (5H, m) | (FAB) 283 ($MH^+$) |
| 23 | 1.7 ~ 2.3 (14H, m), 2.48 (1H, d, J=7.6 Hz), 2.53 (1H, d, J=7.6 Hz), 3.67 (1.5H, s), 3.75 (1.5H, s), 7.1 ~ 7.4 (5H, m) | (FAB) 285 ($MH^+$) |
| 24 | 1.5 ~ 2.1 (14H, m), 2.41 (1H, d, J=7.6 Hz), 2.45 (1H, d, J=7.6 Hz), 7.1 ~ 7.4 (5H, m) | |
| 25 | 1.6 ~ 2.2 (11H, m), 2.41 (0.5H, b), 2.46 (0.5H, b), 3.69 (3H, s), 3.70 (3H, s), 4.01 (1H, b), 4.08 (1H, b), 5.67 (0.5H, s), 5.79 (0.5H, s) | (FAB) 223 ($MH^+$) |

| | | |
|---|---|---|
| 26 | 1.4 ~ 2.2 (16H, m), 3.66 (1.5H, s), 3.68 (1.5H, s), 3.93 (1H, b) | |
| 28 | 1.6 ~ 2.2 (9H, m), 2.44 (1H, b), 2.56 (1H, b), 2.98 (0.5H, b), 3.08 (0.5H, b), 3.68 (1H, s), 3.71 (1H, s), 3.72 (1H, s), 4.06 (0.5H, b), 4.74 (0.5H, b), 5.59 (0.5H, s), 5.70 (0.25H, s), 5.76 (0.25H, s), 7.1 ~ 7.5 (5H, m) | (FAB) 283 ($MH^+$) |
| 29 | 1.5 ~ 2.6 (15H, m), 3.0 ~ 3.1 (1H, m), 3.66 (1H, s), 3.70 (1H, s), 3.71 (1H, s), 7.1 ~ 7.4 (5H, m) | |
| 30 | 1.5 ~ 2.6 (15H, m), 3.0 ~ 3.1 (1H, m), 7.1 ~ 7.5 (5H, m) | (FAB) 269 ($M^- H^-$) |

Table 6

| Exam. No. | $^1H$-NMR ($CDCl_3$) δ (ppm) | MS |
|---|---|---|
| 1 | 0.77 (3H, s), 0.84 (3H, s), 0.89 (3H, s), 1.2 ~ 2.1 (22H, m), 2.2 ~ 2.8 (6H, m) | (FAB) 316 ($MH^+$) |
| 2 | 0.77 (3H, s), 0.84 (3H, s), 0.89 (3H, s), 0.9 ~ 1.9 (14H, m), 2.1 ~ 2.4 (4H, m), 2.49 (1H, s), 2.50 (1H, s) | (FAB) 276 ($MH^+$) |

| | | |
|---|---|---|
| 3 | 0.77 (3H, s), 0.84 (3H, s), 0.89 (3H, s), 0.9 ~ 1.2 (2H, m), 1.4 ~ 1.8 (14H, m), 2.19 (1H, d, J=10.6), 2.25 (1H, d, J=10.6), 2.35 (2H, t, J=7.8), 2.48 (1H, s), 2.49 (1H, s) | (FAB) 250 ($MH^+$) |
| 4 | 0.77 (3H, s), 0.85 (3H, s), 0.89 (3H, s), 1.1 ~ 1.4 (6H, m), 1.5 ~ 1.8 (12H, m), 2.18 (1H, d, J=10.2), 2.25 (1H, d, J=10.6), 2.36 (2H, dd, J=6.9, 7.3), 2.48 (2H, s) | (FAB) 264 ($MH^+$) |
| 5 | 0.86 (1.5H, s), 0.87 (1.5H, s), 0.89 (3H, s), 0.99 (3H, s), 1.1 ~ 2.0 (12H, m), 2.1 ~ 2.3 (4H, m), 2.80 (1H, d, J=12.9), 3.07 (1H, d, J=12.9), 3.14 (2H, d, J=2.3), 3.80 (1H, d, J=13.5), 4.02 (1H, dd, J=3.0, 13.5) | , |
| 6 | 0.77 (3H, s), 0.84 (3H, s), 0.89 (3H, s), 1.1 ~ 1.4 (6H, m), 1.5 ~ 1.8 (12H, m), 2.18 (1H, d, J=10.2), 2.24 (1H, d, J=10.6), 2.36 (2H, dd, J=6.9, 7.6), 2.48 (2H, s) | (FAB) 264 ($MH^+$) |
| 7 | 0.77 (3H, s), 0.84 (3H, s), 0.89 (3H, s), 1.1 ~ 1.8 (18H, m), 2.17 (1H, d, J=10.6), 2.25 (1H, d, J=10.6), 2.35 (2H, t, J=7.6), 2.48 (2H, d, J=2.6) | (FAB) 278 ($MH^+$) |
| 8 | 0.78 (3H, s), 0.85 (3H, s), 0.90 (3H, s), 1.2 ~ 1.8 (22H, m), 2.21 (1H, d, J=10.6), 2.27 (1H, d, J=10.6), 2.78 (2H, dd, J=7.3, 7.9), | (FAB) 292 ($MH^+$) |

| | | |
|---|---|---|
| | 2.50 (1H, s), 2.51 (1H, s) | |
| 9 | 0.77 (3H, s), 0.85 (3H, s), 0.90 (3H, s), 1.0 ~ 1.8 (22H, m), 2.20 (1H, d, J=10.2), 2.25 (1H, d, J=10.6), 2.34 (2H, t, J=7.3), 2.49 (2H, s) | (FAB) 292 ($MH^+$) |
| 10 | 0.77 (3H, s), 0.85 (3H, s), 0.90 (3H, s), 1.0 ~ 1.9 (20H, m), 2.20 (1H, d, J=10.2), 2.27 (1H, d, J=10.2), 2.32 (2H, t, J=7.3), 2.49 (1H, s), 2.50 (1H, s) | (FAB) 278 ($MH^+$) |
| 11 | 0.76 (3H, s), 0.84 (3H, s), 0.89 (3H, s), 1.0 ~ 1.9 (16H, m), 2.10 (2H, d, J=7.3), 2.1 ~ 2.2 (1H, m), 2.19 (1H, d, J=10.6), 2.40 (1H, dd, J=3.6, 10.6), 2.46 (1H, d, J=10.6) | (FAB) 250 ($MH^+$) |
| 12 | 0.76 (3H, s), 0.84 (3H, s), 0.89 (3H, s), 1.1 ~ 1.3 (3H, m), 1.4 ~ 1.8 (13H, m), 2.10 (2H, d, J=7.6), 2.1 ~ 2.2 (1H, m), 2.23 (1H, d, J=10.6), 2.40 (1H, dd, J=3.3, 10.6), 2.47 (1H, d, J=10.6) | (FAB) 250 ($MH^+$) |
| 13 | 0.81 (3H, s), 0.87 (3H, s), 0.92 (3H, s), 1.0 ~ 2.0 (15H, m), 2.2 ~ 2.9 (5H, m) | (FAB) 236 ($MH^+$) |
| 14 | 0.77 (3H, s), 0.84 (3H, s), 0.89 (3H, s), 1.2 ~ 2.1 (22H, m), 2.23 (2H, s), 2.36 (2H, | (FAB) 316 |

| | | |
|---|---|---|
| | dd, J=7.6, 8.6 Hz), 2.47 (1H, d, J=10.9 Hz), 2.53 (1H, d, 10.9 Hz) | ($MH^+$) |
| 15 | 0.77 (3H, s), 0.85 (3H, s), 0.90 (3H, s), 1.4 ~ 1.9 (22H, m), 2.18 (1H, d, J=10.6 Hz), 2.26 (1H, d, J=10.6 Hz), 2.34 (2H, t, J=7.6 Hz), 2.48 (1H, s), 2.49 (1H, s) | (FAB) 316 ($MH^+$) |
| 16 | 0.78 (3H, s), 0.85 (3H, s), 0.90 (3H, s), 1.5 ~ 1.9 (22H, m), 2.1 ~ 2.3 (2H, m), 2.36 (2H, t, J=7.3 Hz), 2.52 (2H, s) | (FAB) 316 ($MH^+$) |
| 18 | 0.70 (3H, s), 0.78 (3H, s), 0.82 (3H, s), 0.8 ~ 0.9 (3H, m), 1.1 ~ 1.8 (17H, m), 2.1 ~ 2.2 (2H, m), 2.3 ~ 2.4 (2H, m), 2.4 ~ 2.5 (2H, m) | (FAB) 278 ($MH^+$) |
| 19 | 0.70 (3H, s), 0.71 (3H, s), 0.78 (3H, s), 0.83 (3H, s), 1.1 ~ 2.0 (21H, m), 2.1 ~ 2.6 (6H, m) | (FAB) 330 ($MH^+$) |
| 20 | 0.75 (3H, s), 0.83 (3H, s), 0.92 (3H, s), 1.5 ~ 1.8 (18H, m), 2.16 (2H, s), 2.42 (2H, s), 2.48 (1H, dd, J=3.6, 10.6 Hz), 2.64 (1H, d, J=10.2 Hz) | (FAB) 288 ($MH^+$) |
| 21 | 0.79 (3H, s), 0.86 (3H, s), 0.92 (3H, s), 1.4 ~ 1.9 (22H, m), 2.2 ~ 2.6 (6H, m) | (FAB) 304 ($MH^+$) |

| | | |
|---|---|---|
| 22 | 0.73 (3H, s), 0.82 (3H, s), 0.92 (3H, s), 1.4 ~ 1.8 (20H, m), 1.92 (2H, s), 2.10 (1H, d, J=10.2 Hz), 2.41 (1H, dd, J=10.2, 10.6 Hz), 2.48 (1H, d, J=10.2 Hz), 2.72 (1H, d, J=10.2 Hz) | (FAB) 302 ($MH^+$) |
| 23 | 0.79 (3H, s), 0.86 (3H, s), 0.91 (3H, s), 1.3 ~ 2.2 (21H, m), 2.2 ~ 2.3 (2H, m), 2.3 ~ 2.4 (2H, m), 2.5 ~ 2.6 (2H, m) | (EI) 331 ($M^+$) |
| 24 | 0.79 (3H, s), 0.86 (3H, s), 0.91 (3H, s), 1.3 ~ 2.2 (21H, m), 2.2 ~ 2.3 (2H, m), 2.3 ~ 2.4 (2H, m), 2.5 ~ 2.6 (2H, m), 3.23 (1.5H, s), 3.24 (1.5H, s) | (FAB) 346 ($MH^+$) |
| 25 | 0.78 (3H, s), 0.85 (3H, s), 0.90 (3H, s), 1.4 ~ 2.2 (21H, m), 2.2 ~ 2.3 (2H, m), 2.36 (2H, t, J=7.3 Hz), 2.51 (2H, s) | (FAB) 350 ($MH^+$) |
| 26 | 0.79 (1.5H, s), 0.80 (1.5H, s), 0.86 (1.5H, s), 0.87 (1.5H, s), 0.91 (1.5H, s), 0.93 (1.5H, s), 1.5 ~ 2.1 (21H, m), 2.3 ~ 2.4 (2H, m), 2.4 ~ 2.5 (2H, m), 2.6 ~ 2.7 (2H, m), 7.1 ~ 7.4 (5H, m) | (FAB) 392 ($MH^+$) |
| 27 | 0.81 (3H, s), 0.87 (3H, s), 0.94 (3H, s), 1.4 ~ 2.0 (20H, m), 2.2 ~ 2.3 (2H, m), 2.4 ~ 2.5 (2H, m), 2.5~ 2.6 (2H, m), 3.8 ~ 3.9 (1H, m) | (FAB) 332 ($MH^+$) |

| | | |
|---|---|---|
| 28 | 0.78 (1.5H, s), 0.79 (1.5H, s), 0.85 (3H, s), 0.90 (1.5H, s), 0.92 (1.5H, s), 1.5 ~ 2.0 (20H, m), 2.2 ~ 2.3 (2H, m), 2.4 ~ 2.5 (2H, m), 2.5 ~ 2.6 (2H, m), 2.98 (0.5H, b), 3.12 (0.5H, b), 7.1 ~ 7.5 (5H, m) | (FAB) 392 ($MH^+$) |
| 29 | 0.78 (1.5H, s), 0.79 (1.5H, s), 0.85 (3H, s), 0.91 (1.5H, s), 0.92 (1.5H, s), 1.5 ~ 2.0 (20H, m), 2.2 ~ 2.3 (2H, m), 2.4 ~ 2.6 (4H, m), 2.98 (0.5H, b), 3.12 (0.5H, b), 7.1 ~ 7.5 (5H, m) | (FAB) 392 ($MH^+$) |
| 30 | 0.79 (3H, s), 0.86 (3H, s), 0.91 (3H, s), 1.3 ~ 2.2 (21H, m), 2.2 ~ 2.3 (2H, m), 2.3 ~ 2.4 (2H, m), 2.5 ~ 2.6 (2H, m), 3.23 (1.5H, s), 3.24 (1.5H, s) | (FAB) 346 ($MH^+$) |
| 31 | 0.79 (3H, s), 0.86 (3H, s), 0.91 (3H, s), 1.3 ~ 2.2 (21H, m), 2.2 ~ 2.3 (2H, m), 2.3 ~ 2.4 (2H, m), 2.5 ~ 2.6 (2H, m) | (FAB) 332 ($MH^+$) |
| 32 | 0.78 (3H, s), 0.85 (3H, s), 0.90 (3H, s), 1.4 ~ 2.2 (21H, m), 2.2 ~ 2.3 (2H, m), 2.3 ~ 2.4 (2H, m), 2.51 (2H, s) | (FAB) 350 ($MH^+$) |
| 33 | 0.79 (1.5H, s), 0.80 (1.5H, s), 0.86 (1.5H, s), 0.87 (1.5H, s), 0.91 (1.5H, s), 0.93 (1.5H, s), 1.5 ~ 2.1 (21H, m), 2.3 ~ 2.4 (2H, m), 2.4 | (FAB) 392 ($MH^+$) |

| | | |
|---|---|---|
| | ~ 2.5 (2H, m), 2.6 ~ 2.7 (2H, m), 7.1 ~ 7.4 (5H, m) | |
| 34 | 0.81 (3H, s), 0.87 (3H, s), 0.94 (3H, s), 1.4 ~ 2.0 (20H, m), 2.2 ~ 2.3 (2H, m), 2.4 ~ 2.5 (2H, m), 2.5 ~ 2.6 (2H, m), 3.8 ~ 3.9 (1H, m) | (FAB) 332 ($MH^+$) |
| 35 | 0.70 (3H, s), 0.71 (3H, s), 0.78 (3H, s), 0.83 (3H, s), 1.1 ~ 2.0 (21H, m), 2.1 ~ 2.6 (6H, m) | (FAB) 330 ($MH^+$) |
| 36 | 0.78 (3H, s), 0.85 (3H, s), 0.91 (3H, s), 1.1 ~ 2.0 (24H, m), 2.1 ~ 2.2 (2H, m), 2.2 ~ 2.3 (2H, m), 2.4 ~ 2.5 (2H, m) | (FAB) 330 ($MH^+$) |
| 37 | 0.78 (3H, s), 0.86 (3H, s), 0.91 (3H, s), 1.2 ~ 2.1 (24H, m), 2.1 ~ 2.2 (2H, m), 2.2 ~ 2.3 (2H, m), 2.4 ~ 2.5 (2H, m) | (FAB) 330 ($MH^+$) |
| 38 | 0.76 (3H, s), 0.83 (3H, s), 0.91 (3H, s), 1.5 ~ 2.0 (21H, m), 2.1 ~ 2.2 (2H, m), 2.2 ~ 2.3 (2H, m), 2.4 ~ 2.5 (2H, m) | (FAB) 302 ($MH^+$) |
| 39 | 0.74 (3H, s), 0.83 (3H, s), 0.91 (3H, s), 1.5 ~ 1.8 (20H, m), 1.90 (2H, s), 2.1 ~ 2.5 (4H, m) | (FAB) 302 ($MH^+$) |
| 40 | 0.74 (3H, s), 0.83 (3H, s), 0.90 (3H, s), | (FAB) |

| | | |
|---|---|---|
| | 1.5 ～ 1.8 (20H, m), 1.90 (2H, s), 2.1 ～ 2.5 (4H, m) | 302 ($MH^+$) |

Table 7

| Exam. No. | $^1$H-NMR ($CDCl_3$) δ (ppm) | MS |
|---|---|---|
| 43 | 0.72 (1.5H, s, $CH_3$), 0.86 (3H, s, $CH_3$), 0.88 (1.5H, s, $CH_3$), 0.97 (1.5H, s, $CH_3$), 0.98 (1.5H, s, $CH_3$), 1.1 ～ 1.9 (5H, m, $CH_2$×2, CH), 2.92 (1H, d, $CH_2$, J=13.5 Hz), 3.32 (1H, dd, $CH_2$, J=2.6, 11.9 Hz), 3.46 (1H, d, $CH_2$, J=11.9 Hz), 3.89 (1H, d, $CH_2$, J=13.2 Hz), 4.10 (1H, d, $CH_2$, J=13.2 Hz), 4.18 (1H, d, $CH_2$, J=13.5 Hz), 7.34 (1H, d, Ar-H, J=6.9 Hz), 7.43 (1H, dd, Ar-H, J=7.3, 7.9 Hz), 7.4 ～ 7.6 (2H, m, Ar-H), 7.77 (1H, d, Ar-H, J=7.9 Hz), 7.87 (1H, d, Ar-H, J=7.3 Hz), 7.97 (1H, d, Ar-H, J=7.6 Hz) | |
| 44 | 0.75 (1.5H, s, $CH_3$), 0.83 (3H, s, $CH_3$), 0.86 (1.5H, s, $CH_3$), 0.94 (1.5H, s, $CH_3$), 0.95 (1.5H, s, $CH_3$), 1.3 ～ 1.8 (5H, m, $CH_2$×2, CH), 2.87 (1H, d, $CH_2$, J=13.5 Hz), 3.14 (1H, d, $CH_2$, J=13.5 Hz), 3.86 (1H, d, $CH_2$, J=13.5 Hz), 3.88 (2H, s, $CH_2$), 4.08 (1H, dd, $CH_2$, J=2.6, 13.5 Hz), 7.3 ～ 7.5 (3H, m, Ar-H), 7.68 (1H, s, Ar-H), 7.8 ～ 7.9 (3H, m, Ar-H) | |
| 45 | 0.81 (3H, s, $CH_3$), 0.88 (3H, s, $CH_3$), 0.94 (3H, | (FAB) |

| | | |
|---|---|---|
| | s, $CH_3$), 1.5 ～ 1.8 (5H, m, $CH_2$×2, CH), 2.33 (1H, d, $CH_2$, J=10.2 Hz), 2.45 (1H, d, $CH_2$, J= 10.2 Hz), 2.6 ～ 2.8 (4H, m, $CH_2$× 2 ) , 3.23 (2H, dd, $CH_2$, J=7.6, 8.3 Hz), 7.39 (2H, d, Ar-H, J=5.6 Hz), 7.5 ～ 7.6 (2H, m, Ar-H), 7.6 ～ 7.7 (1H, m, Ar-H), 7.85 (1H, d, Ar-H, J=7.6 Hz), 8.08 (1H, d, Ar-H, J=7.6 Hz) | 308 ($MH^+$ ) |
| 46 | 0.80 (3H, s, $CH_3$), 0.86 (3H, s, $CH_3$), 0.93 (3H, s, $CH_3$), 1.5 ～ 1.8 (5H, m, $CH_2$×2, CH), 2.30 (1H, d, $CH_2$, J=9.9 Hz), 2.41 (1H, d, $CH_2$, J= 10.9 Hz), 2.6 ～ 2.8 (4H, m, $CH_2$×2), 2.93 (2H, t, $CH_2$, J=7.6 Hz), 7.3 ～ 7.5 (3H, m, Ar-H), 7.68 (1H, s, Ar-H), 7.7 ～ 7.9 (3H, m, Ar-H) | (FAB) 308 ($MH^+$ ) |
| 47 | 0.77 (3H, s, $CH_3$), 0.84 (3H, s, $CH_3$), 0.91 (3H, s, $CH_3$), 1.4 ～ 1.8 (5H, m, $CH_2$×2, CH), 2.24 (1H, d, $CH_2$, J=10.2 Hz), 2.40 (1H, d, $CH_2$, J= 10.9 Hz), 2.5 ～ 2.7 (2H, m, $CH_2$), 2.78 (2H, t, $CH_2$, J=7.9 Hz), 2.95 (2H, t, $CH_2$, J=7.3 Hz), 7.10 (1H, dd, pyridine, J=6.3, 7.6 Hz), 7.2 ～ 7.3 (1H, m, pyridine), 7.58 (1H, dt, pyridine, J=2.0, 7.6 Hz), 8.51 (1H, d, pyridine, J=4.0 Hz) | (FAB) 259 ($MH^+$ ) |
| 48 | 0.78 (3H, s, $CH_3$), 0.85 (3H, s, $CH_3$), 0.91 (3H, s, $CH_3$), 1.4 ～ 1.7 (5H, m, $CH_2$×2, CH), 2.21 (1H, d, $CH_2$, J=10.6 Hz), 2.36 (1H, d, $CH_2$, J= 10.2 Hz), 2.5 ～ 2.8 (6H, m, $CH_2$×3), 7.16 (2H, d, pyridine, J=5.9 Hz), 8.48 (2H, d, pyridine, | (FAB) 259 ($MH^+$ ) |

| | | |
|---|---|---|
| | J=5.9 Hz) | |
| 49 | 0.81 (3H, s, $CH_3$), 0.88 (3H, s, $CH_3$), 0.94 (3H, s, $CH_3$), 1.5 ~ 1.8 (5H, m, $CH_2$×2, CH), 2.33 (1H, d, J=10.2 Hz, $CH_2$), 2.45 (1H, d, J=10.6 Hz, $CH_2$), 2.62 (1H, dd, J=10.2, 10.6 Hz, $CH_2$), 2.76 (2H, dd, J=7.6, 8.3 Hz, $CH_2$), 2.78 (1H, d, J=10.6 Hz, $CH_2$), 3.24 (2H, dd, J=7.6, 8.3 Hz, $CH_2$), 7.4 ~ 7.6 (4H, m, Ar-H), 7.71 (1H, dd, J=4.0, 5.6 Hz, Ar-H), 7.85 (1H, d, J=7.6 Hz, Ar-H), 8.08 (1H, d, J=8.6 Hz, Ar-H) | (FAB) 308 ($MH^+$) |
| 50 | 0.80 (3H, s, $CH_3$), 0.86 (3H, s, $CH_3$), 0.93 (3H, s, $CH_3$), 1.5 ~ 1.9 (5H, m, $CH_2$×2, CH), 2.30 (1H, d, J=10.6 Hz, $CH_2$), 2.40 (1H, d, J=10.6 Hz, $CH_2$), 2.6 ~ 2.8 (4H, m, $CH_2$×2), 2.93 (2H, dd, J=6.9, 8.3 Hz, $CH_2$), 7.3 ~ 7.5 (3H, m, Ar-H), 7.68 (1H, s, Ar-H), 7.7 ~ 7.9 (3H, m, Ar-H) | (FAB) 308 ($MH^+$) |
| 51 | 0.78 (3H, s, $CH_3$), 0.85 (3H, s, $CH_3$), 0.91 (3H, s, $CH_3$), 1.4 ~ 1.8 (5H, m, $CH_2$×2, CH), 2.2 ~ 2.3 (1H, m, $CH_2$), 2.41 (1H, d, J=10.2 Hz, $CH_2$), 2.5 ~ 3.0 (6H, m, $CH_2$×3), 7.10 (1H, dd, J=4.3, 7.6 Hz, pyridine), 7.23 (1H, d, J=8.2 Hz, pyridine), 7.58 (1H, dt, J=1.3, 7.6 Hz, pyridine), 8.51 (1H, d, J=5.0 Hz, pyridine) | (FAB) 259 ($MH^+$) |
| 52 | 0.78 (3H, s, $CH_3$), 0.85 (3H, s, $CH_3$), 0.91 (3H, s, $CH_3$), 1.5 ~ 1.8 (5H, m, $CH_2$×2, CH), 2.22 | (FAB) 259 |

| | | |
|---|---|---|
| | (1H, d, J=10.6 Hz, $CH_2$), 2.36 (1H, d, J=10.6 Hz, $CH_2$), 2.5 ~ 2.8 (6H, m, $CH_2 \times 3$), 7.20 (1H, dd, J=7.6, 7.9 Hz, pyridine), 7.57 (1H, d, J=7.9 Hz, pyridine), 8.43 (1H, d, J=3.6 Hz, pyridine), 8.48 (1H, d, J=2.0 Hz, pyridine) | ($MH^+$) |
| 53 | 0.78 (3H, s, $CH_3$), 0.85 (3H, s, $CH_3$), 0.91 (3H, s, $CH_3$), 1.5 ~ 1.8 (5H, m, $CH_2 \times 2$, CH), 2.21 (1H, d, J=10.2 Hz, $CH_2$), 2.36 (1H, d, J=10.6 Hz, $CH_2$), 2.50 (1H, dd, J=10.2, 10.6 Hz, $CH_2$), 2.60 (1H, d, J=10.6 Hz, $CH_2$), 2.65 (2H, t, J=6.6 Hz, $CH_2$), 2.75 (2H, t, J=6.6 Hz, $CH_2$), 7.17 (2H, d, J=5.6 Hz, pyridine), 8.47 (2H, d, J=5.9 Hz, pyridine) | (FAB) 259 ($MH^+$) |
| 54 | 0.79 (3H, s, $CH_3$), 0.86 (3H, s, $CH_3$), 0.91 (3H, s, $CH_3$), 1.4 ~ 1.8 (5H, m, $CH_2 \times 2$, CH), 2.25 (1H, d, J=10.2 Hz, $CH_2$), 2.35 (1H, d, J=10.2 Hz, $CH_2$), 2.5 ~ 2.8 (4H, m, $CH_2 \times 2$), 5.91 (2H, s, $CH_2$), 6.7 ~ 6.8 (3H, m, Ar-H) | (FAB) 301 ($MH^+$) |
| 55 | 0.79 (3H, s, $CH_3$), 0.86 (3H, s, $CH_3$), 0.92 (3H, s, $CH_3$), 1.5 ~ 1.8 (5H, m, $CH_2 \times 2$, CH), 2.27 (1H, d, J=10.6 Hz, $CH_2$), 2.36 (1H, d, J=10.6 Hz, $CH_2$), 2.58 (2H, d, J=3.0 Hz, $CH_2$), 2.66 (2H, dd, J=6.9, 7.3 Hz, $CH_2$), 2.97 (2H, dd, J=6.9, 7.3 Hz, $CH_2$), 6.83 (1H, d, J=3.3 Hz, thiophene), 6.91 (1H, dd, J=3.3, 5.3 Hz, thiophene), 7.12 (1H, d, J=5.3 Hz, thiophene) | (FAB) 264 ($MH^+$) |

| | | |
|---|---|---|
| 31 | 0.95 (3H, s, $CH_3$), 1.04 (3H, s, $CH_3$), 1.10 (3H, s, $CH_3$), 1.5 ~ 1.7 (1H, m, $CH_2$), 1.7 ~ 1.8 (1H, m, $CH_2$), 1.8 ~ 1.9 (1H, m, CH), 2.0 ~ 2.2 (2H, m, $CH_2$), 3.00 (1H, d, $CH_2$, J= 10.9 Hz), 3.48 (1H, dd, $CH_2$, J=3.0, 10.9 Hz), 5.50 (1H, bs, NH) | (FAB) 168 ($MH^+$) |
| 32 | 0.75 (3H, s, $CH_3$), 0.86 (3H, s, $CH_3$), 1.03 (3H, s, $CH_3$), 1.4 ~ 1.7 (4H, m, $CH_2$×2), 1.8 ~ 2.0 (1H, m, CH), 2.13 (1H, d, $CH_2$, J=12.9 Hz), 2.42 (1H, dd, $CH_2$, J=3.0, 12.9 Hz), 2.86 (1H, d, $CH_2$, J=13.2 Hz), 3.16 (1H, d, $CH_2$, J=12.9 Hz) | (EI) 153 ($M^+$) |

## Claims

1. Azepine derivative of the formula

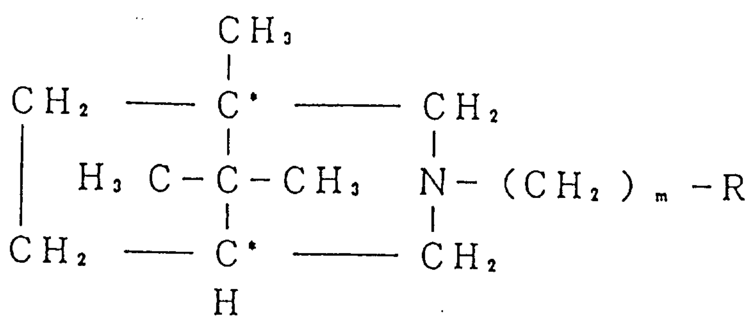

(1)

wherein R is the formula

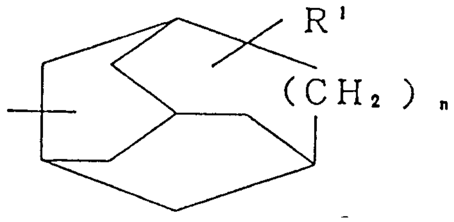

(2)

in which $R^1$ is hydrogen, $C_{1-4}$ alkyl, $C_{1-4}$ alkoxy, hydroxy, halogen or phenyl optionally substituted by 1-3 groups of $C_{1-4}$ alkyl, $C_{1-4}$ alkoxy, hydroxy or halogen, n is 0 or 1, cycloalkyl of $C_{5-8}$ which is optionally substituted by $C_{1-4}$ alkyl, norbornyl, bicyclo [3.3.1 ] nonyl, naphthyl, 1,3 benzodioxolyl, pyridyl or thienyl, m is an integer of 0-4, and C* is an asymmetric carbon, or a nontoxic salt thereof.

**2.** The compound according to claim 1, wherein the azepine derivative of the formula (1) is selected from the group consisting of

(1S)-3-[2-(1-adamantyl)ethyl]-1,8,8-trimethyl-3-azabicylo[3.2.1]octane,
(1S)-3-[2-(1-norbornyl)ethyl]-1,8,8-trimethyl-3-azabicylo[3.2.1]octane,
(1S)-3-(2-cyclopentylethyl)-1,8,8-trimethyl-3-azabicylo[3.2.1]octane,
(1S)-3-(2-cyclohexylethyl)-1,8,8-trimethyl-3-azabicylo[3.2.1]octane,
(1R)-3-(2-cyclohexylethyl)-1,8,8-trimethyl-3-azabicylo[3.2.1]octane,
(1S)-3-(2-cycloheptylethyl)-1,8,8-trimethyl-3-azabicylo[3.2.1]octane,
(1S)-3-(2-cyclooctylethyl)-1,8,8-trimethyl-3-azabicylo[3.2.1]octane,
(1S)-3-(4-cyclohexyl-1-butyl)-1,8,8-trimethyl-3-azabicylo[3.2.1]octane,
(1S)-3-(3-cyclohexylpropyl)-1,8,8-trimethyl-3-azabicylo[3.2.1]octane,
(1S)-3-cyclohexylmethyl-1,8,8-trimethyl-3-azabicylo[3.2.1]octane,

(1R)-3-cyclohexylmethyl-1,8,8-trimethyl-3-azabicylo[3.2.1]octane,
(1S)-3-cyclohexyl-1,8,8-trimethyl-3-azabicylo[3.2.1]octane,
(1R)-3-[2-(1-adamantyl)ethyl]-1,8,8-trimethyl-3-azabicylo[3.2.1]octane,
(1R)-3-[2-(2-adamantyl)ethyl]-1,8,8-trimethyl-3-azabicylo[3.2.1]octane,
(1S)-3-[2-(4-methylcyclohexyl)ethyl]-1,8,8-trimethyl-3-azabicylo[3.2.1]octane,
(1S)-3-[2-(3-methyl-1-adamantyl)ethyl]-1,8,8-trimethyl-3-azabicylo[3.2.1]octane,
(1S)-3-(3-noradamantylmethyl)-1,8,8-trimethyl-3-azabicylo[3.2.1]octane,
(1S)-3-[2-(bicyclo[3.3.1]nonan-9-yl)-1-ethyl]-1,8,8-trimethyl-3-azabicylo[3.2.1]octane,
(1S)-3-(1-adamantylmethyl)-1,8,8-trimethyl-3-azabicylo[3.2.1]octane,
(1S)-3-[2-(5-hydroxy-2-adamantyl)ethyl]-1,8,8-trimethyl-3-azabicylo[3.2.1]octane,
(1S)-3-[2-(5-methoxy-2-adamantyl)ethyl]-1,8,8-trimethyl-3-azabicylo[3.2.1]octane,
(1S)-3-[2-(5-chloro-2-adamantyl)ethyl]-1,8,8-trimethyl-3-azabicylo[3.2.1]octane,
(1S)-3-[2-(5-phenyl-2-adamantyl)ethyl]-1,8,8-trimethyl-3-azabicylo[3.2.1]octane,
(1S)-3-[2-(4-hydroxy-2-adamantyl)ethyl]-1,8,8-trimethyl-3-azabicylo[3.2.1]octane,
(1S)-3-[2-(4-phenyl-2-adamantyl)ethyl]-1,8,8-trimethyl-3-azabicylo[3.2.1]octane,
(1R)-3-[2-(4-phenyl-2-adamantyl)ethyl]-1,8,8-trimethyl-3-azabicylo[3.2.1]octane,
(1R)-3-[2-(5-methoxy-2-adamantyl)ethyl]-1,8,8-trimethyl-3-azabicylo[3.2.1]octane,
(1R)-3-[2-(5-hydroxy-2-adamantyl)ethyl]-1,8,8-trimethyl-3-azabicylo[3.2.1]octane,
(1R)-3-[2-(5-chloro-2-adamantyl)ethyl]-1,8,8-trimethyl-3-azabicylo[3.2.1]octane,
(1R)-3-[2-(5-phenyl-2-adamantyl)ethyl]-1,8,8-trimethyl-3-azabicylo[3.2.1]octane,
(1R)-3-[2-(4-hydroxy-2-adamantyl)ethyl]-1,8,8-trimethyl-3-azabicylo[3.2.1]octane,
(1R)-3-[2-(3-methyl-1-adamantyl)ethyl]-1,8,8-trimethyl-3-azabicylo[3.2.1]octane,
(1S)-3-[3-(2-adamantyl)-1-propyl]-1,8,8-trimethyl-3-azabicylo[3.2.1]octane,
(1R)-3-[3-(2-adamantyl)-1-propyl]-1,8,8-trimethyl-3-azabicylo[3.2.1]octane,
(1S)-3-[2-(3-noradamantyl)ethyl]-1,8,8-trimethyl-3-azabicylo[3.2.1]octane,
(1S)-3-(2-adamantylmethyl)-1,8,8-trimethyl-3-azabicylo[3.2.1]octane, and
(1R)-3-(2-adamantylmethyl)-1,8,8-trimethyl-3-azabicylo[3.2.1]octane
or a nontoxic salt thereof.

**3.** The compound according to claim 1, wherein the azepine derivative of the formula (1) is selected from the group consisting of

(1R)-3-[2-(1-naphtyl)ethyl]-1,8,8-trimethyl-3-azabicylo[3.2.1]octane,
(1R)-3-[2-(2-naphtyl)ethyl]-1,8,8-trimethyl-3-azabicylo[3.2.1]octane,
(1R)-3-[2-(2-pyridyl)ethyl]-1,8,8-trimethyl-3-azabicylo[3.2.1]octane,
(1R)-3-[2-(4-pyridyl)ethyl]-1,8,8-trimethyl-3-azabicylo[3.2.1]octane,
(1S)-3-[2-(1-naphtyl)ethyl]-1,8,8-trimethyl-3-azabicylo[3.2.1]octane,
(1S)-3-[2-(2-naphtyl)ethyl]-1,8,8-trimethyl-3-azabicylo[3.2.1]octane,

(1S)-3-[2-(2-pyridyl)ethyl]-1,8,8-trimethyl-3-azabicylo[3.2.1]octane,
(1S)-3-[2-(3-pyridyl)ethyl]-1,8,8-trimethyl-3-azabicylo[3.2.1]octane,
(1S)-3-[2-(4-pyridyl)ethyl]-1,8,8-trimethyl-3-azabicylo[3.2.1]octane,
(1S)-3-[2-(1,3-benzodioxol-5-yl)ethyl]-1,8,8-trimethyl-3-azabicylo[3.2.1]octane, and
(1S)-3-[2-(2-thienyl)ethyl]-1,8,8-trimethyl-3-azabicylo[3.2.1]octane
or a nontoxic salt thereof.

4. The compound according to claim 1, wherein the azepine derivative of the formula (1) is

(1S)-3-[2-(2-adamantyl)ethyl]-1,8,8-trimethyl-3-azabicylo[3.2.1]octane
or a nontoxic salt thereof.

5. A process for the production of an azepine derivative of the formula (1) defined in claim 1 or a nontoxic salt thereof comprising reducing a carbonyl group of the compound of the formula

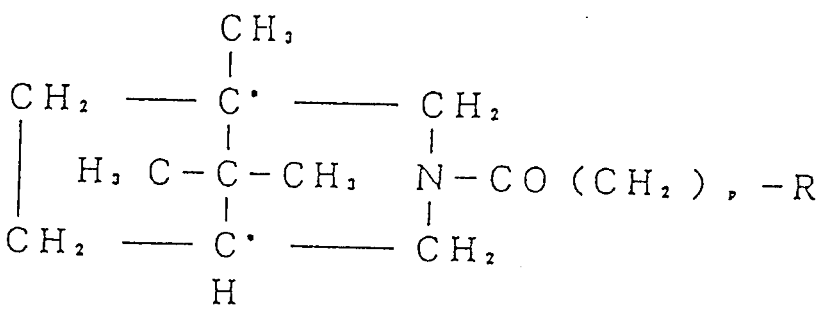 (3)

wherein R has the same meanings as in claim 1, p is an integer of 0-3, and C* is an asymmetric carbon, and if required, converting the compound to a nontoxic salt thereof.

6. A process according to claim 5 wherein the azepine derivative of the formula (1) defined in claim 1 is selected from the group consisting of

(1S)-3-[2-(1-adamantyl)ethyl]-1,8,8-trimethyl-3-azabicyclo[3.2.1]octane,
(1S)-3-[2-(1-norbornyl)ethyl]-1,8,8-trimethyl-3-azabicyclo[3.2.1]octane,
(1S)-3-(2-cyclopentylethyl)-1,8,8-trimethyl-3-azabicyclo[3.2.1]octane,
(1S)-3-(2-cyclohexylethyl)-1,8,8-trimethyl-3-azabicyclo[3.2.1]octane,
(1R)-3-(2-cyclohexylethyl)-1,8,8-trimethyl-3-azabicyclo[3.2.1]octane,
(1S)-3-(2-cycloheptylethyl)-1,8,8-trimethyl-3-azabicyclo[3.2.1]octane,
(1S)-3-(2-cyclooctylethyl)-1,8,8-trimethyl-3-azabicyclo[3.2.1]octane,
(1S)-3-(4-cyclohexyl-1-butyl)-1,8,8-trimethyl-3-azabicyclo[3.2.1]octane,
(1S)-3-(3-cyclohexylpropyl)-1,8,8-trimethyl-3-azabicyclo[3.2.1]octane,
(1S)-3-cyclohexylmethyl-1,8,8-trimethyl-3-azabicyclo[3.2.1]octane,
(1R)-3-cyclohexylmethyl-1,8,8-trimethyl-3-azabicyclo[3.2.1]octane,
(1S)-3-cyclohexyl-1,8,8-trimethyl-3-azabicyclo[3.2.1]octane,
(1R)-3-[2-(1-adamantyl)ethyl]-1,8,8-trimethyl-3-azabicyclo[3.2.1]octane,
(1R)-3-[2-(2-adamantyl)ethyl]-1,8,8-trimethyl-3-azabicyclo[3.2.1]octane,
(1S)-3-[2-(4-methylcyclohexyl)ethyl]-1,8,8-trimethyl-3-azabicyclo[3.2.1]octane,
(1S)-3-[2-(3-methyl-1-adamantyl)ethyl]-1,8,8-trimethyl-3-azabicyclo[3.2.1]octane,
(1S)-3-(3-noradamantylmethyl)-1,8,8-trimethyl-3-azabicyclo[3.2.1]octane,
(1S)-3-[2-(bicyclo[3.3.1]nonan-9-yl)-1-ethyl]-1,8,8-trimethyl-3-azabicyclo[3.2.1]octane,
(1S)-3-(1-adamantylmethyl)-1,8,8-trimethyl-3-azabicyclo[3.2.1]octane,
(1S)-3-[2-(5-hydroxy-2-adamantyl)ethyl]-1,8,8-trimethyl-3-azabicyclo[3.2.1]octane,

(1S)-3-[2-(5-methoxy-2-adamantyl)ethyl]-1,8,8-trimethyl-3-azabicylo[3.2.1]octane,
(1S)-3-[2-(5-chloro-2-adamantyl)ethyl]-1,8,8-trimethyl-3-azabicylo[3.2.1]octane,
(1S)-3-[2-(5-phenyl-2-adamantyl)ethyl]-1,8,8-trimethyl-3-azabicylo[3.2.1]octane,
(1S)-3-[2-(4-hydroxy-2-adamantyl)ethyl]-1,8,8-trimethyl-3-azabicylo[3.2.1]octane,
(1S)-3-[2-(4-phenyl-2-adamantyl)ethyl]-1,8,8-trimethyl-3-azabicylo[3.2.1]octane,
(1R)-3-[2-(4-phenyl-2-adamantyl)ethyl]-1,8,8-trimethyl-3-azabicylo[3.2.1]octane,
(1R)-3-[2-(5-methoxy-2-adamantyl)ethyl]-1,8,8-trimethyl-3-azabicylo[3.2.1]octane,
(1R)-3-[2-(5-hydroxy-2-adamantyl)ethyl]-1,8,8-trimethyl-3-azabicylo[3.2.1]octane,
(1R)-3-[2-(5-chloro-2-adamantyl)ethyl]-1,8,8-trimethyl-3-azabicylo[3.2.1]octane,
(1R)-3-[2-(5-phenyl-2-adamantyl)ethyl]-1,8,8-trimethyl-3-azabicylo[3.2.1]octane,
(1R)-3-[2-(4-hydroxy-2-adamantyl)ethyl]-1,8,8-trimethyl-3-azabicylo[3.2.1]octane,
(1R)-3-[2-(3-methyl-1-adamantyl)ethyl]-1,8,8-trimethyl-3-azabicylo[3.2.1]octane,
(1S)-3-[3-(2-adamantyl)-1-propyl]-1,8,8-trimethyl-3-azabicylo[3.2.1]octane,
(1R)-3-[3-(2-adamantyl)-1-propyl]-1,8,8-trimethyl-3-azabicylo[3.2.1]octane,
(1S)-3-[2-(3-noradamantyl)ethyl]-1,8,8-trimethyl-3-azabicylo[3.2.1]octane,
(1S)-3-(2-adamantylmethyl)-1,8,8-trimethyl-3-azabicylo[3.2.1]octane, and
(1R)-3-(2-adamantylmethyl)-1,8,8-trimethyl-3-azabicylo[3.2.1]octane
or a nontoxic salt thereof.

7. A process according to claim 5 wherein the azepine derivative of the formula (1) defined in claim 1 is selected from the group consisting of

(1R)-3-[2-(1-naphtyl)ethyl]-1,8,8-trimethyl-3-azabicylo[3.2.1]octane,
(1R)-3-[2-(2-naphtyl)ethyl]-1,8,8-trimethyl-3-azabicylo[3.2.1]octane,
(1R)-3-[2-(2-pyridyl)ethyl]-1,8,8-trimethyl-3-azabicylo[3.2.1]octane,
(1R)-3-[2-(4-pyridyl)ethyl]-1,8,8-trimethyl-3-azabicylo[3.2.1]octane,
(1S)-3-[2-(1-naphtyl)ethyl]-1,8,8-trimethyl-3-azabicylo[3.2.1]octane,
(1S)-3-[2-(2-naphtyl)ethyl]-1,8,8-trimethyl-3-azabicylo[3.2.1]octane,
(1S)-3-[2-(2-pyridyl)ethyl]-1,8,8-trimethyl-3-azabicylo[3.2.1]octane,
(1S)-3-[2-(3-pyridyl)ethyl]-1,8,8-trimethyl-3-azabicylo[3.2.1]octane,
(1S)-3-[2-(4-pyridyl)ethyl]-1,8,8-trimethyl-3-azabicylo[3.2.1]octane,
(1S)-3-[2-(1,3-benzodioxol-5-yl)ethyl]-1,8,8-trimethyl-3-azabicylo[3.2.1]octane, and
(1S)-3-[2-(2-thienyl)ethyl]-1,8,8-trimethyl-3-azabicylo[3.2.1]octane
or a nontoxic salt thereof.

8. A process according to claim 5 wherein the azepine derivative of the formula (1) defined in claim 1 is

(1S)-3-[2-(2-adamantyl)ethyl]-1,8,8-trimethyl-3-azabicyclo[3.2.1]octane or a nontoxic salt thereof.

**9.** A process for the production of an azepine derivative of the formula (1) defined in claim 1 or a nontoxic salt thereof, comprising reacting an amine of the formula

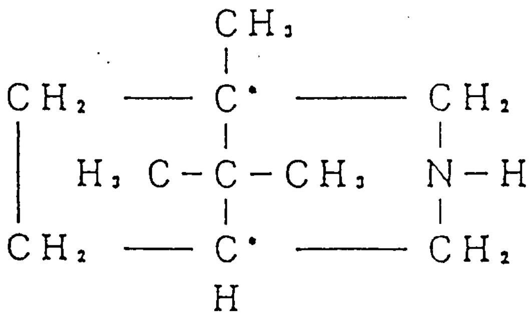

(4)

wherein C* is an asymmetric carbon, with a reactive derivative of the formula

$$X\text{-}(CH_2)m\text{-}R$$

wherein X is a reactive organic sulfonyloxy or a halogen, R has the same meanings as in claim 1, and m is an integer of 0-4, in an inert organic solvent in the presence of base, and, if required, converting the compound to a nontoxic salt thereof.

**10.** A process according to claim 9 wherein the azepine derivative of the formula (1) defined in claim 1 is selected from the group consisting of

(1S)-3-[2-(1-adamantyl)ethyl]-1,8,8-trimethyl-3-azabicyclo[3.2.1]octane,
(1S)-3-[2-(1-norbornyl)ethyl]-1,8,8-trimethyl-3-azabicyclo[3.2.1]octane,
(1S)-3-(2-cyclopentylethyl)-1,8,8-trimethyl-3-azabicyclo[3.2.1]octane,
(1S)-3-(2-cyclohexylethyl)-1,8,8-trimethyl-3-azabicyclo[3.2.1]octane,
(1R)-3-(2-cyclohexylethyl)-1,8,8-trimethyl-3-azabicyclo[3.2.1]octane,
(1S)-3-(2-cycloheptylethyl)-1,8,8-trimethyl-3-azabicyclo[3.2.1]octane,
(1S)-3-(2-cyclooctylethyl)-1,8,8-trimethyl-3-azabicyclo[3.2.1]octane,
(1S)-3-(4-cyclohexyl-1-butyl)-1,8,8-trimethyl-3-azabicyclo[3.2.1]octane,
(1S)-3-(3-cyclohexylpropyl)-1,8,8-trimethyl-3-azabicyclo[3.2.1]octane,
(1S)-3-cyclohexylmethyl-1,8,8-trimethyl-3-azabicyclo[3.2.1]octane,

(1R)-3-cyclohexylmethyl-1,8,8-trimethyl-3-azabicyclo[3.2.1]octane,
(1S)-3-cyclohexyl-1,8,8-trimethyl-3-azabicyclo[3.2.1]octane,
(1R)-3-[2-(1-adamantyl)ethyl]-1,8,8-trimethyl-3-azabicyclo[3.2.1]octane,
(1R)-3-[2-(2-adamantyl)ethyl]-1,8,8-trimethyl-3-azabicyclo[3.2.1]octane,
(1S)-3-[2-(4-methylcyclohexyl)ethyl]-1,8,8-trimethyl-3-azabicyclo[3.2.1]octane,
(1S)-3-[2-(3-methyl-1-adamantyl)ethyl]-1,8,8-trimethyl-3-azabicyclo[3.2.1]octane,
(1S)-3-(3-noradamantylmethyl)-1,8,8-trimethyl-3-azabicyclo[3.2.1]octane,
(1S)-3-[2-(bicyclo[3.3.1]nonan-9-yl)-1-ethyl]-1,8,8-trimethyl-3-azabicyclo[3.2.1]octane,
(1S)-3-(1-adamantylmethyl)-1,8,8-trimethyl-3-azabicyclo[3.2.1]octane,
(1S)-3-[2-(5-hydroxy-2-adamantyl)ethyl]-1,8,8-trimethyl-3-azabicyclo[3.2.1]octane,
(1S)-3-[2-(5-methoxy-2-adamantyl)ethyl]-1,8,8-trimethyl-3-azabicyclo[3.2.1]octane,
(1S)-3-[2-(5-chloro-2-adamantyl)ethyl]-1,8,8-trimethyl-3-azabicyclo[3.2.1]octane,
(1S)-3-[2-(5-phenyl-2-adamantyl)ethyl]-1,8,8-trimethyl-3-azabicyclo[3.2.1]octane,
(1S)-3-[2-(4-hydroxy-2-adamantyl)ethyl]-1,8,8-trimethyl-3-azabicyclo[3.2.1]octane,
(1S)-3-[2-(4-phenyl-2-adamantyl)ethyl]-1,8,8-trimethyl-3-azabicyclo[3.2.1]octane,
(1R)-3-[2-(4-phenyl-2-adamantyl)ethyl]-1,8,8-trimethyl-3-azabicyclo[3.2.1]octane,
(1R)-3-[2-(5-methoxy-2-adamantyl)ethyl]-1,8,8-trimethyl-3-azabicyclo[3.2.1]octane,
(1R)-3-[2-(5-hydroxy-2-adamantyl)ethyl]-1,8,8-trimethyl-3-azabicyclo[3.2.1]octane,
(1R)-3-[2-(5-chloro-2-adamantyl)ethyl]-1,8,8-trimethyl-3-azabicyclo[3.2.1]octane,
(1R)-3-[2-(5-phenyl-2-adamantyl)ethyl]-1,8,8-trimethyl-3-azabicyclo[3.2.1]octane,
(1R)-3-[2-(4-hydroxy-2-adamantyl)ethyl]-1,8,8-trimethyl-3-azabicyclo[3.2.1]octane,
(1R)-3-[2-(3-methyl-1-adamantyl)ethyl]-1,8,8-trimethyl-3-azabicyclo[3.2.1]octane,
(1S)-3-[3-(2-adamantyl)-1-propyl]-1,8,8-trimethyl-3-azabicyclo[3.2.1]octane,
(1R)-3-[3-(2-adamantyl)-1-propyl]-1,8,8-trimethyl-3-azabicyclo[3.2.1]octane,
(1S)-3-[2-(3-noradamantyl)ethyl]-1,8,8-trimethyl-3-azabicyclo[3.2.1]octane,
(1S)-3-(2-adamantylmethyl)-1,8,8-trimethyl-3-azabicyclo[3.2.1]octane, and
(1R)-3-(2-adamantylmethyl)-1,8,8-trimethyl-3-azabicyclo[3.2.1]octane
or a nontoxic salt thereof.

**11.** A process according to claim 9 wherein the azepine derivative of the formula (1) defined in claim 1 is selected from the group consisting of

(1R)-3-[2-(1-naphtyl)ethyl]-1,8,8-trimethyl-3-azabicyclo[3.2.1]octane,
(1R)-3-[2-(2-naphtyl)ethyl]-1,8,8-trimethyl-3-azabicyclo[3.2.1]octane,
(1R)-3-[2-(2-pyridyl)ethyl]-1,8,8-trimethyl-3-azabicyclo[3.2.1]octane,
(1R)-3-[2-(4-pyridyl)ethyl]-1,8,8-trimethyl-3-azabicyclo[3.2.1]octane,
(1S)-3-[2-(1-naphtyl)ethyl]-1,8,8-trimethyl-3-azabicyclo[3.2.1]octane,
(1S)-3-[2-(2-naphtyl)ethyl]-1,8,8-trimethyl-3-azabicyclo[3.2.1]octane,

(1S)-3-[2-(2-pyridyl)ethyl]-1,8,8-trimethyl-3-azabicyclo[3.2.1]octane,
(1S)-3-[2-(3-pyridyl)ethyl]-1,8,8-trimethyl-3-azabicyclo[3.2.1]octane,
(1S)-3-[2-(4-pyridyl)ethyl]-1,8,8-trimethyl-3-azabicyclo[3.2.1]octane,
(1S)-3-[2-(1,3-benzodioxol-5-yl)ethyl]-1,8,8-trimethyl-3-azabicyclo[3.2.1]octane, and
(1S)-3-[2-(2-thienyl)ethyl]-1,8,8-trimethyl-3-azabicyclo[3.2.1]octane
or a nontoxic salt thereof.

**12.** A process according to claim 9 wherein the azepine derivative of the formula (1) defined in claim 1 is

(1S)-3-[2-(2-adamantyl)ethyl]-1,8,8-trimethyl-3-azabicyclo[3.2.1]octane
or a nontoxic salt thereof.

**13.** A medicament for treatment of deseases involved in σ-receptor comprising an azepine derivative of the formula (1) defined in claim 1 or a nontoxic salt thereof as an active ingredient.

**14.** A medicament for treatment of schizophrenia containing an azepine derivative of the formula (1) defined claim 1 or a nontoxic salt thereof as an active ingredient.

**15.** Azepine derivative of the formula

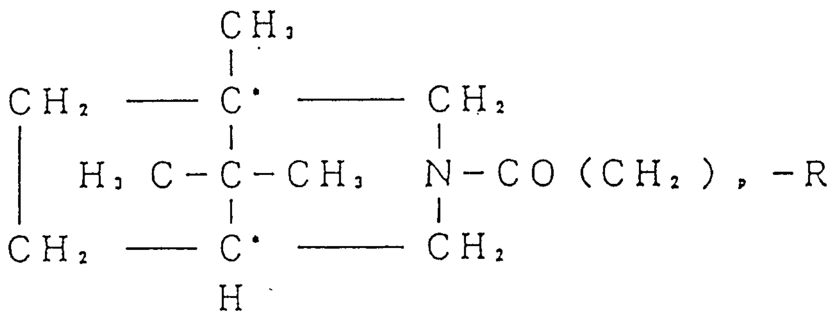 (3)

wherein R is the formula

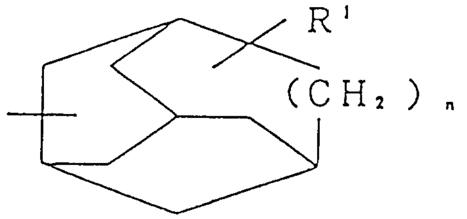

(2)

in which $R^1$ is hydrogen, $C_{1-4}$ alkyl, $C_{1-4}$ alkoxy, hydroxy, halogen or phenyl optionally substituted by 1-3 groups of $C_{1-4}$ alkyl, $C_{1-4}$ alkoxy, hydroxy or halogen and n is 0 or 1, cycloalkyl of $C_{5-8}$ which is optionally substituted by $C_{1-4}$ alkyl, norbornyl, bicyclo [3.3.1 ] nonyl, naphthyl, 1,3-benzodioxolyl, pyridyl or thienyl, p is an integer of 0-3, and C* is an asymmetric carbon, or a nontoxic salt thereof.

**Patentansprüche**

**1.** Azepinderivat der Formel

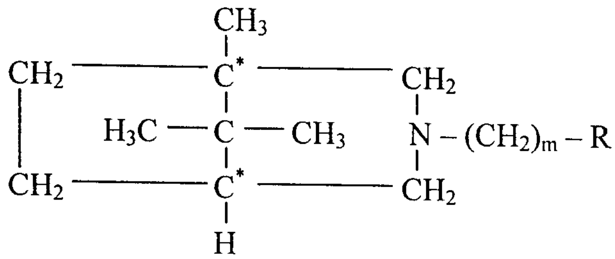

(1)

worin R die Formel

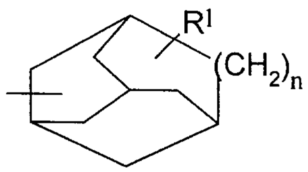

(2)

ist,

in welcher $R^1$ Wasserstoff $C_{1-4}$-Alkyl, $C_{1-4}$-Alkoxy, Hydroxy, Halogen oder Phenyl, welches gegebenenfalls durch 1-3 Gruppen aus $C_{1-4}$-Alkl, $C_{1-4}$-Alkoxy, Hydroxy oder Halogen substituiert ist, n = 0 oder 1 ist, $C_{5-8}$-Cycloalkyl, welches gegebenenfalls durch $C_{1-4}$-Alkyl substituiert ist, Norbornyl, Bicyclo[3.3.1]nonyl, Naphthyl, 1,3-Benzodioxolyl, Pyridyl oder Thienyl ist, m eine ganze Zahl von 0-4 ist und C* ein asymmetrischer Kohlenstoff ist, oder ein nichttoxisches Salz davon.

**2.** Die Verbindung gemäß Anspruch 1, worin das Azepinderivat der Formel (1) aus der Gruppe, bestehend aus

(1S)-3-[2-(1-Adamantyl)ethyl]-1,8,8-trimethyl-3-azabicyclo[3.2.1]octan,
(1S)-3-[2-(1-Norbornyl)ethyl]-1,8,8-trimethyl-3-azabicyclo[3.2.1]octan,
(1S)-3-(2-Cyclopentylethyl)-1,8,8-trimethyl-3-azabicyclo[3.2.1]octan,
(1S)-3-(2-Cyclohexylethyl)-1,8,8-trimethyl-3-azabicyclo[3.2.1]octan,
(1R)-3-(2-Cyclohexylethyl)-1,8,8-trimethyl-3-azabicyclo[3.2.1]octan,
(1S)-3-(2-Cycloheptylethyl)-1,8,8-trimethyl-3-azabicyclo[3.2.1]octan,

(1S)-3-(2-Cyclooctylethyl)-1,8,8-trimethyl-3-azabicyclo[3.2.1]octan,
(1S)-3-(4-Cyclohexyl-1-butyl)-1,8,8-trimethyl-3-azabicyclo[3.2.1]octan,
(1S)-3-(3-Cyclohexylpropyl)-1,8,8-trimethyl-3-azabicyclo[3.2.1]octan,
(1S)-3-Cyclohexylmethyl-1,8,8-trimethyl-3-azabicyclo[3.2.1]octan,
(1R)-3-Cyclohexylmethyl-1,8,8-trimethyl-3-azabicyclo[3.2.1]octan,
(1S)-3-Cyclohexyl-1,8,8-trimethyl-3-azabicyclo[3.2.1]octan,
(1R)-3-[2-(1-Adamantyl)ethyl]-1,8,8-trimethyl-3-azabicyclo[3.2.1]octan,
(1R)-3-[2-(2-Adamantyl)ethyl]-1,8,8-trimethyl-3-azabicyclo[3.2.1]octan,
(1S)-3-[2-(4-Methylcyclohexyl)ethyl]-1,8,8-trimethyl-3-azabicyclo[3.2.1]octan,
(1S)-3-[2-(3-Methyl-1-adamantyl)ethyl]-1,8,8-trimethyl-3-azabicyclo[3.2.1]octan,
(1S)-3-(3-Noradamantylmethyl)-1,8,8-trimethyl-3-azabicyclo[3.2.1]octan,
(1S)-3-[2-(Bicyclo[3.3.1]nonan-9-yl)-1-ethyl]-1,8,8-trimethyl-3-azabicyclo[3.2.1]octan,
(1S)-3-(1-Adamantylmethyl)-1,8,8-trimethyl-3-azabicyclo[3.2.1]octan,
(1S)-3-[2-(5-Hydroxy-2-adamantyl)ethyl]-1,8,8-trimethyl-3-azabicyclo[3.2.1]octan,
(1S)-3-[2-(5-Methoxy-2-adamantyl)ethyl]-1,8,8-trimethyl-3-azabicyclo[3.2.1]octan,
(1S)-3-[2-(5-Chloro-2-adamantyl)ethyl]-1,8,8-trimethyl-3-azabicyclo[3.2.1]octan,
(1S)-3-[2-(5-Phenyl-2-adamantyl)ethyl]-1,8,8-trimethyl-3-azabicyclo[3.2.1]octan,
(1S)-3-[2-(4-Hydroxy-2-adamantyl)ethyl]-1,8,8-trimethyl-3-azabicyclo[3.2.1]octan,
(1S)-3-[2-(4-Phenyl-2-adamantyl)ethyl]-1,8,8-trimethyl-3-azabicyclo[3.2.1]octan,
(1R)-3-[2-(4-Phenyl-2-adamantyl)ethyl]-1,8,8-trimethyl-3-azabicyclo[3.2.1]octan,
(1R)-3-[2-(5-Methoxy-2-adamantyl)ethyl]-1,8,8-trimethyl-3-azabicyclo[3.2.1]octan,
(1R)-3-[2-(5-Hydroxy-2-adamantyl)ethyl]-1,8,8-trimethyl-3-azabicyclo[3.2.1]octan,
(1R)-3-[2-(5-Chloro-2-adamantyl)ethyl]-1,8,8-trimethyl-3-azabicyclo[3.2.1]octan,
(1R)-3-[2-(5-Phenyl-2-adamantyl)ethyl]-1,8,8-trimethyl-3-azabicyclo[3.2.1]octan,
(1R)-3-[2-(4-Hydroxy-2-adamantyl)ethyl]-1,8,8-trimethyl-3-azabicyclo[3.2.1]octan,
(1R)-3-[2-(3-Methyl-1-adamantyl)ethyl]-1,8,8-trimethyl-3-azabicyclo[3.2.1]octan,
(1S)-3-[3-(2-Adamantyl)-1-propyl]-1,8,8-trimethyl-3-azabicyclo[3.2.1]octan,
(1R)-3-[3-(2-Adamantyl)-1-propyl]-1,8,8-trimethyl-3-azabicyclo[3.2.1]octan,
(1S)-3-[2-(3-Noradamantyl)ethyl]-1,8,8-trimethyl-3-azabicyclo[3.2.1]octan,
(1S)-3-(2-Adamantylmethyl)-1,8,8-trimethyl-3-azabicyclo[3.2.1]octan und
(1R)-3-(2-Adamantylmethyl)-1,8,8-trimethyl-3-azabicyclo[3.2.1]octan
oder einem nichttoxischen Salz davon, gewählt ist.

3. Die Verbindung gemäß Anspruch 1, worin das Azepinderivat der Formel (1) aus der Gruppe, bestehend aus

(1R)-3-[2-(1-Naphthyl)ethyl]-1,8,8-trimethyl-3-azabicyclo[3.2.1]octan,
(1R)-3-[2-(2-Naphthyl)ethyl]-1,8,8-trimethyl-3-azabicyclo[3.2.1]octan,
(1R)-3-[2-(2-Pyridyl)ethyl]-1,8,8-trimethyl-3-azabicyclo[3.2.1]octan,
(1R)-3-[2-(4-Pyridyl)ethyl]-1,8,8-trimethyl-3-azabicyclo[3.2.1]octan,
(1S)-3-[2-(1-Naphthyl)ethyl]-1,8,8-trimethyl-3-azabicyclo[3.2.1]octan,
(1S)-3-[2-(2-Naphthyl)ethyl]-1,8,8-trimethyl-3-azabicyclo[3.2.1]octan,
(1S)-3-[2-(2-Pyridyl)ethyl]-1,8,8-trimethyl-3-azabicyclo[3.2.1]octan,
(1S)-3-[2-(3-Pyridyl)ethyl]-1,8,8-trimethyl-3-azabicyclo[3.2.1]octan,
(1S)-3-[2-(4-Pyridyl)ethyl]-1,8,8-trimethyl-3-azabicyclo[3.2.1]octan,
(1S)-3-[2-(1,3-Benzodioxol-5-yl)ethyl]-1,8,8-trimethyl-3-azabicyclo[3.2.1]octan und
(1S)-3-[2-(2-Thienyl)ethyl]-1,8,8-trimethyl-3-azabicyclo[3.2.1]octan
oder einem nichttoxischen Salz davon, gewählt ist.

4. Die Verbindung gemäß Anspruch 1, worin das Azepinderivat der Formel (1) (1S)-3-[2-(2-Adamantyl)ethyl]-1,8,8-trimethyl-3-azabicyclo[3.2.1]octan oder ein nichttoxisches Salz davon ist.

5. Ein Verfahren zur Herstellung eines Azepinderivats der in Anspruch 1 definierten Formel (1) oder eines nichttoxischen Salzes davon, umfassend das Reduzieren einer Carbonylgruppe der Verbindung der Formel

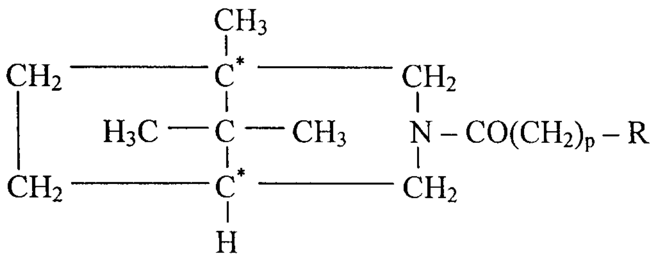

(3)

worin R die gleichen Bedeutungen wie in Anspruch 1 besitzt, p eine ganze Zahl von 0-3 ist und C* ein asymmetrischer Kohlenstoff ist, und falls nötig, das Umsetzen der Verbindung in ein nichttoxisches Salz davon.

**6.** Ein Verfahren gemäß Anspruch 5, wobei das Azepinderivat der in Anspruch 1 definierten Formel (1) aus der Gruppe bestehend aus

(1S)-3-[2-(1-Adamantyl)ethyl]-1,8,8-trimethyl-3-azabicyclo[3.2.1]octan,
(1S)-3-[2-(1-Norbornyl)ethyl]-1,8,8-trimethyl-3-azabicyclo[3.2.1]octan,
(1S)-3-(2-Cyclopentylethyl)-1,8,8-trimethyl-3-azabicyclo[3.2.1]octan,
(1S)-3-(2-Cyclohexylethyl)-1,8,8-trimethyl-3-azabicyclo[3.2.1]octan,
(1R)-3-(2-Cyclohexylethyl)-1,8,8-trimethyl-3-azabicyclo[3.2.1]octan,
(1S)-3-(2-Cycloheptylethyl)-1,8,8-trimethyl-3-azabicyclo[3.2.1]octan,
(1S)-3-(2-Cyclooctylethyl)-1,8,8-trimethyl-3-azabicyclo[3.2.1]octan,
(1S)-3-(4-Cyclohexyl-1-butyl)-1,8,8-trimethyl-3-azabicyclo[3.2.1]octan,
(1S)-3-(3-Cyclohexylpropyl)-1,8,8-trimethyl-3-azabicyclo[3.2.1]octan,
(1S)-3-Cyclohexylmethyl-1,8,8-trimethyl-3-azabicyclo[3.2.1]octan,
(1R)-3-Cyclohexylnlethyl-1,8,8-trimethyl-3-azabicyclo[3.2.1]octan,
(1S)-3-Cyclohexyl-1,8,8-trimethyl-3-azabicyclo[3.2.1]octan,
(1R)-3-[2-(1-Adamantyl)ethyl]-1,8,8-trimethyl-3-azabicyclo[3.2.1]octan,
(1R)-3-[2-(2-Adamantyl)ethyl]-1,8,8-trimethyl-3-azabicyclo[3.2.1]octan,
(1S)-3-[2-(4-Methylcyclohexyl)ethyl]-1,8,8-trimethyl-3-azabicyclo[3.2.1]octan,
(1S)-3-[2-(3-Methyl-1-adamantyl)ethyl]-1,8,8-trimethyl-3-azabicyclo[3.2.1]octan,
(1S)-3-(3-Noradamantylmethyl)-1,8,8-trimethyl-3-azabicyclo[3.2.1]octan,
(1S)-3-[2-(Bicyclo[3.3.1]nonan-9-yl)-1-ethyl]-1,8,8-trimethyl-3-azabicyclo[3.2.1]octan,
(1S)-3-(1-Adamantylmethyl)-1,8,8-trimethyl-3-azabicyclo[3.2.1]octan,
(1S)-3-[2-(5-Hydroxy-2-adamantyl)ethyl]-1,8,8-trimethyl-3-azabicyclo[3.2.1]octan,
(1S)-3-[2-(5-Methoxy-2-adamantyl)ethyl]-1,8,8-trimethyl-3-azabicyclo[3.2.1]octan,
(1S)-3-[2-(5-Chloro-2-adamantyl)ethyl]-1,8,8-trimethyl-3-azabicyclo[3.2.1]octan,
(1S)-3-[2-(5-Phenyl-2-adamantyl)ethyl]-1,8,8-trimethyl-3-azabicyclo[3.2.1]octan,
(1S)-3-[2-(4-Hydroxy-2-adamantyl)ethyl]-1,8,8-trimethyl-3-azabicyclo[3.2.1]octan,
(1S)-3-[2-(4-Phenyl-2-adamantyl)ethyl]-1,8,8-trimethyl-3-azabicyclo[3.2.1]octan,
(1R)-3-[2-(4-Phenyl-2-adamantyl)ethyl]-1,8,8-trimethyl-3-azabicyclo[3.2.1]octan,
(1R)-3-[2-(5-Methoxy-2-adamantyl)ethyl]-1,8,8-trimethyl-3-azabicyclo[3.2.1]octan,
(1R)-3-[2-(5-Hydroxy-2-adamantyl)ethyl]-1,8,8-trimethyl-3-azabicyclo[3.2.1]octan,
(1R)-3-[2-(5-Chloro-2-adamantyl)ethyl]-1,8,8-trimethyl-3-azabicyclo[3.2.1]octan,
(1R)-3-[2-(5-Phenyl-2-adamantyl)ethyl]-1,8,8-trimethyl-3-azabicyclo[3.2.1]octan,
(1R)-3-[2-(4-Hydroxy-2-adamantyl)ethyl]-1,8,8-trimethyl-3-azabicyclo[3.2.1]octan,
(1R)-3-[2-(3-Methyl-1-adamantyl)ethyl]-1,8,8-trimethyl-3-azabicyclo[3.2.1]octan,
(1S)-3-[3-(2-Adamantyl)-1-propyl]-1,8,8-trimethyl-3-azabicyclo[3.2.1]octan,
(1R)-3-[3-(2-Adamantyl)-1-propyl]-1,8,8-trimethyl-3-azabicyclo[3.2.1]octan,
(1S)-3-[2-(3-Noradamantyl)ethyl]-1,8,8-trimethyl-3-azabicyclo[3.2.1]octan,
(1S)-3-(2-Adamantylmethyl)-1,8,8-trimethyl-3-azabicyclo[3.2.1]octan und
(1R)-3-(2-Adamantylmethyl)-1,8,8-trimethyl-3-azabicyclo[3.2.1]octan
oder einem nichttoxischen Salz davon, gewählt ist.

**7.** Ein Verfahren gemäß Anspruch 5, worin das Azepinderivat der in Anspruch 1 definierten Formel (1) aus der

Gruppe bestehend aus

(1R)-3-[2-(1-Naphthyl)ethyl]-1,8,8-trimethyl-3-azabicyclo[3.2.1]octan,
(1R)-3-[2-(2-Naphthyl)ethyl]-1,8,8-trimethyl-3-azabicyclo[3.2.1]octan,
(1R)-3-[2-(2-Pyridyl)ethyl]-1,8,8-trimethyl-3-azabicyclo[3.2.1]octan,
(1R)-3-[2-(4-Pyridyl)ethyl]-1,8,8-trimethyl-3-azabicyclo[3.2.1]octan,
(1S)-3-[2-(1-Naphthyl)ethyl]-1,8,8-trimethyl-3-azabicyclo[3.2.1]octan,
(1S)-3-[2-(2-Naphthyl)ethyl]-1,8,8-trimethyl-3-azabicyclo[3.2.1]octan,
(1S)-3-[2-(2-Pyridyl)ethyl]-1,8,8-trimethyl-3-azabicyclo[3.2.1]octan,
(1S)-3-[2-(3-Pyridyl)ethyl]-1,8,8-trimethyl-3-azabicyclo[3.2.1]octan,
(1S)-3-[2-(4-Pyridyl)ethyl]-1,8,8-trimethyl-3-azabicyclo[3.2.1]octan,
(1S)-3-[2-(1,3-Benzodioxol-5-yl)ethyl]-1,8,8-trimethyl-3-azabicyclo[3.2.1]octan und
(1S)-3-[2-(2-Thienyl)ethyl]-1,8,8-trimethyl-3-azabicyclo[3.2.1]octan
oder einem nichttoxischen Salz davon, gewählt ist.

**8.** Ein Verfahren gemäß Anspruch 5, worin das Azepinderivat der in Anspruch 1 definierten Formel (1)

(1S)-3-[2-(2-Adamantyl)ethyl]-1,8,8-trimethyl-3-azabicyclo[3.2.1]octan
oder ein nichttoxisches Salz davon ist.

**9.** Ein Verfahren zur Herstellung eines Azepinderivats der in Anspruch 1 definierten Formel (1) oder eines nichttoxischen Salzes davon, umfassend das zur Reaktion bringen eines Amins der Formel

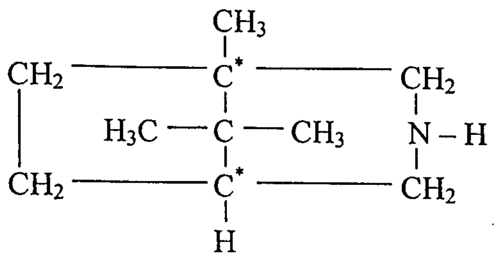

(4)

worin C* ein asymmetrischer Kohlenstoff ist, mit einem reaktiven Derivat der Formel

$$X\text{-}(CH_2)_m\text{-}R$$

worin X für reaktives organisches Sulfonyloxy oder Halogen steht, R die gleichen Bedeutungen wie in Anspruch 1 hat und m eine ganze Zahl von 0-4 ist, in einem inerten organischen Lösungsmittel in Gegenwart einer und wenn nötig, das Umsetzen der Verbindung in ein nichttoxisches Salz davon.

**10.** Ein Verfahren gemäß Anspruch 9, worin das Azepinderivat der in Anspruch 1 definierten Formel (1) aus der Gruppe bestehend aus

(1S)-3-[2-(1-Adamantyl)ethyl]-1,8,8-trimethyl-3-azabicyclo[3.2.1]octan,
(1S)-3-[2-(1-Norbornyl)ethyl]-1,8,8-trimethyl-3-azabicyclo[3.2.1]octan,
(1S)-3-(2-Cyclopentylethyl)-1,8,8-trimethyl-3-azabicyclo[3.2.1]octan,
(1S)-3-(2-Cyclohexylethyl)-1,8,8-trimethyl-3-azabicyclo[3.2.1]octan,
(1R)-3-(2-Cyclohexylethyl)-1,8,8-trimethyl-3-azabicyclo[3.2.1]octan,
(1S)-3-(2-Cycloheptylethyl)-1,8,8-trimethyl-3-azabicyclo[3.2.1]octan,
(1S)-3-(2-Cyclooctylethyl)-1,8,8-trimethyl-3-azabicyclo[3.2.1]octan,
(1S)-3-(4-Cyclohexyl-1-butyl)-1,8,8-trimethyl-3-azabicyclo[3.2.1]octan,
(1S)-3-(3-Cyclohexylpropyl)-1,8,8-trimethyl-3-azabicyclo[3.2.1]octan,
(1S)-3-Cyclohexylmethyl-1,8,8-trimethyl-3-azabicyclo[3.2.1]octan,
(1R)-3-Cyclohexylmethyl-1,8,8-trimethyl-3-azabicyclo[3.2.1]octan,
(1S)-3-Cyclohexyl-1,8,8-trimethyl-3-azabicyclo[3.2.1]octan,
(1R)-3-[2-(1-Adamantyl)ethyl]-1,8,8-trimethyl-3-azabicyclo[3.2.1]octan,

(1R)-3-[2-(2-Adamantyl)ethyl]-1,8,8-trimethyl-3-azabicyclo[3.2.1]octan,
(1S)-3-[2-(4-Methylcyclohexyl)ethyl]-1,8,8-trimethyl-3-azabicyclo[3.2.1]octan,
(1S)-3-[2-(3-Methyl-1-adamantyl)ethyl]-1,8,8-trimethyl-3-azabicyclo[3.2.1]octan,
(1S)-3-(3-Noradamantylmethyl)-1,8,8-trimethyl-3-azabicyclo[3.2.1]octan,
(1S)-3-[2-(Bicyclo[3.3.1]nonan-9-yl)-1-ethyl]-1,8,8-trimethyl-3-azabicyclo[3.2.1]octan,
(1S)-3-(1-Adamantylmethyl)-1,8,8-trimethyl-3-azabicyclo[3.2.1]octan,
(1S)-3-[2-(5-Hydroxy-2-adamantyl)ethyl]-1,8,8-trimethyl-3-azabicyclo[3.2.1]octan,
(1S)-3-[2-(5-Methoxy-2-adamantyl)ethyl]-1,8,8-trimethyl-3-azabicyclo[3.2.1]octan,
(1S)-3-[2-(5-Chloro-2-adamantyl)ethyl]-1,8,8-trimethyl-3-azabicyclo[3.2.1]octan,
(1S)-3-[2-(5-Phenyl-2-adamantyl)ethyl]-1,8,8-trimethyl-3-azabicyclo[3.2.1]octan,
(1S)-3-[2-(4-Hydroxy-2-adamantyl)ethyl]-1,8,8-trimethyl-3-azabicyclo[3.2.1]octan,
(1S)-3-[2-(4-Phenyl-2-adamantyl)ethyl]-1,8,8-trimethyl-3-azabicyclo[3.2.1]octan,
(1R)-3-[2-(4-Phenyl-2-adamantyl)ethyl]-1,8,8-trimethyl-3-azabicyclo[3.2.1]octan,
(1R)-3-[2-(5-Methoxy-2-adamantyl)ethyl]-1,8,8-trimethyl-3-azabicyclo[3.2.1]octan,
(1R)-3-[2-(5-Hydroxy-2-adamantyl)ethyl]-1,8,8-trimethyl-3-azabicyclo[3.2.1]octan,
(1R)-3-[2-(5-Chloro-2-adamantyl)ethyl]-1,8,8-trimethyl-3-azabicyclo[3.2.1]octan,
(1R)-3-[2-(5-Phenyl-2-adamantyl)ethyl]-1,8,8-trimethyl-3-azabicyclo[3.2.1]octan,
(1R)-3-[2-(4-Hydroxy-2-adamantyl)ethyl]-1,8,8-trimethyl-3-azabicyclo[3.21]octan,
(1R)-3-[2-(3-Methyl-1-adamantyl)ethyl]-1,8,8-trimethyl-3-azabicyclo[3.2.1]octan,
(1S)-3-[3-(2-Adamantyl)-1-propyl]-1,8,8-trimethyl-3-azabicyclo[3.2.1]octan,
(1R)-3-[3-(2-Adamantyl)-1-propyl]-1,8,8-trimethyl-3-azabicyclo[3.2.1]octan,
(1S)-3-[2-(3-Noradamantyl)ethyl]-1,8,8-trimethyl-3-azabicyclo[3.2.1]octan,
(1S)-3-(2-Adamantylmethyl)-1,8,8-trimethyl-3-azabicyclo[3.2.1]octan und
(1R)-3-(2-Adamantylmethyl)-1,8,8-trimethyl-3-azabicyclo[3.2.1]octan
oder einem nichttoxischen Salz davon, gewählt ist.

**11.** Ein Verfahren gemäß Anspruch 9, worin das Azepinderivat der in Anspruch 1 definierten Formel (1) aus der Gruppe bestehend aus

(1R)-3-[2-(1-Naphthyl)ethyl]-1,8,8-trimethyl-3-azabicyclo[3.2.1]octan,
(1R)-3-[2-(2-Naphthyl)ethyl]-1,8,8-trimethyl-3-azabicyclo[3.2.1]octan,
(1R)-3-[2-(2-Pyridyl)ethyl]-1,8,8-trimethyl-3-azabicyclo[3.2.1]octan,
(1R)-3-[2-(4-Pyridyl)ethyl]-1,8,8-trimethyl-3-azabicyclo[3.2.1]octan,
(1S)-3-[2-(1-Naphthyl)ethyl]-1,8,8-trimethyl-3-azabicycio[3.2.1]octan,
(1S)-3-[2-(2-Naphthyl)ethyl]-1,8,8-trimethyl-3-azabicyclo[3.2.1]octan,
(1S)-3-[2-(2-Pyridyl)ethyl]-1,8,8-trimethyl-3-azabicyclo[3.2.1]octan,
(1S)-3-[2-(3-Pyridyl)ethyl]-1,8,8-trimethyl-3-azabicyclo[3.2.1]octan,
(1S)-3-[2-(4-Pyridyl)ethyl]-1,8,8-trimethyl-3-azabicyclo[3.2.1]octan,
(1S)-3-[2-(1,3-Benzodioxol-5-yl)ethyl]-1,8,8-trimethyl-3-azabicyclo[3.2.1]octan und
(1S)-3-[2-(2-Thienyl)ethyl]-1,8,8-trimethyl-3-azabicyclo[3.2.1]octan
oder einem nichttoxischen Salz davon, gewählt ist.

**12.** Ein Verfahren gemäß Anspruch 9, worin das Azepinderivat der in Anspruch 1 definierten Formel (1)

(1S)-3-[2-(2-Adamantyl)ethyl]-1,8,8-trimethyl-3-azabicyclo[3.2.1]octan
oder ein nichttoxisches Salz davon ist.

**13.** Ein Medikament zur Behandlung von Krankheiten, die am σ-Rezeptor beteiligt sind, umfassend ein Azepinderivat der in Anspruch 1 definierten Formel (1) oder ein nichttoxisches Salz davon, als aktiven Bestandteil.

**14.** Ein Medikament zur Behandlung von Schizophrenie, enthaltend ein Azepinderivat der in Anspruch 1 definierten Formel (1) oder ein nichttoxische Salz davon, als aktiven Bestandteil.

**15.** Azepinderivat der Formel

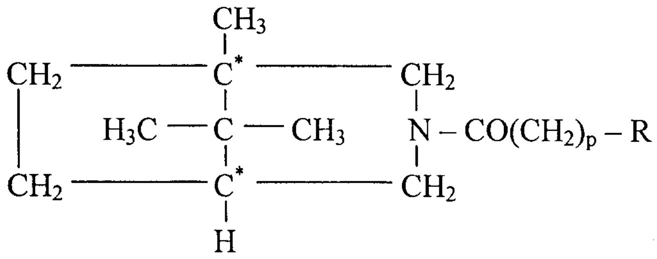

(3)

worin R die Formel

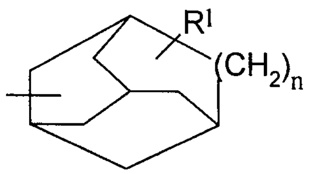

(2)

ist,

in welcher $R^1$ Wasserstoff, $C_{1-4}$-Alkyl, $C_{1-4}$-Alkoxy, Hydroxy, Halogen oder Phenyl, welches gegebenenfalls durch 1-3 Gruppen aus $C_{1-4}$-Alkyl, $C_{1-4}$-Alkoxy, Hydroxy oder Halogen substituiert ist, und n = 0 oder 1 ist,
$C_{5-8}$-Cycloalkyl, welches gegebenenfalls durch $C_{1-4}$-Alkyl substituiert ist,
Norbornyl, Bicyclo[3.3.1]nonyl, Naphthyl, 1,3-Benzodioxolyl, Pyridyl oder Thienyl ist,
p eine ganze Zahl von 0-3 ist und C* ein asymmetrischer Kohlenstoff ist, oder ein nichttoxisches Salz davon.

**Revendications**

1. Dérivé d'azépine de formule

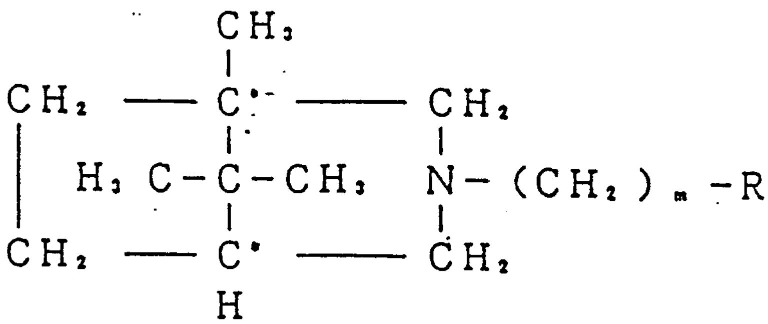

(1)

dans laquelle R a la formule

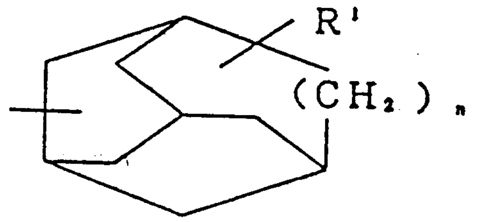

(2)

dans laquelle R1 est l'hydrogène, alcoyle ayant de 1 à 4 atomes de carbone, alcoxy ayant de 1 à 4 atomes de carbone, hydroxy, halogène ou phényle éventuellement substitué par de 1 à 3 groupes alcoyle ayant de 1 à 4 atomes de carbone, alcoxy ayant de 1 à 4 atomes de carbone, hydroxy ou halogène, n est 0 ou 1, cycloalcoyle ayant de 5 à 8 atomes de carbone qui est éventuellement substitue par un alcoyle ayant de 1 à 4 atomes de carbone, norbornyle, bicyclo [3.3.1] nonyle, naphtyle, 1,3-benzodioxolyle, pyridyle ou thiényle, m est un nombre entier de 0 à 4, et C* est un carbone asymétrique, ou l'un de ses sels non toxiques.

**2.** Composé suivant la revendication 1, dans lequel le dérivé d'azépine de formule (I) est choisi dans le groupe consistant en

(1S)-3-[2-(1-adamantyl)éthyl]-1,8,8-triméthyl-3-azabicylo[3.2.1]octane,
(1S)-3-[2-(1-norbornyl)éthyl]-1,8,8-triméthyl-3-azabicylo[3.2.1]octane,
(1S)-3-(2-cyclopentyléthyl)-1,8,8-triméthyl-3-azabicylo[3.2.1]octane,
(1S)-3-(2-cyclohexyléthyl)-1,8,8-triméthyl-3-azabicylo[3.2.1]octane,
(1R)-3-(2-cyclohexyléthyl)-1,8,8-triméthyl-3-azabicylo[3.2.1]octane,
(1S)-3-(2-cycloheptyléthyl)-1,8,8-triméthyl-3-azabicylo[3.2.1]octane,
(1S)-3-(2-cyclooctyléthyl)-1,8,8-triméthyl-3-azabicylo[3.2.1]octane,
(1S)-3-(4-cyclohexyl-1-butyl)-1,8,8-triméthyl-3-azabicylo[3.2.1]octane,
(1S)-3-(3-cyclohexylpropyl)-1,8,8-triméthyl-3-azabicylo[3.2.1]octane,
(1S)-3-cyclohexylméthyl-1,8,8-triméthyl-3-azabicylo[3.2.1]octane,
(1R)-3-cyclohexylméthyl-1,8,8-triméthyl-3-azabicylo[3.2.1]octane,
(1S)-3-cyclohexyl-1,8,8-triméthyl-3-azabicylo[3.2.1]octane,
(1R)-3-[2-(1-adamantyl)éthyl]-1,8,8-triméthyl-3-azabicylo[3.2.1]octane,
(1R)-3-[2-(2-adamantyl)éthyl]-1,8,8-triméthyl-3-azabicylo[3.2.1]octane,
(1S)-3-[2-(4-méthylcyclohexyl)ethyl]-1,8,8-triméthyl-3-azabicylo[3.2.1]octane,
(1S)-3-[2-(3-méthyl-1-adamantyl)éthyl]-1,8,8-triméthyl-3-azabicylo[3.2.1]octane,
(1S)-3-(3-noradamantylméthyl)-1,8,8-triméthyl-3-azabicylo[3.2.1]octane,
(1S)-3-[2-(bicyclo[3.3.1]nonan-9-yl)-1-éthyl]-1,8,8-triméthyl-3-azabicyclo[3.2.1]octane
(1S)-3-(1-adamantylméthyl)-1,8,8-triméthyl-3-azabicylo[3.2.1]octane,
(1S)-3-[2-(5-hydroxy-2-adamantyl)éthyl]-1,8,8-triméthyl-3-azabicylo[3.2.1]octane,
(1S)-3-[2-(5-méthoxy-2-adamantyl)éthyl]-1,8,8-triméthyl-3-azabicylo[3.2.1]octane,
(1S)-3-[2-(5-chloro-2-adamantyl)éthyl]-1,8,8-triméthyl-3-azabicylo[3.2.1]octane,
(1S)-3-[2-(5-phényl-2-adamantyl)éthyl]-1,8,8-triméthyl-3-azabicylo[3.2.1]octane,
(1S)-3-[2-(4-hydroxy-2-adamantyl)éthyl]-1,8,8-triméthyl-3-azabicylo[3.2.1]octane,
(1S)-3-[2-(4-phényl-2-adamantyl)éthyl]-1,8,8-triméthyl-3-azabicylo[3.2.1]octane,
(1R)-3-[2-(4-phényl-2-adamantyl)éthyl]-1,8,8-triméthyl-3-azabicylo[3.2.1]octane,
(1R)-3-[2-(5-méthoxy-2-adamantyl)éthyl]-1,8,8-triméthyl-3-azabicylo[3.2.1]octane,
(1R)-3-[2-(5-hydroxy-2-adamantyl)éthyl]-1,8,8-triméthyl-3-azabicylo[3.2.1]octane,
(1R)-3-[2-(5-chloro-2-adamantyl)éthyl]-1,8,8-triméthyl-3-azabicylo[3.2.1]octane.

(1R)-3-[2-(5-phényl-2-adamantyl)éthyl]-1,8,8-triméthyl-3-azabicylo[3.2.1]octane,
(1R)-3-[2-(4-hydroxy-2-adamantyl)éthyl]-1,8,8-triméthyl-3-azabicylo[3.2.1]octane,
(1R)-3-[2-(3-méthyl-1-adamantyl)éthyl]-1,8,8-triméthyl-3-azabicylo[3.2.1]octane,
(1S)-3-[3-(2-adamantyl)-1-propyl]-1,8,8-triméthyl-3-azabicylo[3.2.1]octane,
(1R)-3-[3-(2-adamantyl)-1-propyl]-1,8,8-triméthyl-3-azabicylo[3.2.1]octane,
(1S)-3-[2-(3-noradamantyl)éthyl]-1,8,8-triméthyl-3-azabicylo[3.2.1]octane,
(1S)-3-(2-adamantylméthyl)-1,8,8-triméthyl-3-azabicylo[3.2.1]octane, et
(1R)-3-(2-adamantylméthyl)-1,8,8-triméthyl-3-azabicylo[3.2.1]octane
ou l'un de leurs sels non toxiques.

3. Composé suivant la revendication 1, dans lequel le dérivé d'azépine de formule (I) est choisi dans le groupe consistant en

(1R)-3-[2-(1-naphtyl)éthyl]-1,8,8-triméthyl-3-azabicylo[3.2.1]octane,
(1R)-3-[2-(2-naphtyl)éthyl]-1,8,8-triméthyl-3-azabicylo[3.2.1]octane,
(1R)-3-[2-(2-pyridyl)éthyl]-1,8,8-triméthyl-3-azabicylo[3.2.1]octane,
(1R)-3-[2-(4-pyridyl)éthyl]-1,8,8-triméthyl-3-azabicylo[3.2.1]octane,
(1S)-3-[2-(1-naphtyl)éthyl]-1,8,8-triméthyl-3-azabicylo[3.2.1]octane,
(1S)-3-[2-(2-naphtyl)éthyl]-1,8,8-triméthyl-3-azabicylo[3.2.1]octane,
(1S)-3-[2-(2-pyridyl)éthyl]-1,8,8-triméthyl-3-azabicylo[3.2.1]octane,
(1S)-3-[2-(3-pyridyl)éthyl]-1,8,8-triméthyl-3-azabicylo[3.2.1]octane,
(1S)-3-[2-(4-pyridyl)éthyl]-1,8,8-triméthyl-3-azabicylo[3.2.1]octane,
(1S)-3-[2-(1,3-benzodioxol-5-yl)éthyl]-1,8,8-triméthyl-3-azabicylo[3.2.1]octane, et
(1S)-3-[2-(2-thiényl)éthyl]-1,8,8-triméthyl-3-azabicylo[3.2.1]octane
ou l'un de leurs sels non toxiques.

4. Composé suivant la revendication 1, dans lequel le dérivé d'azépine de formule (I) est le

(1S)-3-[2-(2-adamantyl)éthyl]-1,8,8-triméthyl-3-azabicyclo[3.2.1] octane
ou l'un de ses sels non toxiques.

5. Procédé de préparation d'un dérivé d'azépine de formule (I) défini à la revendication 1 ou de l'un de ses sels non toxiques qui comprend réduire une groupe carbonyle du composé de formule

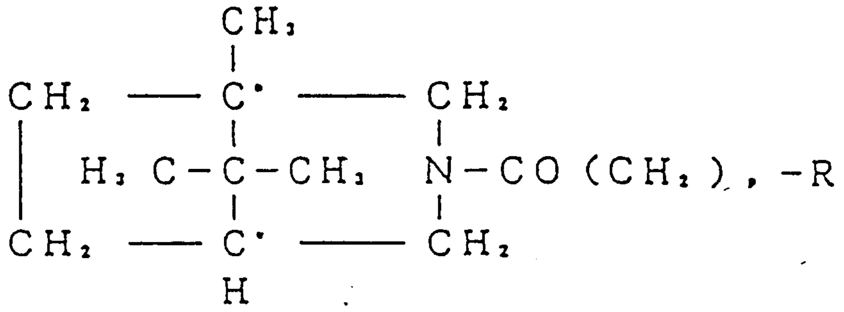

(3)

dans laquelle R a la même signification qu'à la revendication 1, p est un nombre entier de 0 à 3 et C* est un carbone asymétrique et, si nécessaire, transformer le composé en l'un de ses sels non toxiques.

**6.** Procédé suivant la revendication 5 dans lequel le dérivé d'azépine de formule (I) défini à la revendication 1 est choisi dans le groupe consistant en

(1S)-3-[2-(1-adamantyl)éthyl]-1,8,8-triméthyl-3-azabicylo[3.2.1]octane,
(1S)-3-[2-(1-norbornyl)éthyl]-1,8,8-triméthyl-3-azabicylo[3.2.1]octane,
(1S)-3-(2-cyclopentyléthyl)-1,8,8-triméthyl-3-azabicylo[3.2.1]octane,
(1S)-3-(2-cyclohexyléthyl)-1,8,8-triméthyl-3-azabicylo[3.2.1]octane,
(1R)-3-(2-cyclohexyléthyl)-1,8,8-triméthyl-3-azabicylo[3.2.1]octane,
(1S)-3-(2-cycloheptyléthyl)-1,8,8-triméthyl-3-azabicylo[3.2.1]octane,
(1S)-3-(2-cyclooctyléthyl)-1,8,8-triméthyl-3-azabicylo[3.2.1]octane,
(1S)-3-(4-cyclohexyl-1-butyl)-1,8,8-triméthyl-3-azabicylo[3.2.1]octane,
(1S)-3-(3-cyclohexylpropyl)-1,8,8-triméthyl-3-azabicylo[3.2.1]octane,
(1S)-3-cyclohexylméthyl-1,8,8-triméthyl-3-azabicylo[3.2.1]octane,
(1R)-3-cyclohexylméthyl-1,8,8-triméthyl-3-azabicylo[3.2.1]octane,
(1S)-3-cyclohexyl-1,8,8-trimethyl-3-azabicylo[3.2.1]octane,
(1R)-3-[2-(1-adamantyl)éthyl]-1,8,8-triméthyl-3-azabicylo[3.2.1]octane,
(1R)-3-[2-(2-adamantyl)éthyl]-1,8,8-triméthyl-3-azabicylo[3.2.1]octane,
(1S)-3-[2-(4-méthylcyclohexyl)éthyl]-1,8,8-trimethyl-3-azabicylo[3.2.1]octane,
(1S)-3-[2-(3-méthyl-1-adamantyl)éthyl]-1,8,8-trimethyl-3-azabicylo[3.2.1]octane,
(1S)-3-(3-noradamantylméthyl)-1,8,8-triméthyl-3-azabicylo[3.2.1]octane,
(1S)-3-[2-(bicyclo[3.3.1]nonan-9-yl)-1-éthyl]-1,8,8-triméthyl-3-azabicylo[3.2.1]octane
(1S)-3-(1-adamantylméthyl)-1,8,8-triméthyl-3-azabicylo[3.2.1]octane,
(1S)-3-[2-(5-hydroxy-2-adamantyl)éthyl]-1,8,8-triméthyl-3-azabicylo[3.2.1]octane,
(1S)-3-[2-(5-méthoxy-2-adamantyl)éthyl]-1,8,8-triméthyl-3-azabicylo[3.2.1]octane
(1S)-3-[2-(5-chloro-2-adamantyl)éthyl]-1,8,8-triméthyl-3-azabicylo[3.2.1]octane,
(1S)-3-[2-(5-phényl-2-adamantyl)éthyl]-1,8,8-triméthyl-3-azabicylo[3.2.1]octane,
(1S)-3-[2-(4-hydroxy-2-adamantyl)éthyl]-1,8,8-triméthyl-3-azabicylo[3.2.1]octane,
(1S)-3-[2-(4-phényl-2-adamantyl)éthyl]-1,8,8-triméthyl-3-azabicylo[3.2.1]octane,
(1R)-3-[2-(4-phényl-2-adamantyl)éthyl]-1,8,8-triméthyl-3-azabicylo[3.2.1]octane,
(1R)-3-[2-(5-méthoxy-2-adamantyl)éthyl]-1,8,8-triméthyl-3-azabicylo[3.2.1]octane,
(1R)-3-[2-(5-hydroxy-2-adamantyl)éthyl]-1,8,8-triméthyl-3-azabicylo[3.2.1]octane,

(1R)-3-[2-(5-chloro-2-adamantyl)éthyl]-1,8,8-triméthyl-3-azabicyclo[3.2.1]octane,
(1R)-3-[2-(5-phenyl-2-adamantyl)éthyl]-1,8,8-triméthyl-3-azabicyclo[3.2.1]octane,
(1R)-3-[2-(4-hydroxy-2-adamantyl)éthyl]-1,8,8-triméthyl-3-azabicyclo[3.2.1]octane,
(1R)-3-[2-(3-methyl-1-adamantyl)éthyl]-1,8,8-triméthyl-3-azabicyclo[3.2.1]octane,
(1S)-3-[3-(2-adamantyl)-1-propyl]-1,8,8-triméthyl-3-azabicyclo[3.2.1]octane,
(1R)-3-[3-(2-adamantyl)-1-propyl]-1,8,8-triméthyl-3-azabicyclo[3.2.1]octane,
(1S)-3-[2-(3-noradamantyl)éthyl]-1,8,8-triméthyl-3-azabicyclo[3.2.1]octane,
(1S)-3-(2-adamantylméthyl)-1,8,8-triméthyl-3-azabicyclo[3.2.1]octane, et
(1R)-3-(2-adamantylméthyl)-1,8,8-triméthyl-3-azabicyclo[3.2.1]octane
ou l'un de leurs sels non toxiques.

7. Procédé suivant la revendication 5, dans lequel le dérivé d'azépine de formule (I) défini à la revendication 1 est choisi dans le groupe consistant en

(1R)-3-[2-(1-naphtyl)éthyl]-1,8,8-triméthyl-3-azabicyclo[3.2.1]octane,
(1R)-3-[2-(2-naphtyl)éthyl]-1,8,8-triméthyl-3-azabicyclo[3.2.1]octane,
(1R)-3-[2-(2-pyridyl)éthyl]-1,8,8-triméthyl-3-azabicyclo[3.2.1]octane,
(1R)-3-[2-(4-pyridyl)éthyl]-1,8,8-triméthyl-3-azabicyclo[3.2.1]octane,
(1S)-3-[2-(1-naphtyl)éthyl]-1,8,8-triméthyl-3-azabicyclo[3.2.1]octane,
(1S)-3-[2-(2-naphtyl)éthyl]-1,8,8-triméthyl-3-azabicyclo[3.2.1]octane,
(1S)-3-[2-(2-pyridyl)éthyl]-1,8,8-triméthyl-3-azabicyclo[3.2.1]octane,
(1S)-3-[2-(3-pyridyl)éthyl]-1,8,8-triméthyl-3-azabicyclo[3.2.1]octane,
(1S)-3-[2-(4-pyridyl)éthyl]-1,8,8-triméthyl-3-azabicyclo[3.2.1]octane,
(1S)-3-[2-(1,3-benzodioxol-5-yl)éthyl]-1,8,8-triméthyl-3-azabicyclo[3.2.1]octane, et
(1S)-3-[2-(2-thiényl)éthyl]-1,8,8-triméthyl-3-azabicyclo[3.2.1]octane
ou l'un de leurs sels non toxiques.

8. Procédé suivant la revendication 5, dans lequel le dérivé d'azépine de formule (I) défini à la revendication 1 est le

(1S)-3-[2-(2-adamantyl)éthyl]-1,8,8-triméthyl-3-azabicyclo[3.2.1]octane
ou l'un de ses sels non toxiques.

9. Procédé de préparation d'un dérivé d'azépine de formule (I) défini à la revendication 1 ou de l'un de ses sels non toxiques, qui comprend faire réagir une amine de formule

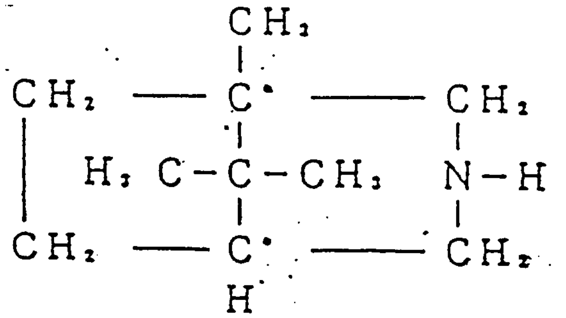

(4)

dans laquelle C* est un carbone asymétrique sur un dérivé réactif de formule

$$X\text{-}(CH_2)m\text{-}R$$

dans laquelle X est un sulfonyloxy organic réactif ou un halogène, R a la même signification qu'à la revendication 1 et m est un nombre entier de 0 à 4 dans un solvant organique inerte en la présence d'une base et, si nécessaire, transformer le composé en l'un de ses sels non toxiques.

**10.** Procédé suivant la revendication 9, dans lequel le dérivé d'azépine de formule (I) défini à la revendication 1 est choisi dans le groupe consistant en

(1S)-3-[2-(1-adamantyl)éthyl]-1,8,8-triméthyl-3-azabicyclo[3.2.1]octane,
(1S)-3-[2-(1-norbornyl)éthyl]-1,8,8-triméthyl-3-azabicyclo[3.2.1]octane,
(1S)-3-(2-cyclopentyléthyl)-1,8,8-triméthyl-3-azabicyclo[3.2.1]octane,
(1S)-3-(2-cyclohexyléthyl)-1,8,8-triméthyl-3-azabicyclo[3.2.1]octane,
(1R)-3-(2-cyclohexyléthyl)-1,8,8-triméthyl-3-azabicyclo[3.2.1]octane,
(1S)-3-(2-cycloheptyléthyl)-1,8,8-triméthyl-3-azabicyclo[3.2.1]octane,
(1S)-3-(2-cyclooctyléthyl)-1,8,8-triméthyl-3-azabicyclo[3.2.1]octane,
(1S)-3-(4-cyclohexyl-1-butyl)-1,8,8-triméthyl-3-azabicyclo[3.2.1]octane,
(1S)-3-(3-cyclohexylpropyl)-1,8,8-triméthyl-3-azabicyclo[3.2.1]octane,
(1S)-3-cyclohexylméthyl-1,8,8-triméthyl-3-azabicyclo[3.2.1]octane,
(1R)-3-cyclohexylméthyl-1,8,8-triméthyl-3-azabicyclo[3.2.1]octane,
(1S)-3-cyclohexyl-1,8,8-triméthyl-3-azabicyclo[3.2.1]octane,
(1R)-3-[2-(1-adamantyl)éthyl]-1,8,8-triméthyl-3-azabicyclo[3.2.1]octane,
(1R)-3-[2-(2-adamantyl)éthyl]-1,8,8-triméthyl-3-azabicyclo[3.2.1]octane,
(1S)-3-[2-(4-méthylcyclohexyl)éthyl]-1,8,8-triméthyl-3-azabicyclo[3.2.1]octane,
(1S)-3-[2-(3-méthyl-1-adamantyl)éthyl]-1,8,8-triméthyl-3-azabicyclo[3.2.1]octane,
(1S)-3-(3-noradamantylméthyl)-1,8,8-triméthyl-3-azabicyclo[3.2.1]octane,
(1S)-3-[2-(bicyclo[3.3.1]nonan-9-yl)-1-éthyl]-1,8,8-triméthyl-3-azabicyclo[3.2.1]octane

(1S)-3-(1-adamantylméthyl)-1,8,8-triméthyl-3-azabicyclo[3.2.1]octane,
(1S)-3-[2-(5-hydroxy-2-adamantyl)éthyl]-1,8,8-triméthyl-3-azabicyclo[3.2.1]octane,
(1S)-3-[2-(5-méthoxy-2-adamantyl)éthyl]-1,8,8-triméthyl-3-azabicyclo[3.2.1]octane
(1S)-3-[2-(5-chloro-2-adamantyl)éthyl]-1,8,8-triméthyl-3-azabicyclo[3.2.1]octane,
(1S)-3-[2-(5-phényl-2-adamantyl)éthyl]-1,8,8-triméthyl-3-azabicyclo[3.2.1]octane;
(1S)-3-[2-(4-hydroxy-2-adamantyl)éthyl]-1,8,8-triméthyl-3-azabicyclo[3.2.1]octane,
(1S)-3-[2-(4-phényl-2-adamantyl)éthyl]-1,8,8-triméthyl-3-azabicyclo[3.2.1]octane,
(1R)-3-[2-(4-phényl-2-adamantyl)éthyl]-1,8,8-triméthyl-3-azabicyclo[3.2.1]octane,
(1R)-3-[2-(5-méthoxy-2-adamantyl)éthyl]-1,8,8-triméthyl-3-azabicyclo[3.2.1]octane,
(1R)-3-[2-(5-hydroxy-2-adamantyl)éthyl]-1,8,8-triméthyl-3-azabicyclo[3.2.1]octane,
(1R)-3-[2-(5-chloro-2-adamantyl)éthyl]-1,8,8-triméthyl-3-azabicyclo[3.2.1]octane,
(1R)-3-[2-(5-phényl-2-adamantyl)éthyl]-1,8,8-triméthyl-3-azabicyclo[3.2.1]octane,
(1R)-3-[2-(4-hydroxy-2-adamantyl)éthyl]-1,8,8-triméthyl-3-azabicyclo[3.2.1]octane,
(1R)-3-[2-(3-méthyl-1-adamantyl)éthyl]-1,8,8-triméthyl-3-azabicyclo[3.2.1]octane,
(1S)-3-[3-(2-adamantyl)-1-propyl]-1,8,8-triméthyl-3-azabicyclo[3.2.1]octane,
(1R)-3-[3-(2-adamantyl)-1-propyl]-1,8,8-triméthyl-3-azabicyclo[3.2.1]octane,
(1S)-3-[2-(3-noradamantyl)éthyl]-1,8,8-triméthyl-3-azabicyclo[3.2.1]octane,
(1S)-3-(2-adamantylméthyl)-1,8,8-triméthyl-3-azabicyclo[3.2.1]octane, et
(1R)-3-(2-adamantylméthyl)-1,8,8-triméthyl-3-azabicyclo[3.2.1]octane
ou l'un de leurs sels non toxiques.

**11.** Procédé suivant la revendication 9, dans lequel le dérivé d'azépine de la formule (I) défini à la revendication 1 est choisi dans le groupe consistant en

(1R)-3-[2-(1-naphtyl)éthyl]-1,8,8-triméthyl-3-azabicyclo[3.2.1]octane,
(1R)-3-[2-(2-naphtyl)éthyl]-1,8,8-triméthyl-3-azabicyclo[3.2.1]octane,
(1R)-3-[2-(2-pyridyl)éthyl]-1,8,8-triméthyl-3-azabicyclo[3.2.1]octane,
(1R)-3-[2-(4-pyridyl)éthyl]-1,8,8-triméthyl-3-azabicyclo[3.2.1]octane,
(1S)-3-[2-(1-naphtyl)éthyl]-1,8,8-triméthyl-3-azabicyclo[3.2.1]octane,
(1S)-3-[2-(2-naphtyl)éthyl]-1,8,8-triméthyl-3-azabicyclo[3.2.1]octane,
(1S)-3-[2-(2-pyridyl)éthyl]-1,8,8-triméthyl-3-azabicyclo[3.2.1]octane,
(1S)-3-[2-(3-pyridyl)éthyl]-1,8,8-triméthyl-3-azabicyclo[3.2.1]octane,
(1S)-3-[2-(4-pyridyl)éthyl]-1,8,8-triméthyl-3-azabicyclo[3.2.1]octane,
(1S)-3-[2-(1,3-benzodioxol-5-yl)éthyl]-1,8,8-triméthyl-3-azabicyclo[3.2.1]octane, et
(1S)-3-[2-(2-thiényl)éthyl]-1,8,8-triméthyl-3-azabicyclo[3.2.1]octane
ou l'un de leurs sels non toxiques.

**12.** Procédé suivant la revendication 9, dans lequel le dérivé d'azépine de la formule (I) défini à la revendication 1 est le

(1S)-3-[2-(2-adamantyl)éthyl]-1,8,8-triméthyl-3-azabicyclo[3.2.1]octane

ou l'un de ses sels non toxiques.

**13.** Médicament de traitement de troubles impliqués dans le récepteur σ comprenant un dérivé d'azépine de formule (I) défini à la revendication 1 ou l'un de ses sels non toxiques comme principe actif.

**14.** Médicament de traitement de la schizophrénie contenant un dérivé d'azépine de formule (I) défini à la revendication 1 ou l'un de ses sels non toxiques comme principe actif.

**15.** Dérivé d'azépine de formule

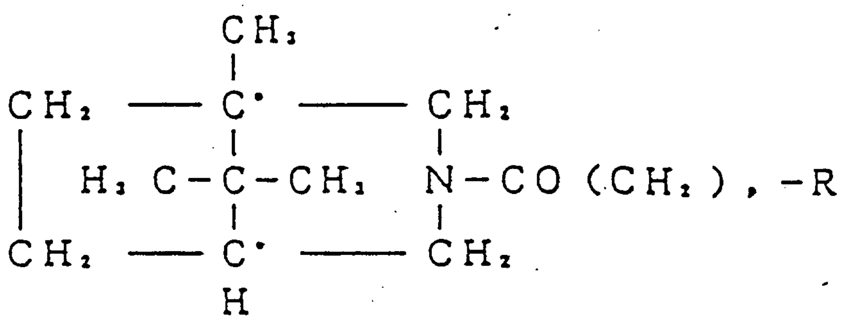

(3)

dans laquelle R est la formule

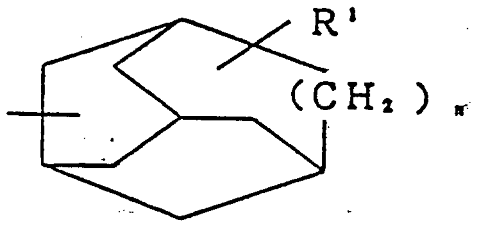

(2)

dans laquelle R1 est hydrogène, alcoyle ayant de 1 à 4 atomes de carbone, alcoxy ayant de 1 à 4 atomes de carbone, hydroxy, halogène ou phényle éventuellement substitué par de 1 à 3 groupes alcoyle ayant de 1 à 4 atomes de carbone, alcoxy ayant de 1 à 4 atomes de carbone, hydroxy ou halogène et n est 0 ou 1, cycloalcoyle ayant de 5 à 8 atomes de carbone qui est éventuellement substitué par un alcoyle ayant de 1 à 4 atomes de carbone, norbornyle, bicyclo [3.3.1] nonyle, naphtyle, 1,3-benzodioxolyle, pyridyle ou thiényle, p est un nombre entier de 0 à 3 et C* est un carbone asymétrique ou l'un de ses sels non toxiques.